# EUROPEAN PATENT APPLICATION

(11) **EP 4 129 964 A1**
(43) Date of publication of application: **08.02.2023**
(21) Application number: 21804847.8
(22) Date of filing: 19.04.2021
(51) Int. Cl.: C07C 22/08, C07D 213/06, C07D 215/06, C07D 307/91, C07D 491/04, C07D 333/76, H01L 51/50

(54) **ORGANIC COMPOUND AND ORGANIC ELECTROLUMINESCENT DEVICE COMPRISING SAME**

(30) Priority: 13.05.2020 KR 20200057311
(71) Applicant: Lapto Co., Ltd., Gyeonggi-do 13215 (KR)
(72) Inventor: KIM, Gyu-ri, Seongnam-si, Gyeonggi-do 13215 (KR); KIM, Gyung-woo, Seongnam-si, Gyeonggi-do 13215 (KR); SEOK, Moon-ki, Seongnam-si, Gyeonggi-do 13215 (KR); KIM, Kyou-sic, Seongnam-si, Gyeonggi-do 13215 (KR); HAN, Kap-jong, Seongnam-si, Gyeonggi-do 13215 (KR); OH, Eu-gene, Seongnam-si, Gyeonggi-do 13215 (KR)
(74) Representative: Isarpatent
(86) International application number: PCT/KR2021/004882
(87) International publication number: WO 2021/230513

(57) **Abstract**

Provided is an organic compound contributing to improving the substantial light efficiency and field of view of an organic electroluminescent device. The organic electroluminescent device according to the present invention comprises: a first electrode; a second electrode; one or more organic material layers disposed between the first electrode and the second electrode; and a capping layer, wherein the organic material layers or capping layer comprises an organic compound represented by chemical formula 1 of the present invention.

## Description

### Technical Field

The present invention relates to an organic compound and an organic electroluminescent device including the organic compound, particularly an organic electroluminescent device including a capping layer in which the organic compound is used to achieve low refractive index.

### Background Art

Organic light emitting diodes (OLEDs) have attracted attention as promising self-luminous displays in the display industry.

In 1963, Pope *et al.* have conducted the first research on carrier injection electroluminescence (EL) utilizing a single crystal of an anthracene aromatic hydrocarbon in the field of OLEDs. Based on this research, basic mechanisms such as charge injection, recombination, exciton formation, and light emission in organic materials, and luminescent properties have been understood and studied.

Particularly, various approaches aimed at increasing the luminescent efficiency of devices have been proposed, for example, in connection with changes in the structure of devices and the development of device materials (Sun, S., Forrest, S. R., Appl. Phys. Lett. 91, 263503 (2007)/Ken-Tsung Wong, Org. Lett., 7, 2005, 5361-5364).

A typical OLED display basically has a multilayer structure consisting of an anode, a hole injection layer (HIL), a hole transporting layer (HTL), an emission layer (EML), an electron transporting layer (ETL), and a cathode. That is, the OLED display has a structure in which an organic multilayer film is sandwiched between both electrodes.

In general, organic luminescence refers to a phenomenon in which organic materials are used to convert electrical energy into light energy. An organic light emitting device using organic luminescence usually has a structure including an anode, a cathode, and an organic layer interposed therebetween. The organic layer often consists of one or more layers composed of different materials to increase the efficiency and stability of the organic light emitting device. For example, the organic layer may include a hole injection layer, a hole transporting layer, an emission layer, an electron transporting layer, and an electron injection layer.

When a voltage is applied between the two electrodes of the organic light emitting device, holes and electrons are injected into the organic layer from the anode and the cathode, respectively. The holes recombine with the electrons in the organic layer to form excitons. The excitons fall to the ground state to emit light. Such organic light emitting devices are known to have many advantages, including self-luminescence, high luminance, high efficiency, low driving voltage, wide viewing angle, high contrast, and fast response.

Materials for organic layers of organic light emitting devices can be classified into light emitting materials and charge transporting materials, for example, hole injection materials, hole transporting materials, electron transporting materials, and electron injection materials, by their functions.

Light emitting materials include blue, green, and red light emitting materials that emit different colors of light and yellow and orange light emitting materials that are necessary to obtain more natural colors. Other light emitting materials are host/dopant systems that can be used to achieve high color purity and luminescent efficiency through energy transfer. The principle is that when a small amount of a dopant, whose energy band gap is smaller and whose luminescent efficiency is higher than those of a host as a main material for an emission layer, is introduced into the emission layer, excitons generated from the host are transported to the dopant to emit light with high efficiency. At this time, since the wavelength of the host is shifted to the wavelength band of the dopant, light of a desired wavelength can be obtained depending on the type of the dopant.

Materials for organic layers of organic light emitting devices, for example, hole injection materials, hole transporting materials, light emitting materials, electron transporting materials, and electron injection materials, have been developed to achieve excellent characteristics of the devices. Organic light emitting devices using commercialized materials for organic layers have been recognized for their performance.

However, as time has passed since the commercialization of organic light emitting devices, characteristics other than the luminescent properties of organic light emitting devices have been taken into consideration.

Most organic light emitting devices are exposed to external light sources for a long time and thus remain exposed to high-energy UV light, which continuously affects organic materials constituting the organic light emitting devices. This problem can be solved by applying UV-absorbing capping layers to organic light emitting devices to prevent their exposure to high-energy light sources.

A typical organic light emitting device is known to have a wide viewing angle but undergoes a large variation in terms of light source's spectrum depending on the viewing angle. This is due to a deviation between the total refractive index of constituent elements and materials (e.g., a glass substrate, organic materials, and electrode materials) of the organic light emitting device and an optimal refractive index depending on the emission wavelength of the organic light emitting device.

Generally, a high refractive index is required for blue light emission and a lower refractive index is required for a longer wavelength. There is thus a need to develop a material for a capping layer that simultaneously meets the aforementioned requirements in terms of UV absorption characteristics and optimal refractive index.

The efficiency of an organic light emitting device can be generally divided into internal luminescent efficiency and external luminescent efficiency. The internal luminescent efficiency is related to the formation efficiency of excitons in the organic layer for photoconversion.

The external luminescent efficiency refers to the emission efficiency of light from the organic layer outside the organic light emitting device.

Not only high internal luminescent efficiency but also high external luminescent efficiency is required to raise the overall efficiency. Thus, there is a need to develop a material for a capping layer (CPL) that has an outstanding ability to increase external luminescent efficiency.

Unlike a bottom-emitting device having a non-resonant structure, a top-emitting device having a resonant structure is constructed such that the produced light is reflected from the anode as a reflective film and is directed toward the cathode, resulting in a significant loss of optical energy due to surface plasmon polaritons (SPPs).

Accordingly, one important approach to improve the shape and efficiency of the EL spectrum is to form a capping layer on the top cathode.

As for SPPs, four metals, including Al, Pt, Ag, and Au, are mainly used for electron emission and surface plasmons are generated on the surfaces of the metal electrodes. For example, when Ag is used for the cathode, emitted light is quenched by SPPs (light energy is lost by Ag), resulting in efficiency reduction.

In contrast, the use of a capping layer causes the generation of SPPs at the interface between a MgAg electrode and an organic material. When the organic material has a high refractive index (for example, n > 1.69 at 620 nm), transverse electric (TE) polarized light is dissipated on the surface of the capping layer in the vertical direction by evanescent waves, the wavelength of transverse magnetic (TM) polarized light traveling along the cathode and the capping layer is amplified by surface plasmon resonance, and as a result, the intensity of the peak increases, achieving high efficiency and enabling effective control over color purity.

However, there remains a need to develop materials and structures of organic light emitting devices that can improve various characteristics of the devices in a balanced manner while achieving improved efficiency and color purity of the devices.

### Detailed Description of the Invention

### Problems to be Solved by the Invention

One object of the present invention is to provide a material for a capping layer of an organic light emitting device that can be used to improve the luminescent efficiency and lifetime of the device while achieving improved viewing angle characteristics.

A further object of the present invention is to provide a high-efficiency, long-lasting organic electroluminescent device including a capping layer whose refractive index is controlled, particularly to achieve improved light extraction efficiency.

### Means for Solving the Problems

The present invention provides an organic electroluminescent device including a first electrode, an organic layer arranged on the first electrode, a second electrode arranged on the organic layer, and a capping layer arranged on the second electrode wherein the organic layer or the capping layer includes an organic compound represented by Formula 1: 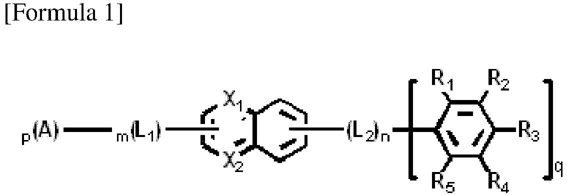 wherein X₁ and X₂ are each independently N or CH, L₁ and L₂ are each independently selected from substituted or unsubstituted phenyl groups, substituted or unsubstituted naphthyl groups, and substituted or unsubstituted pyridyl groups, R₁ to R₅ are each independently F, CF₃ or Si(CH₃)₃, p and q are each independently an integer from 1 to 5, m and n are each independently an integer from 0 to 5, provided that when m and n are 0, L₁ and L₂ are direct bonds and provided that when m and n are 2 or more, L₁ and L₂ are each independently the same as or different from each other, and A is selected from the group consisting of structures represented by the following formulae 2: 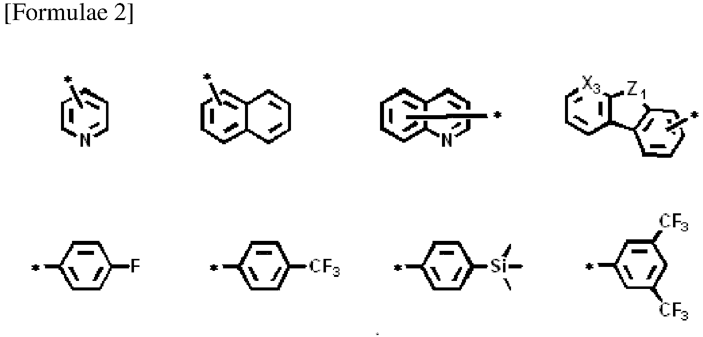 wherein Zi is O or S and X₃ is N or CH.

### Effects of the Invention

The compound of the present invention can be used as a material for a capping layer or an organic layer of an organic light emitting device.

The use of the compound according to the present invention as a material for a low refractive index capping layer of an organic light emitting device can lead to an improvement in the luminous efficiency of the device and a reduction in the full width at half maximum of the emission spectrum, resulting in a significant improvement in color purity.

The organic electroluminescent device of the present invention has a structure in which high refractive index organic thin films and low refractive thin films are continuously introduced on a MgAg electrode. This structure can improve the viewing angle and optical efficiency of light extracted into the air due to waveguide resonance.

### Brief Description of the Drawings

Fig. 1 shows an exemplary structure of an organic light emitting device according to one embodiment of the present invention in which a first electrode 110, a hole injection layer 210, a hole transporting layer 215, an emission layer 220, an electron transporting layer 230, an electron injection layer 235, a second electrode 120, and a capping layer 300 stacked in this order on a substrate 100.
Fig. 2 shows the refraction and absorption properties of light when organic compounds according to exemplary embodiments of the present invention were used.

### Best Mode for Carrying out the Invention

The present invention will now be described in more detail.

As the present invention allows for various changes and numerous embodiments, particular embodiments will be illustrated in drawings and described in detail in the written description. However, this is not intended to limit the present invention to particular modes of practice, and it is to be appreciated that all changes, equivalents, and substitutes that do not depart from the spirit and technical scope of the present invention are encompassed in the present invention.

In the description of drawings, like reference numerals are used to designate like components. In the accompanying drawings, the dimensions of structures may be exaggerated for clarity of the present invention. While such terms as "first" and "second," etc., may be used to describe various components, such components must not be limited to the above terms. The above terms are used only to distinguish one component from another. For example, a first component may be referred to as a second component without departing from the scope of rights of the present invention, and likewise a second component may be referred to as a first component. As used herein, the singular forms "a", "an" and "the" are intended to include the plural forms as well, unless the context clearly indicates otherwise.

In the present application, it is to be understood that the terms such as "comprises", "comprising", "includes", "including", "has" or "having" are intended to indicate the existence of the features, numbers, steps, operations, components, parts, or combinations thereof disclosed in the specification, and are not intended to preclude the possibility that one or more other features, numbers, steps, operations, components, parts, or combinations thereof may exist or may be added. It will also be understood that when an element such as a layer, film, region or plate is referred to as being "on" another element, it can be directly on the other element or intervening elements may also be present.

As used herein, the term "substituted" in the definition of "substituted or unsubstituted" refers to substitution with at least one substituent selected from the group consisting of deuterium and halogen atoms and cyano, nitro, amino, hydroxyl, silyl, boron, phosphine oxide, phosphine sulfide, alkyl, alkoxy, alkenyl, aryl, heteroaryl, and heterocyclic groups. The term "unsubstituted" in the same definition indicates having no substituent. Each of the substituents exemplified above may be optionally further substituted. For example, a biphenyl group may be interpreted as an aryl group or a phenyl group substituted with a phenyl group.

Examples of the halogen atoms include fluorine, chlorine, bromine, and iodine atoms.

The alkyl groups may be linear, branched or cyclic. The number of carbon atoms in each of the alkyl groups is 1 to 50, 1 to 30, 1 to 20, 1 to 10, or 1 to 6. Examples of the alkyl groups include, but are not limited to, methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, and n-triacontyl groups.

As used herein, the term "cyclic hydrocarbon group" refers to any functional group or substituent derived from an alicyclic hydrocarbon. The cyclic hydrocarbon group may be a saturated cyclic hydrocarbon group having 5 to 20 ring carbon atoms.

As used herein, the term "aryl group" refers to any functional group or substituent derived from an aromatic cyclic hydrocarbon ring. The aryl group may be monocyclic or polycyclic. The number of ring carbon atoms in the aryl group may be 6 to 30, 6 to 20, or 6 to 15. Examples of such aryl groups include, but are not limited to, phenyl, naphthyl, fluorenyl, anthracenyl, phenanthryl, biphenyl, terphenyl, quaterphenyl, quinquephenyl, sexiphenyl, triphenylenyl, pyrenyl, perylenyl, naphthacenyl, benzofluoranthenyl, and chrysenyl groups.

The fluorenyl group may be substituted. In this case, two substituents may be bonded to each other to form a spiro structure.

The heteroaryl group may be interrupted by one or more heteroatoms selected from O, N, P, Si, and S. The N and S atoms may be optionally oxidized and the N atom(s) may be optionally quaternized. The number of ring carbon atoms in the heteroaryl group is 2 to 30 or 2 to 20. The heteroaryl group may be monocyclic or polycyclic. For example, the polycyclic heteroaryl group may have a bicyclic or tricyclic structure.

Examples of such heteroaryl groups include, but are not limited to, thiophenyl, furanyl, pyrrolyl, imidazolyl, pyrazolyl, thiazolyl, oxazolyl, oxadiazolyl, triazolyl, pyridinyl, bipyridinyl, pyrimidinyl, triazinyl, tetrazinyl, triazolyl, tetrazolyl, acridyl, pyridazinyl, pyrazinyl, quinolinyl, quinazolinyl, quinoxalinyl, phenoxazinyl, phthalazinyl, pyridopyrimidinyl, pyridopyrazinopyrazinyl, isoquinolinyl, cinnolinyl, indolyl, isoindolyl, indazolyl, carbazolyl, N-arylcarbazolyl, N-heteroarylcarbazolyl, N-alkylcarbazolyl, benzoxazolyl, benzoimidazolyl, benzothiazolyl, benzocarbazolyl, benzothiophenyl, benzoisothiazolyl, benzoisoxazolyl, dibenzothiophenyl, thienothiophenyl, benzofuranyl, phenanthrolinyl, phenanthridinyl, thiazolyl, isoxazolyl, oxadiazolyl, thiadiazolyl, isothiazolyl, phenothiazinyl, benzodioxolyl, dibenzosilolyl, dibenzofuranyl, and isobenzofuranyl groups. An N-oxide aryl group may correspond to the monocyclic or polycyclic heteroaryl group. The N-oxide aryl group may be, for example, a quaternary salt such as a pyridyl N-oxide or quinolyl N-oxide group but is not limited thereto.

As used herein, the term "silyl group" is intended to include alkylsilyl and arylsilyl groups. Examples of such silyl groups include, but are not limited to, trimethylsilyl, triethylsilyl, t-butyldimethylsilyl, vinyldimethylsilyl, propyldimethylsilyl, triphenylsilyl, diphenylsilyl, and phenylsilyl groups.

As used herein, the term "boron group" is intended to include alkyl boron and aryl boron groups. Examples of such boron groups include, but are not limited to, trimethyl boron, triethyl boron, t-butyl dimethyl boron, triphenyl boron, diphenyl boron, and phenyl boron groups.

The alkenyl groups may be linear or branched. The number of carbon atoms in each of the alkeyl groups is 2 to 30, 2 to 20, or 2 to 10, but is not particularly limited thereto. Examples of the alkenyl groups include, but are not limited to, vinyl, 1-butenyl, 1-pentenyl, 1,3-butadienyl, allyl, styrenyl, and styrylvinyl groups.

As used herein, the term "arylamine group" is intended to include substituted or unsubstituted monoarylamine, substituted or unsubstituted diarylamine, and substituted or unsubstituted triarylamine groups. The aryl moieties in the arylamine groups may be monocyclic or polycyclic or may include monocyclic and polycyclic ones.

Specific examples of such arylamine groups include phenylamine, naphthylamine, biphenylamine, anthracenylamine, 3-methyl-phenylamine, 4-methyl-naphthylamine, 2-methyl-biphenylamine, 9-methyl-anthracenylamine, diphenylamine, phenylnaphthylamine, ditolylamine, phenyltolylamine, carbazole, and triphenylamine groups, but are not limited thereto.

As used herein, the term "heteroarylamine group" is intended to include substituted or unsubstituted monoheteroarylamine, substituted or unsubstituted diheteroarylamine, and substituted or unsubstituted triheteroarylamine groups. The heteroaryl moieties in the heteroarylamine groups may be monocyclic or polycyclic. The heteroarylamine group may include two or more heterocyclic moieties. In this case, the heterocyclic moieties may be monocyclic or polycyclic or may include monocyclic and polycyclic ones.

As used herein, the term "arylheteroarylamine group" refers to an amine group substituted with aryl and heterocyclic moieties.

As used herein, the term "adjacent group" may mean a substituent on an atom directly attached to an atom substituted with the corresponding substituent, another substituent on an atom substituted with the corresponding substituent or a substituent disposed sterically closest to the corresponding substituent. For example, the two methyl groups in 1,2-dimethylbenzene can be interpreted as "adjacent groups" and the two ethyl groups in 1,1-diethylcyclopentene can be interpreted as "adjacent groups".

The present invention provides an organic compound that is used to form an organic layer and/or a capping layer.

According to one embodiment of the present invention, the organic compound is represented by Formula 1: 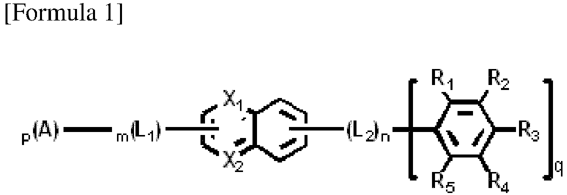 wherein X₁ and X₂ are each independently N or CH, L₁ and L₂ are each independently selected from substituted or unsubstituted phenyl groups, substituted or unsubstituted naphthyl groups, and substituted or unsubstituted pyridyl groups, R₁ to R₅ are each independently F, CF₃ or Si(CH₃)₃, p and q are each independently an integer from 1 to 5, m and n are each independently an integer from 0 to 5, provided that when m and n are 0, L₁ and L₂ are direct bonds and provided that when m and n are 2 or more, L₁ and L₂ are each independently the same as or different from each other, and A is selected from the group consisting of structures represented by the following formulae 2: 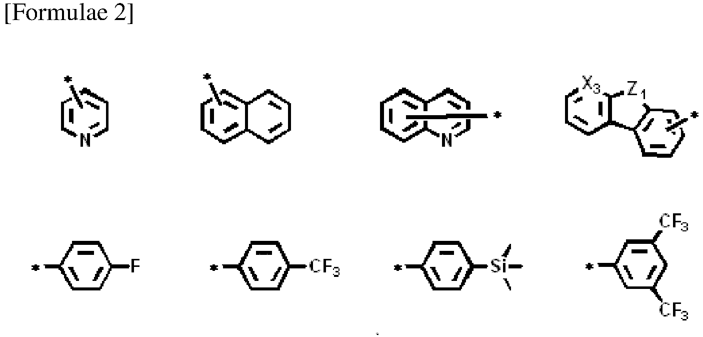 wherein Zi is O or S and X₃ is N or CH.

According to one embodiment of the present invention, the organic compound represented by Formula 1 may be selected from the compounds represented by Formulae 3 to 5:

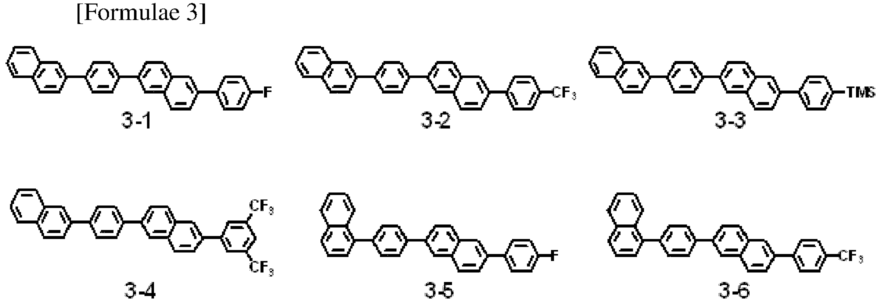

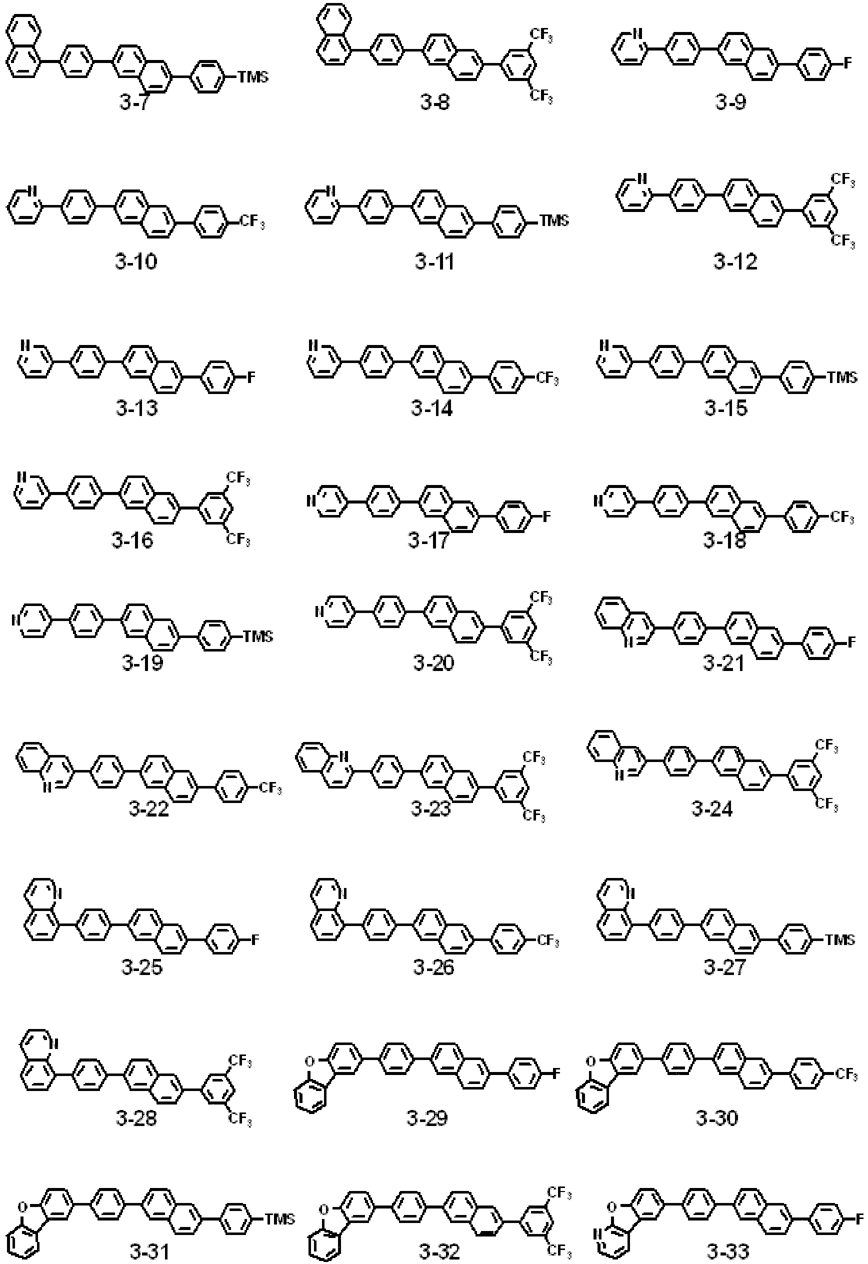

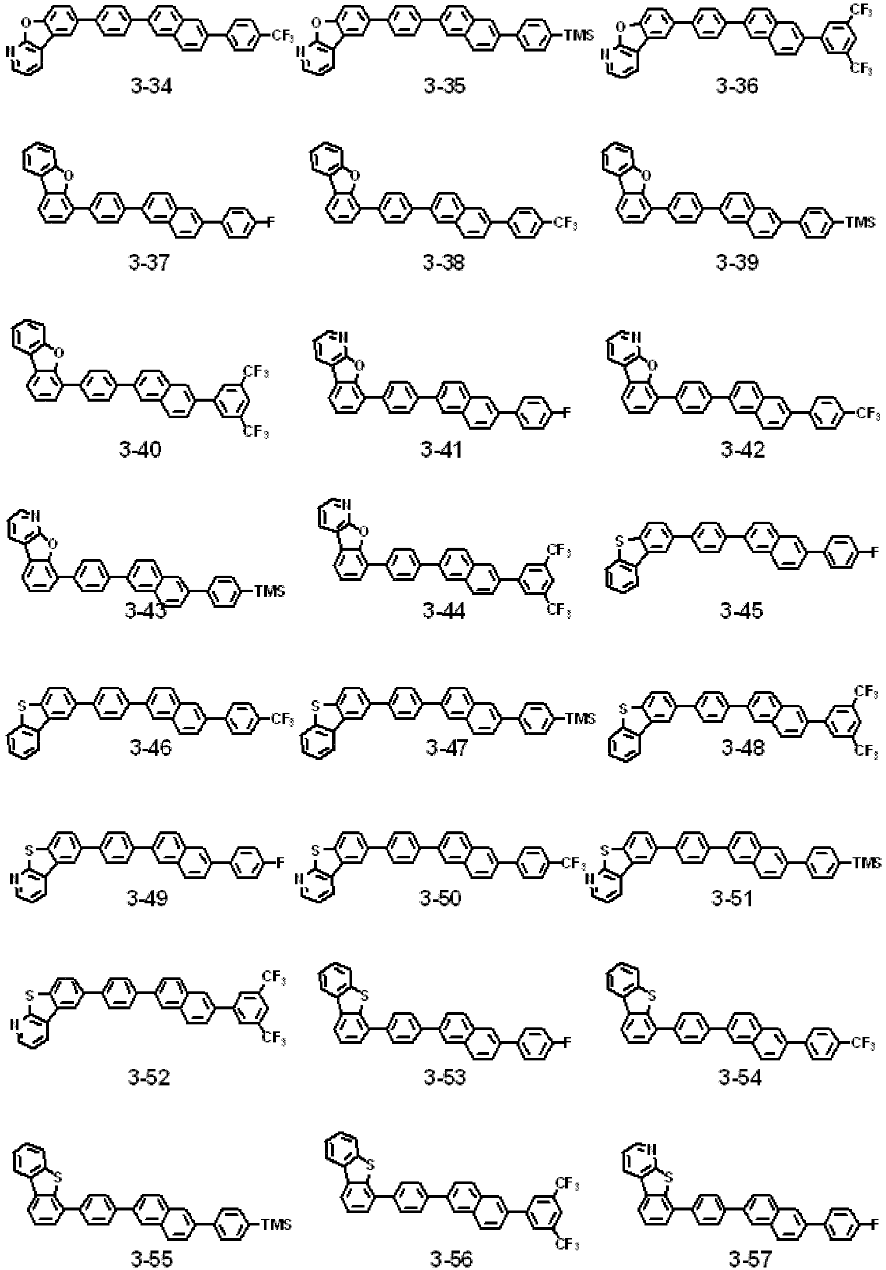

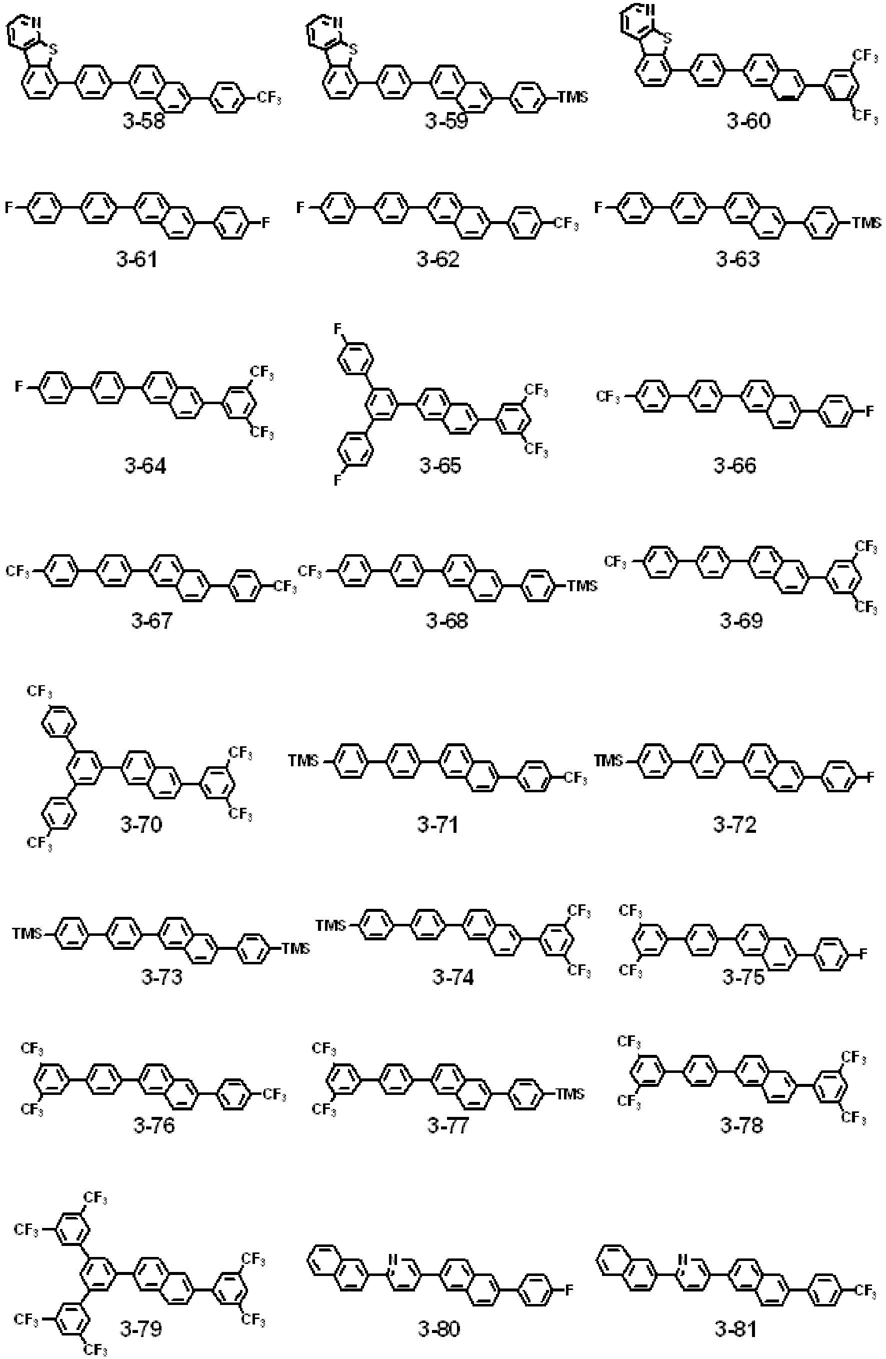

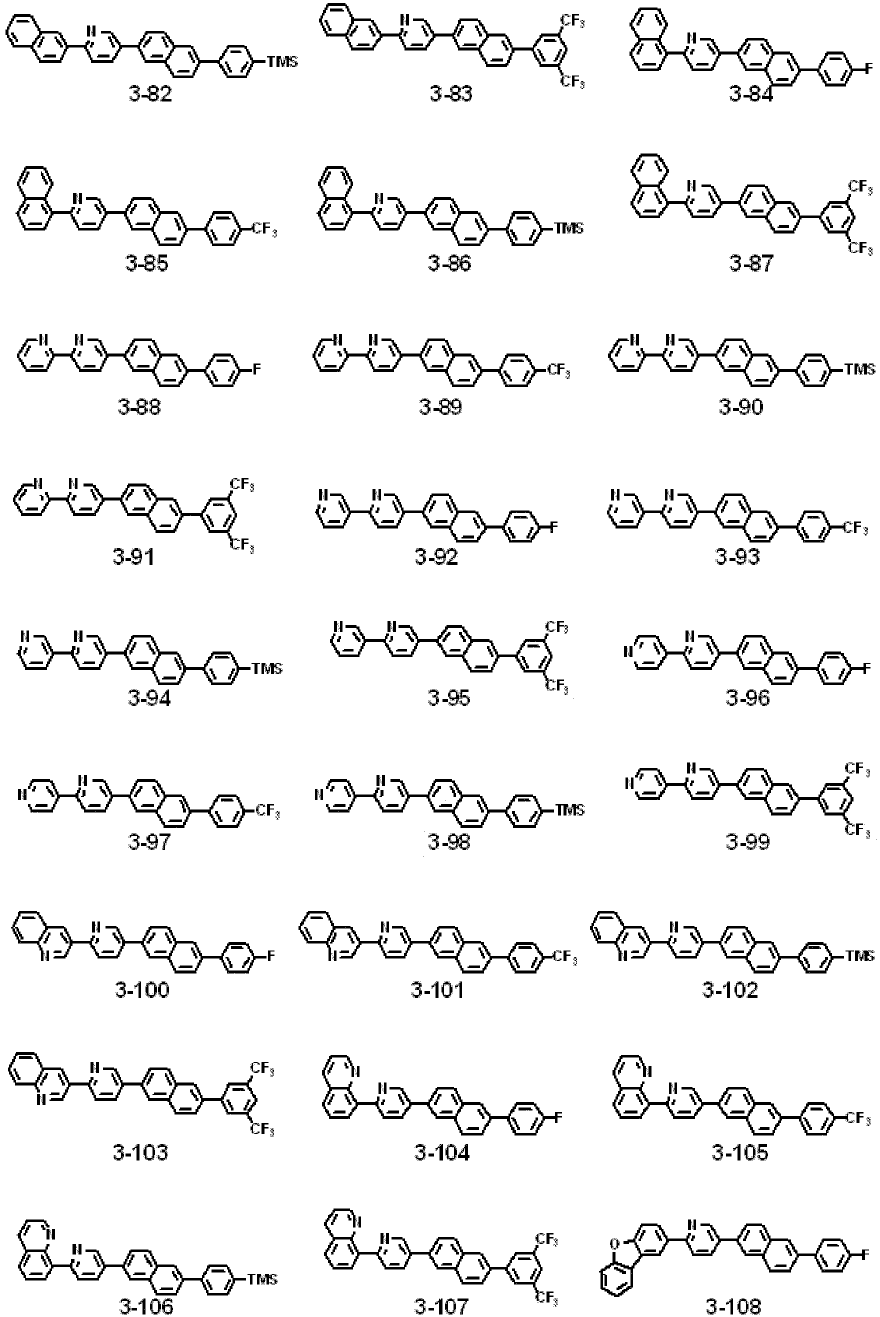

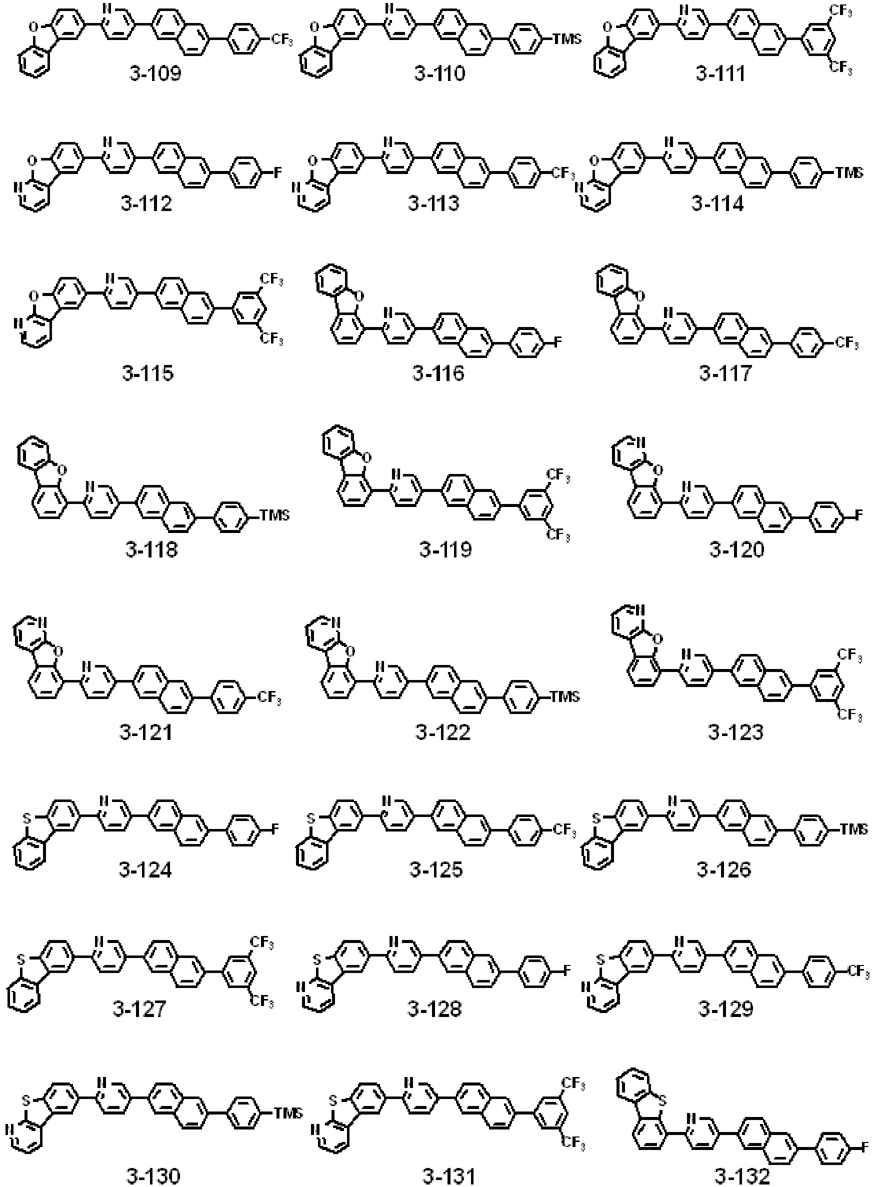

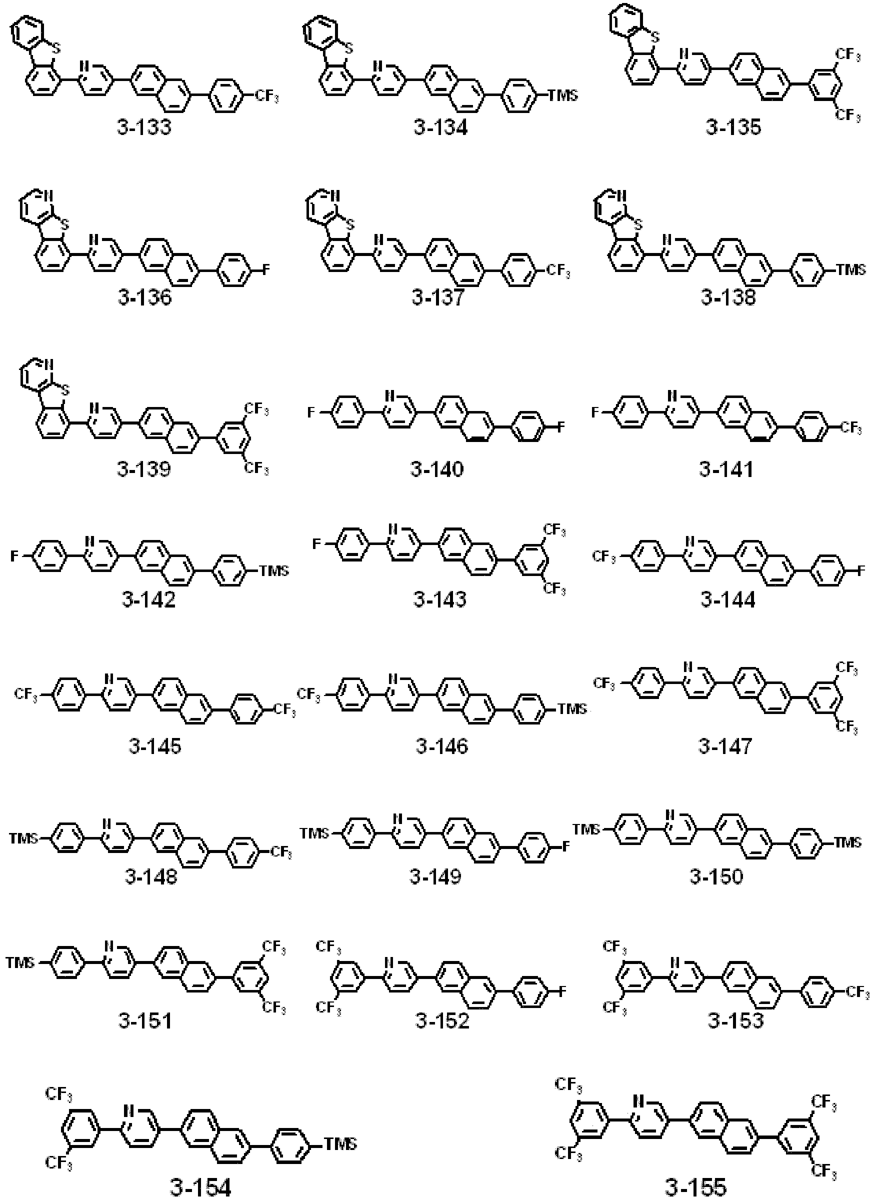

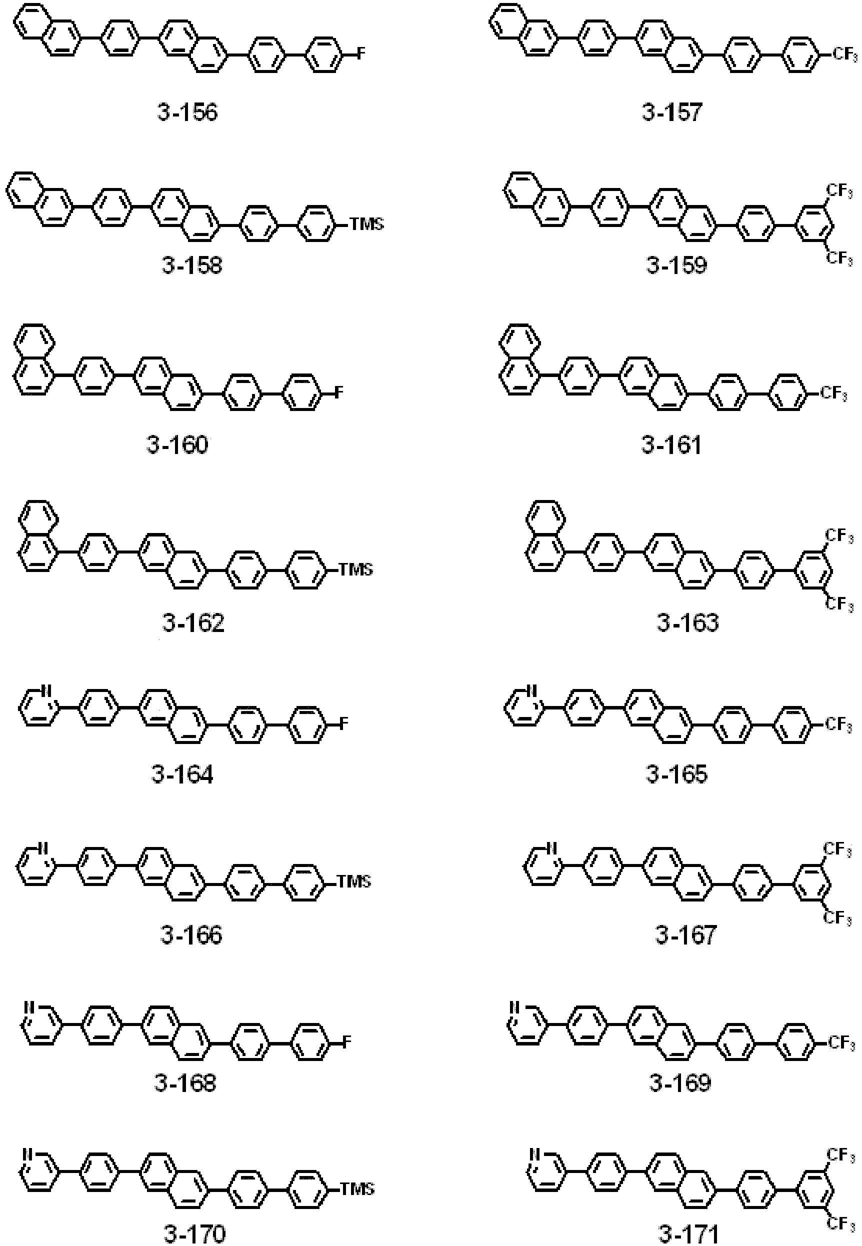

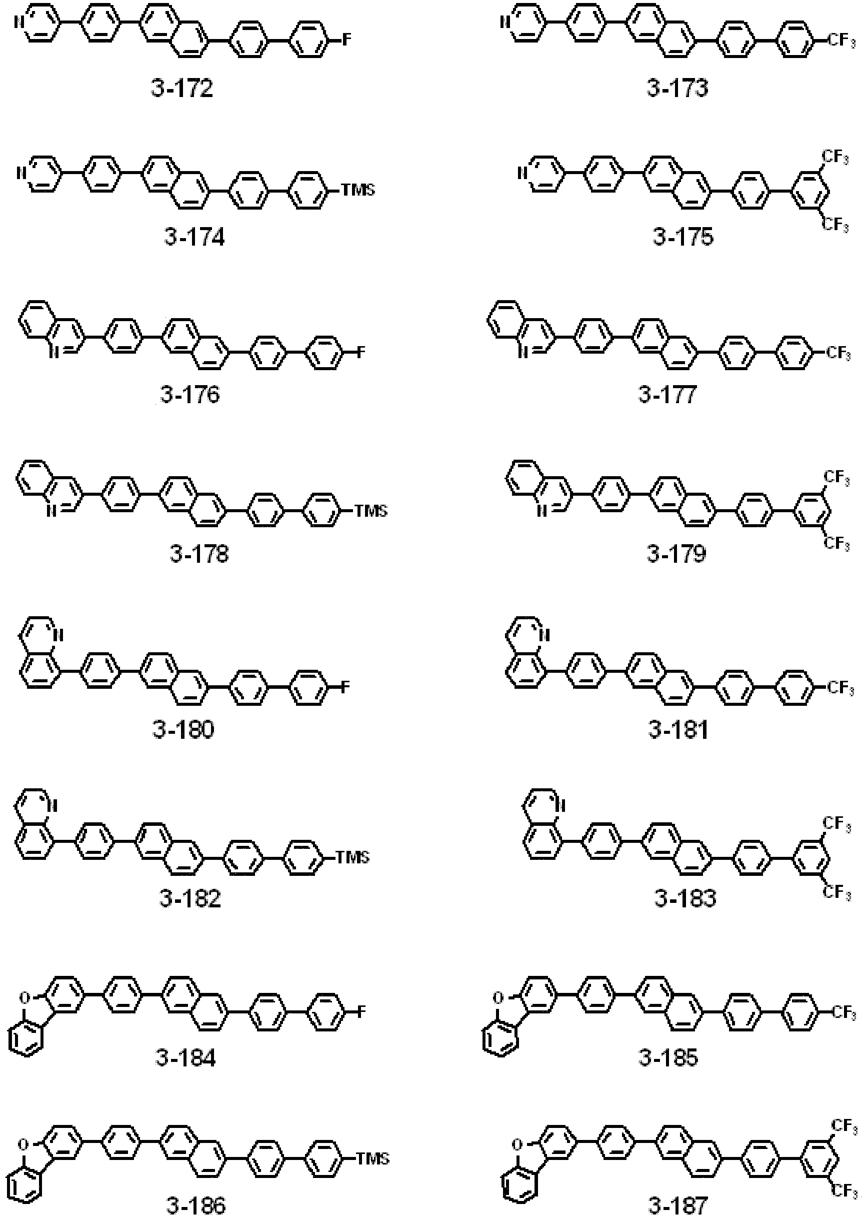

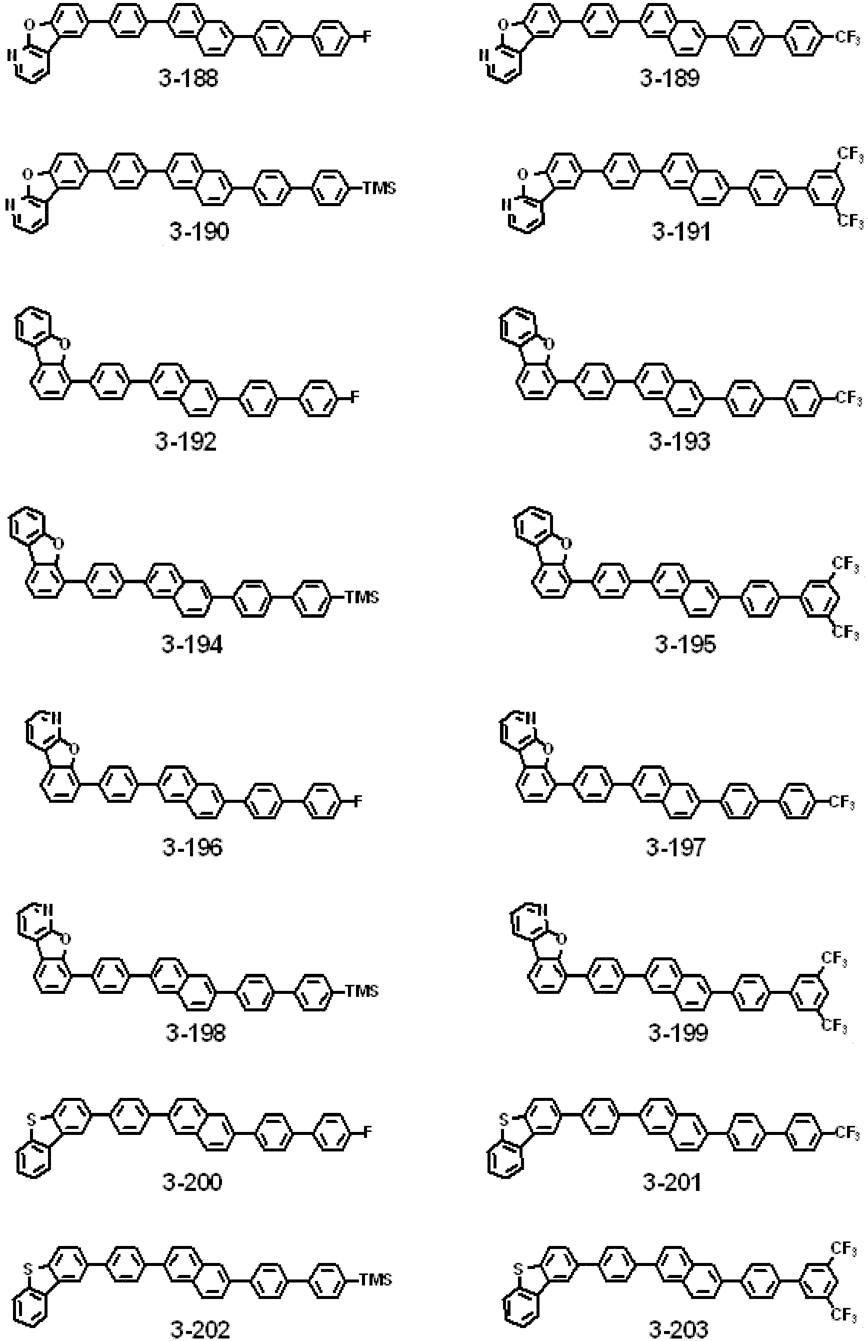

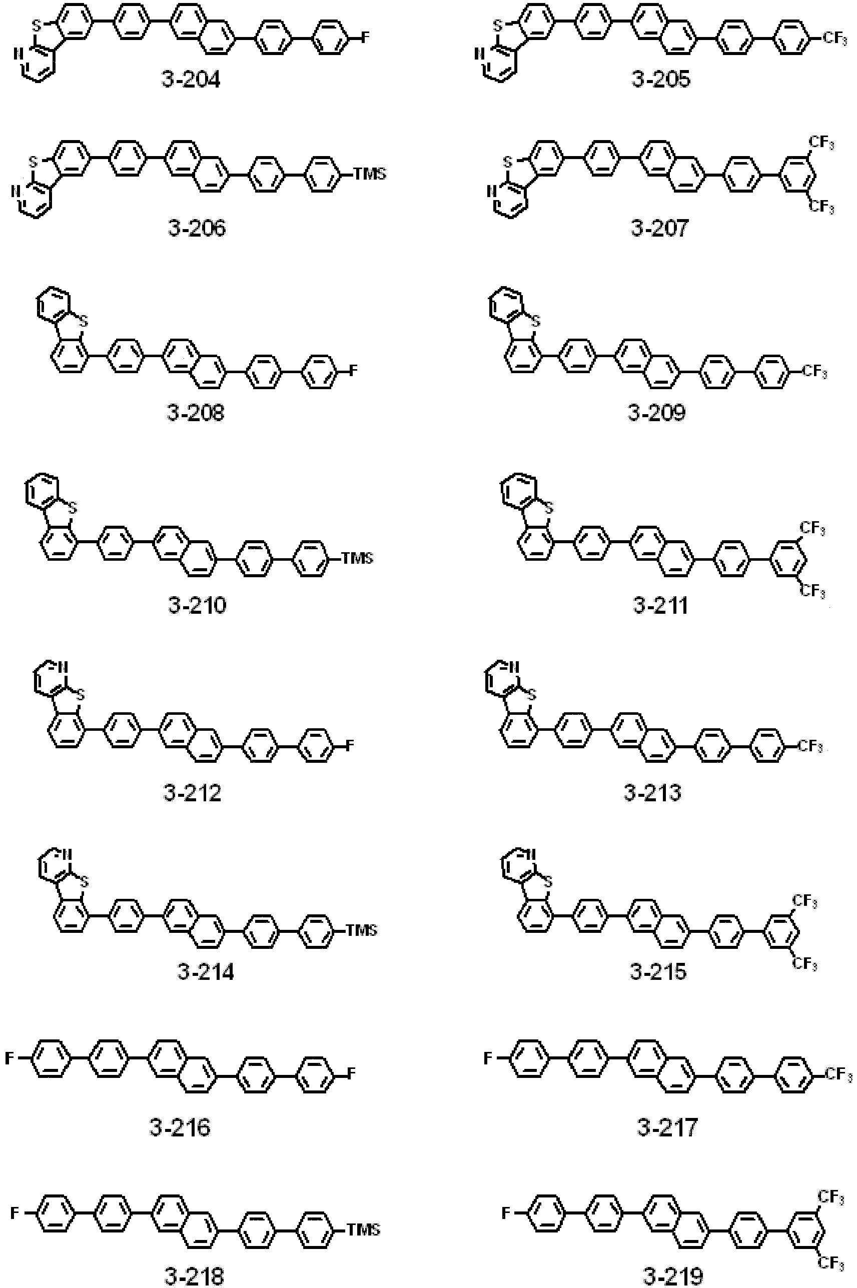

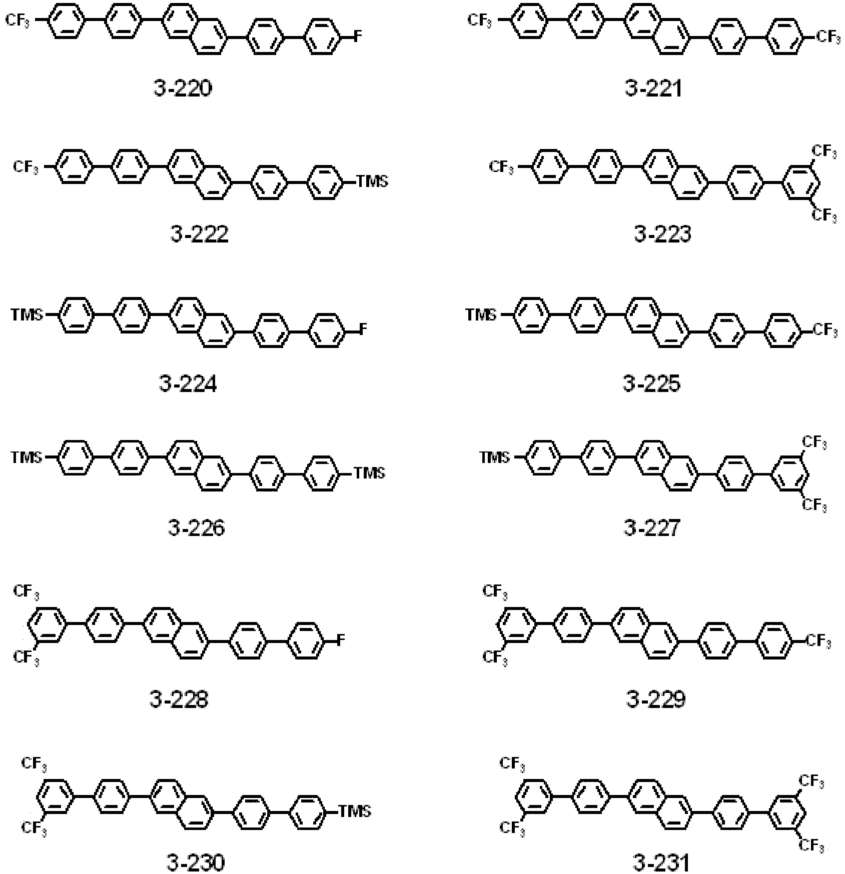

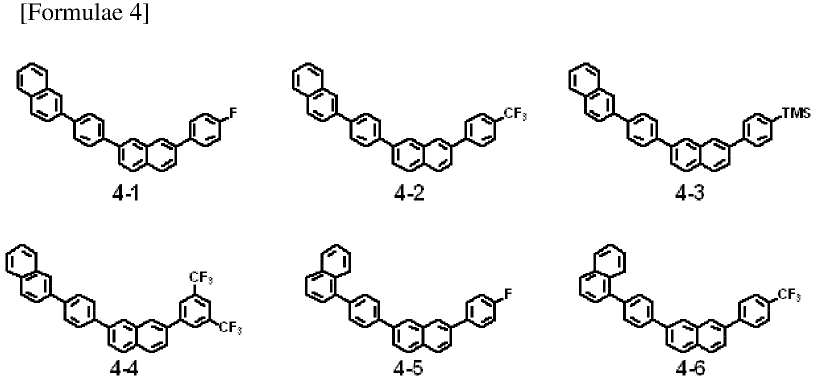

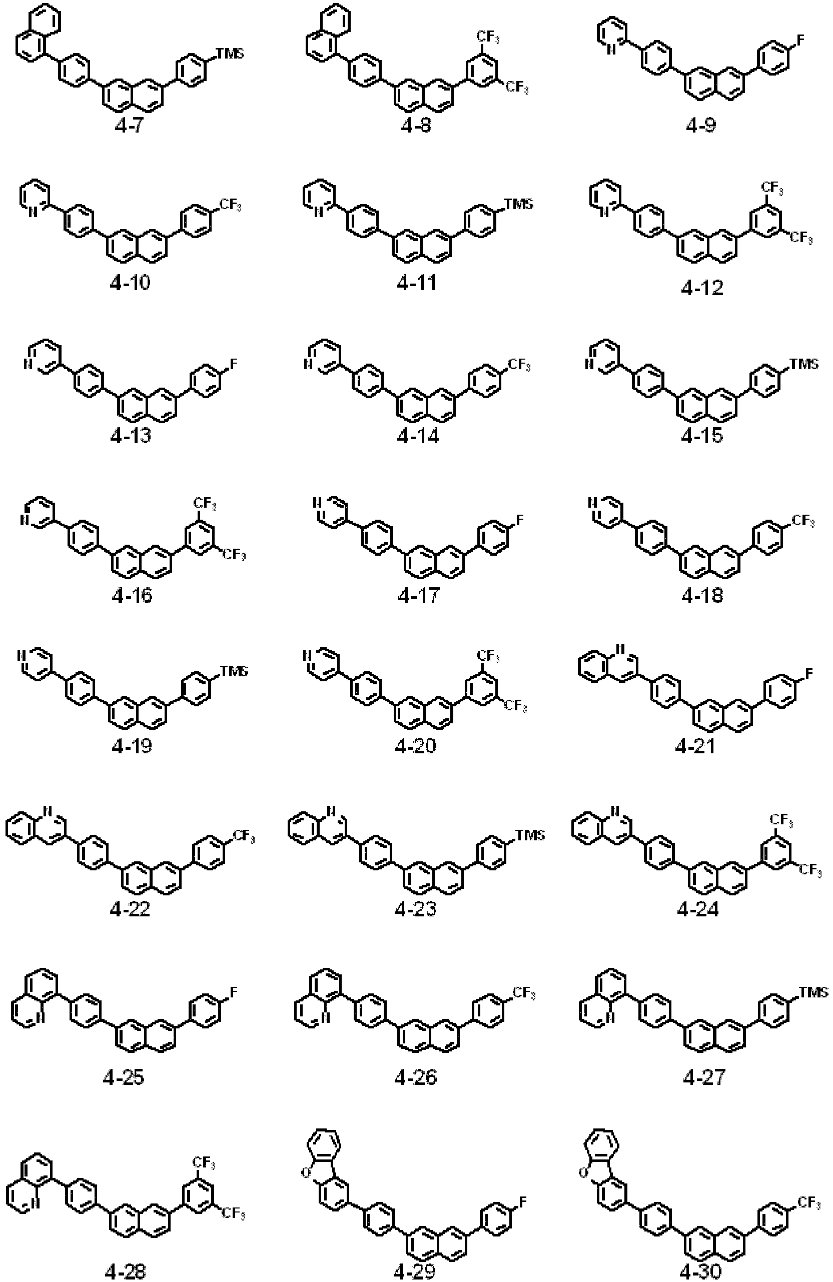

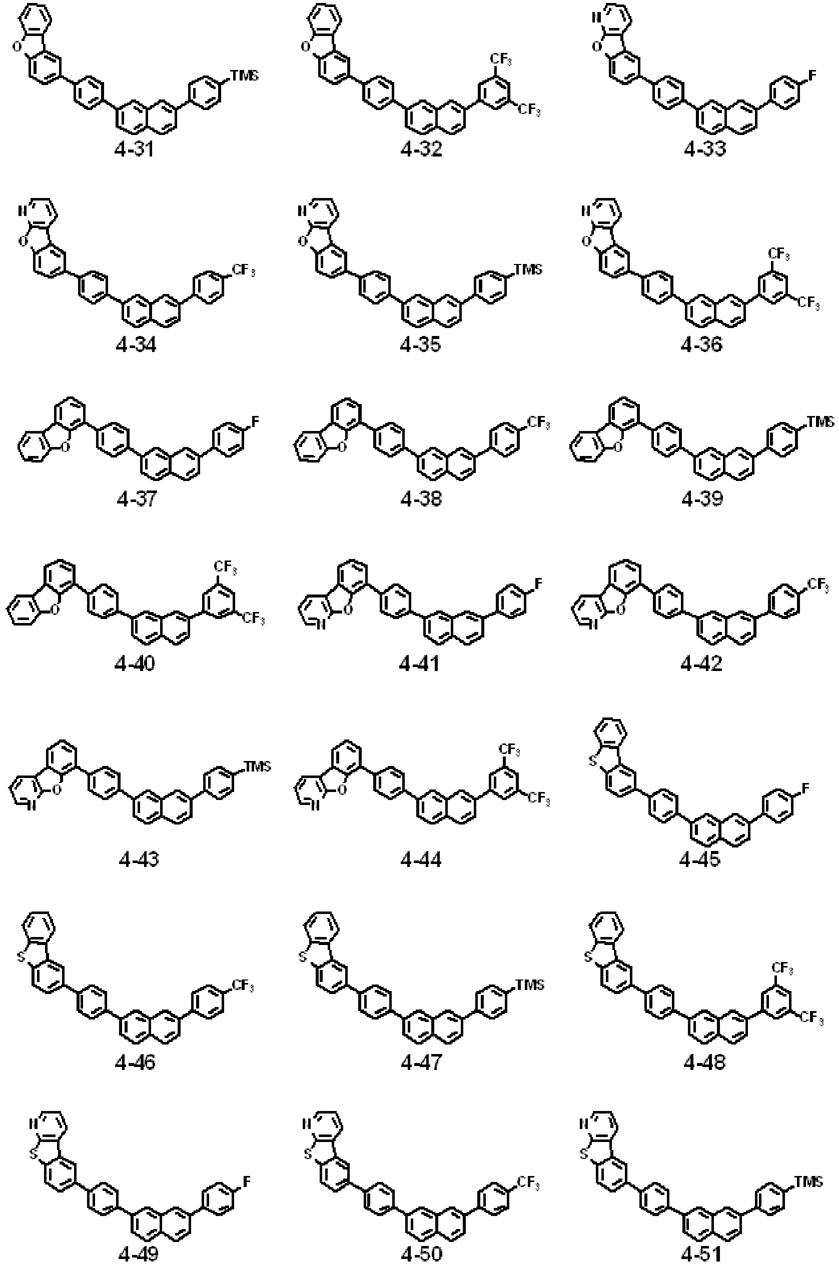

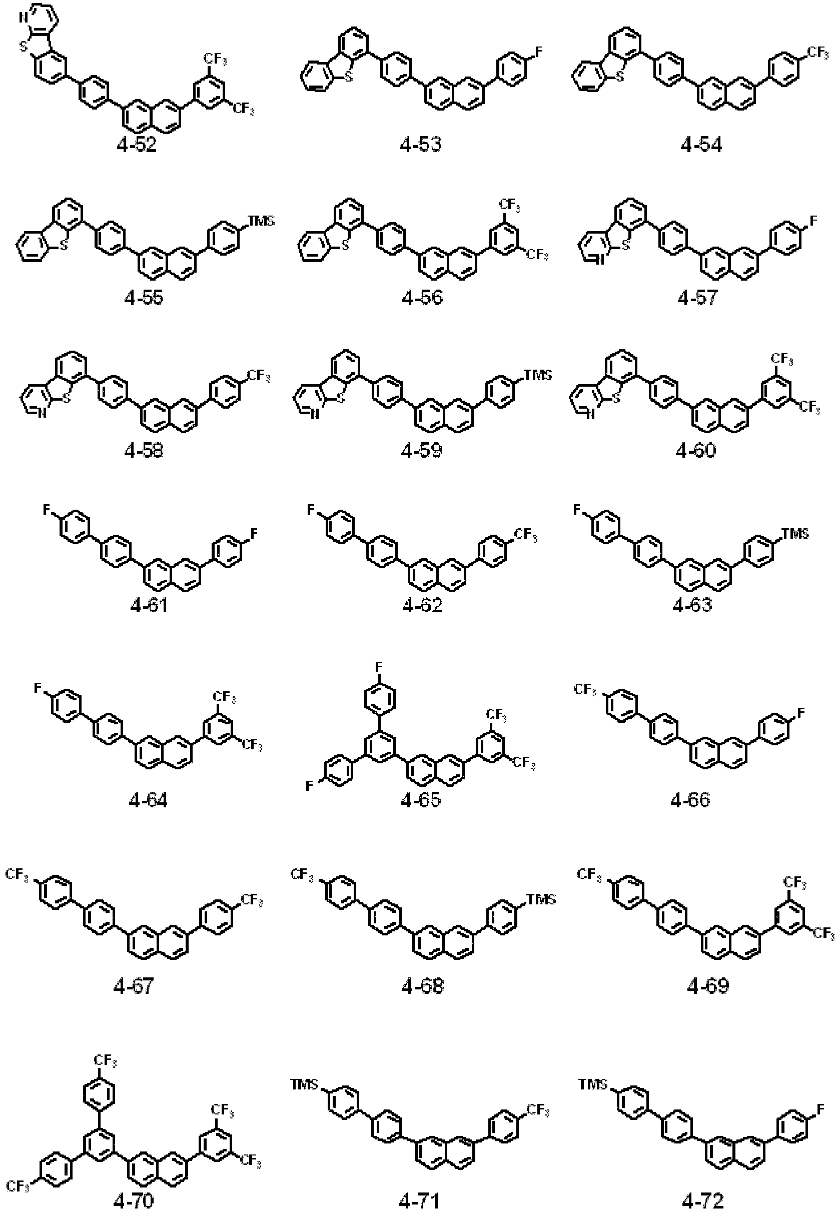

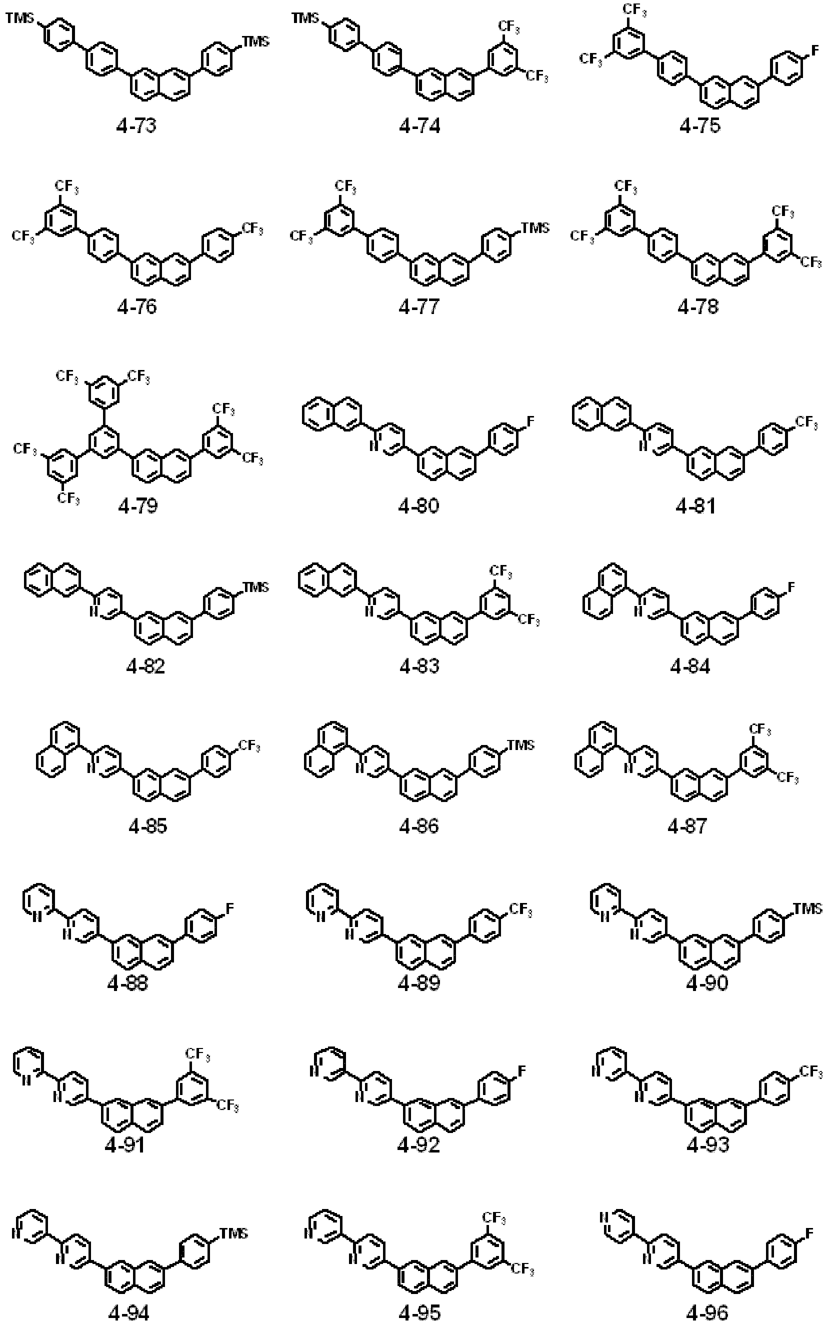

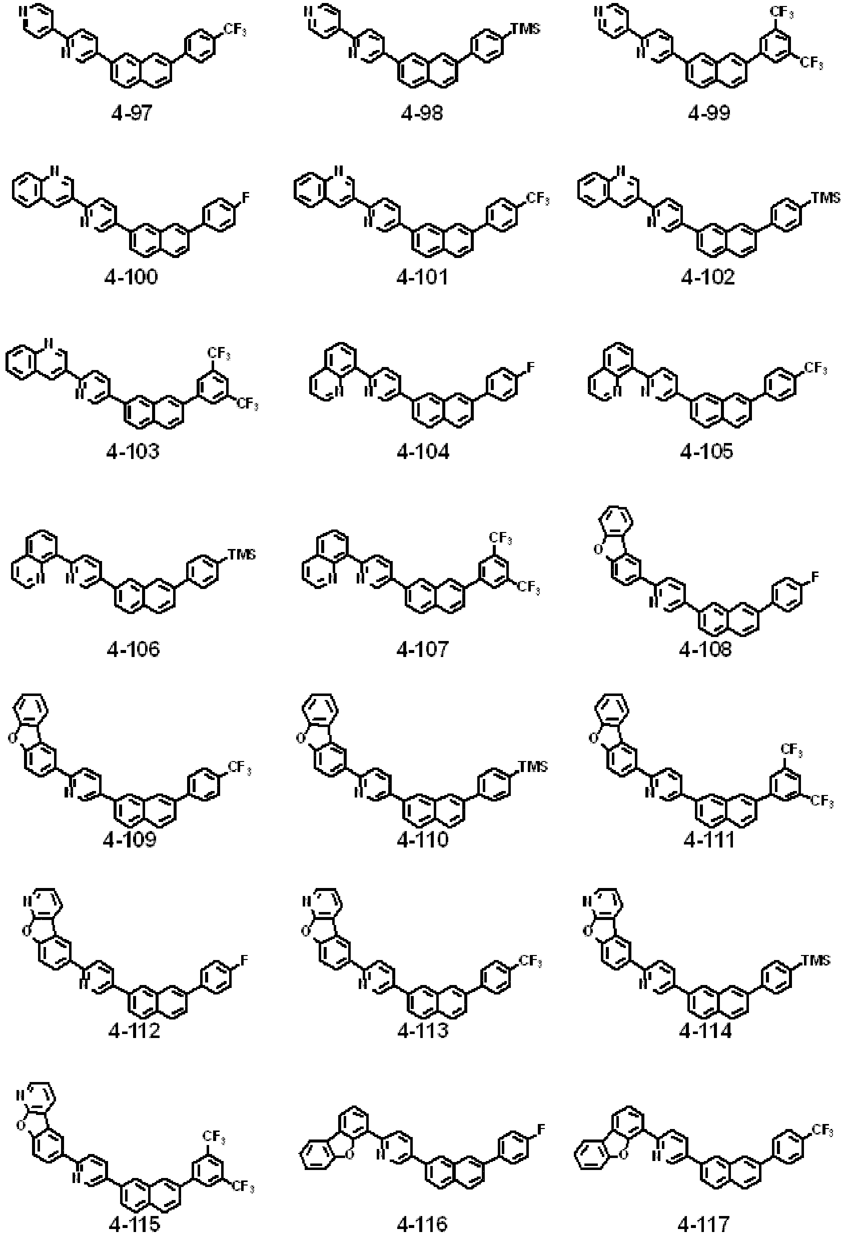

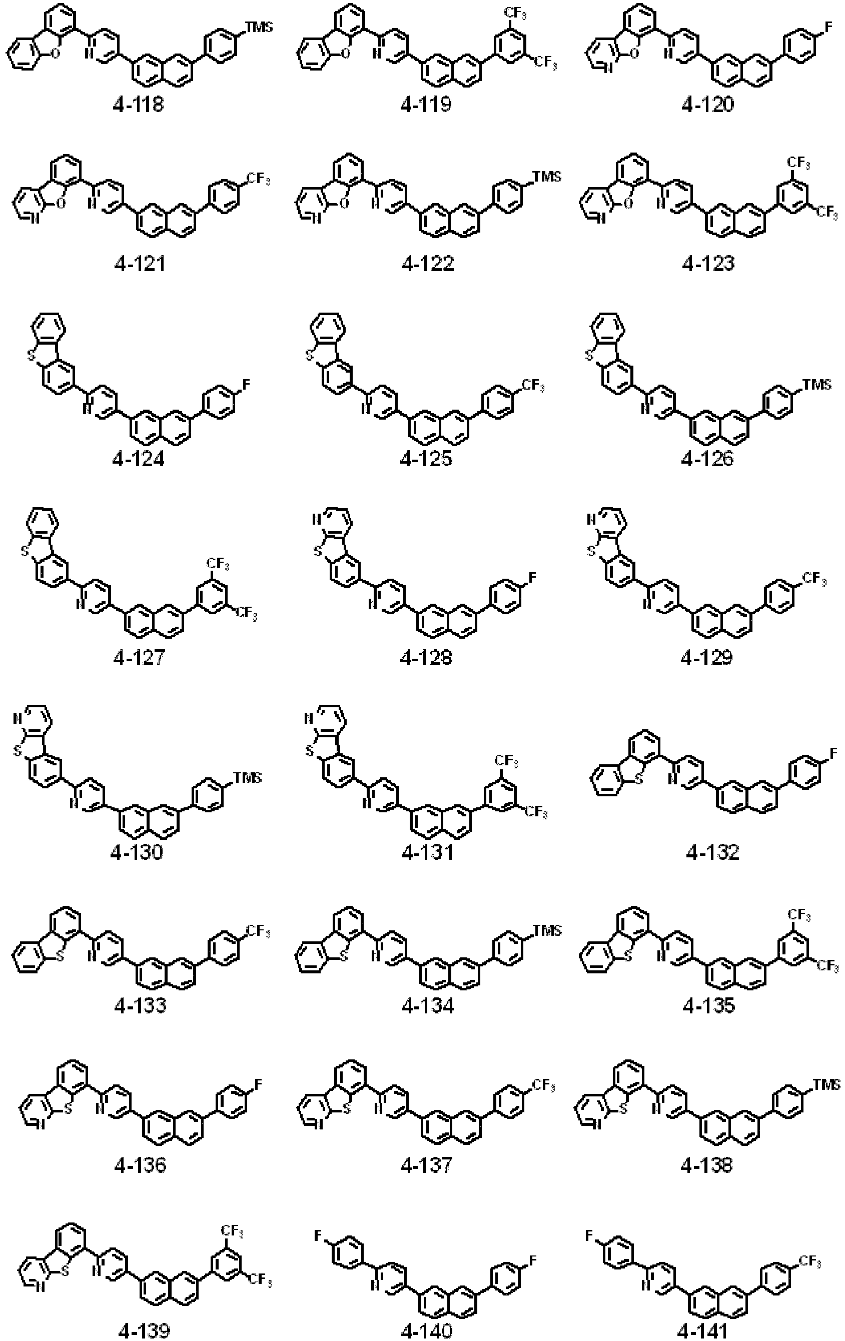

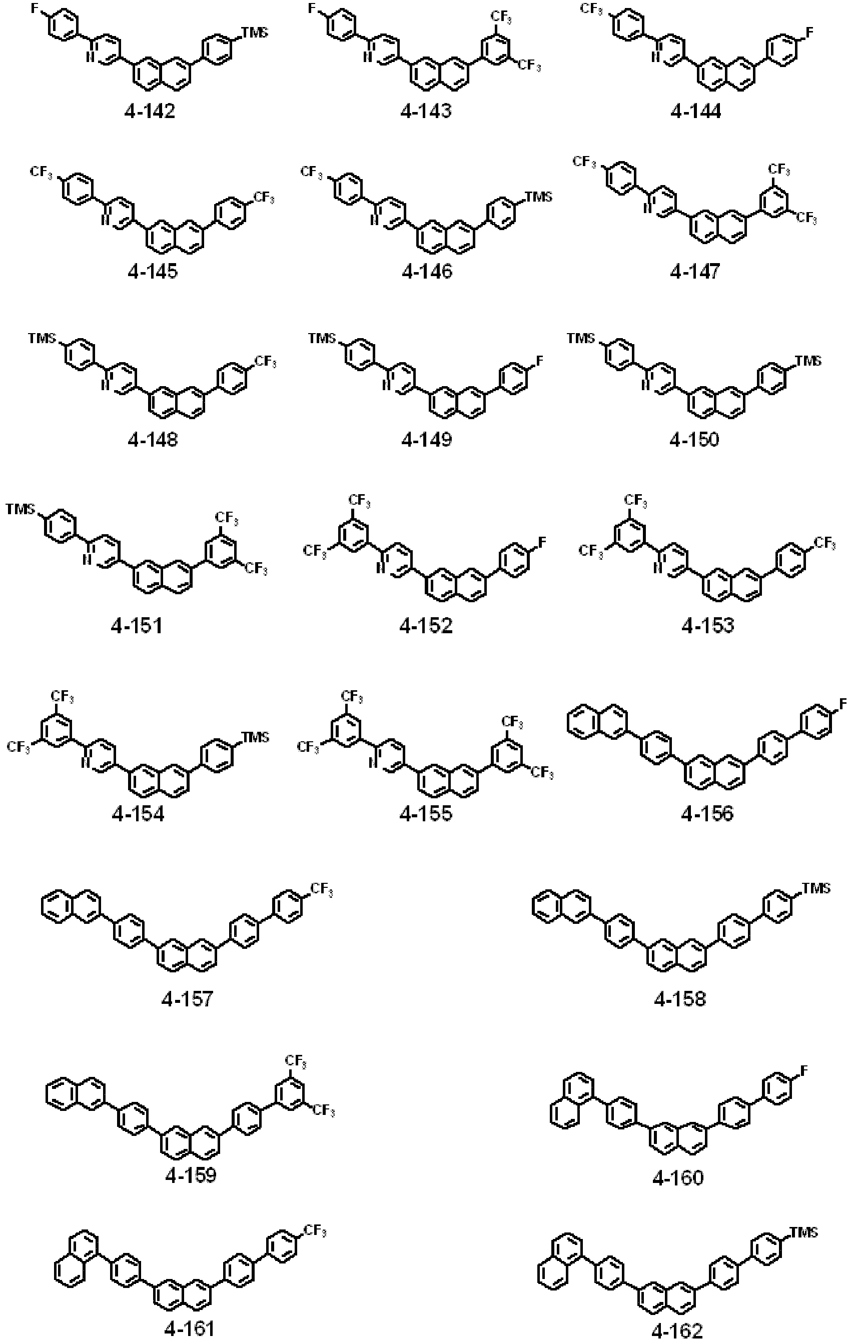

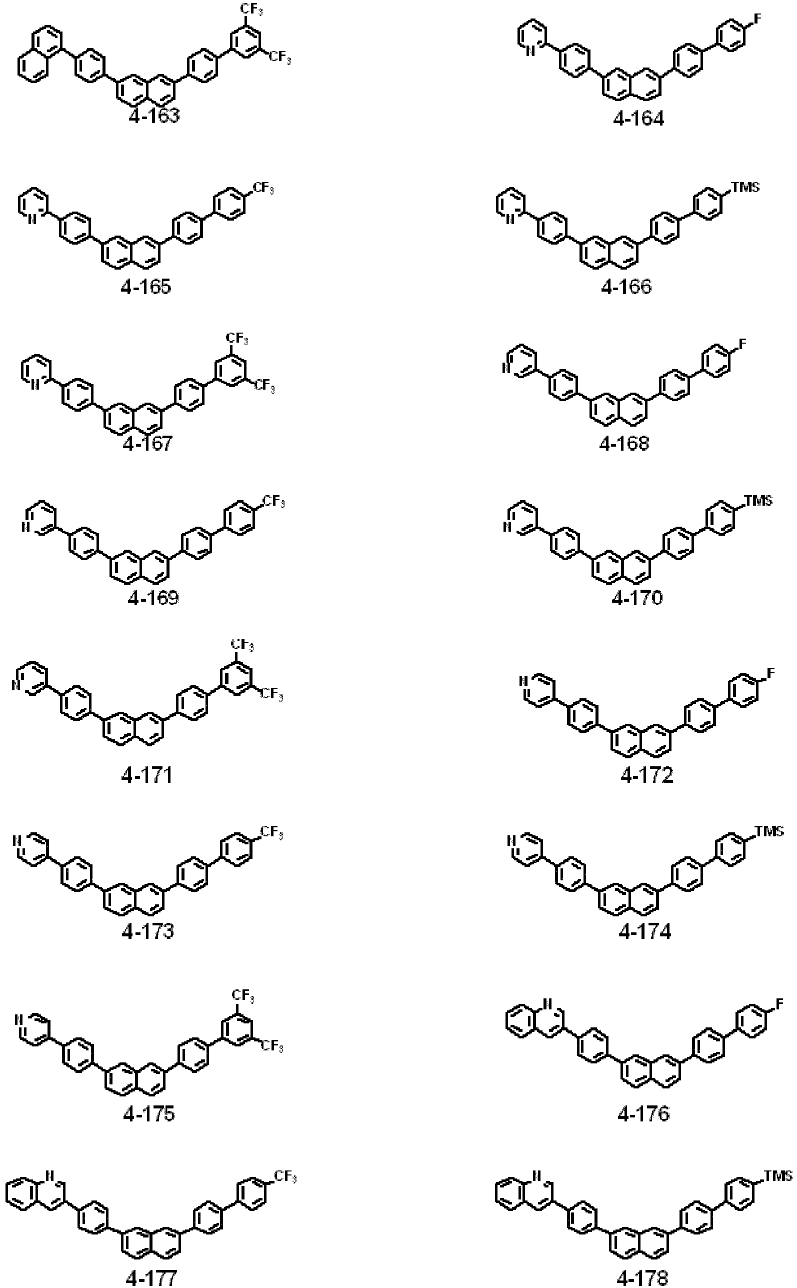

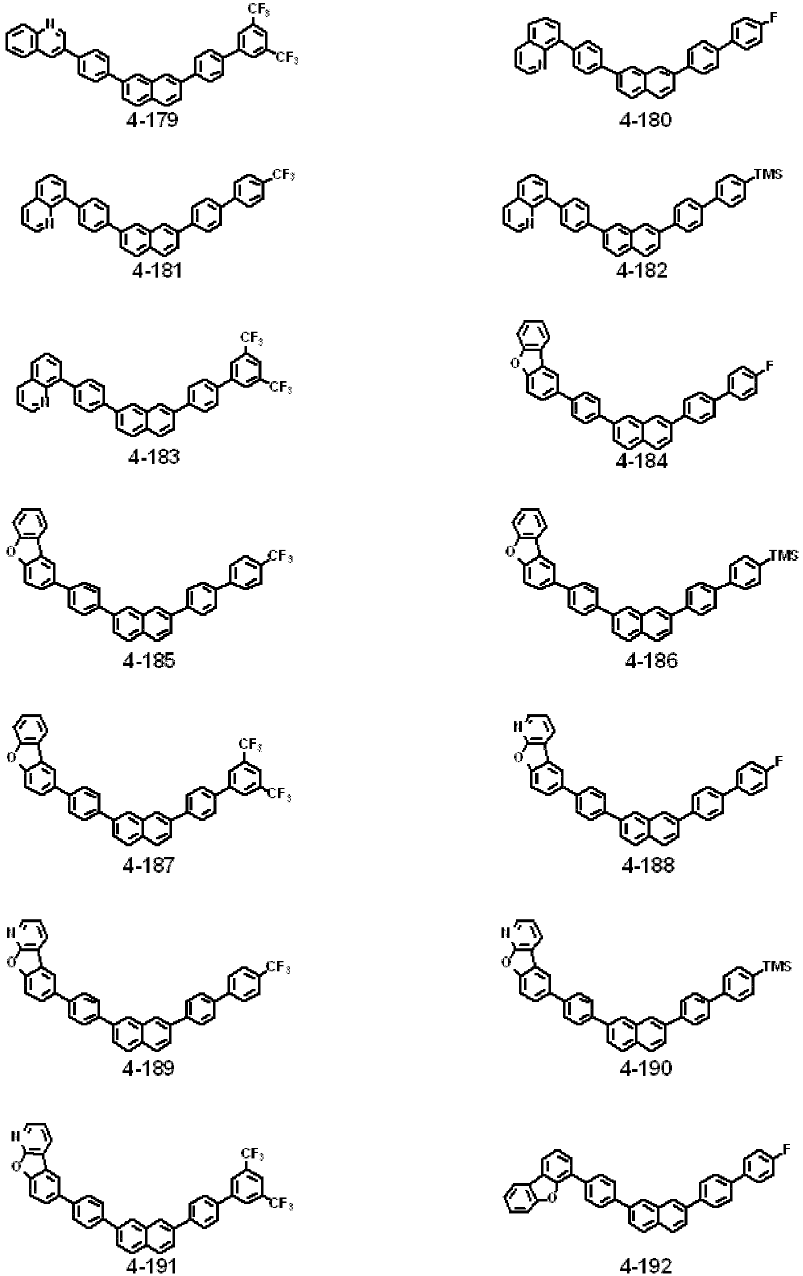

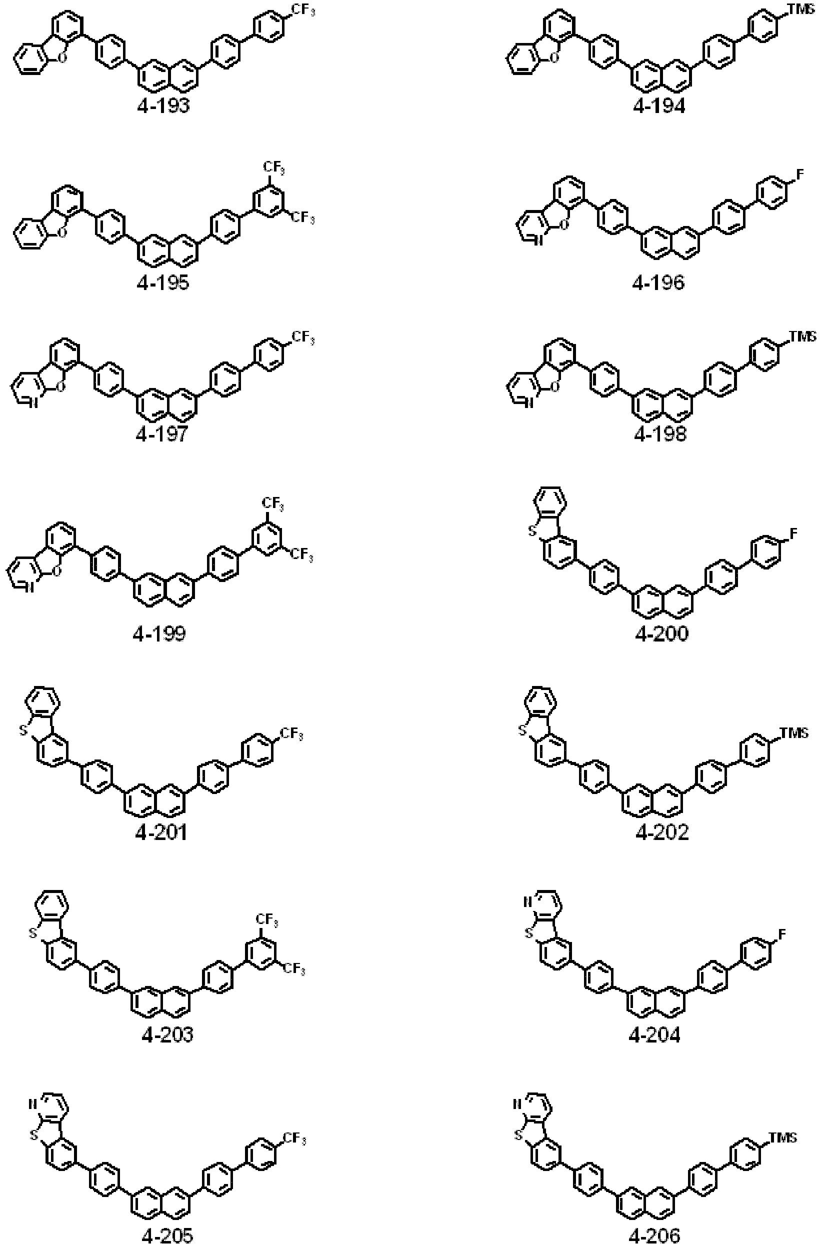

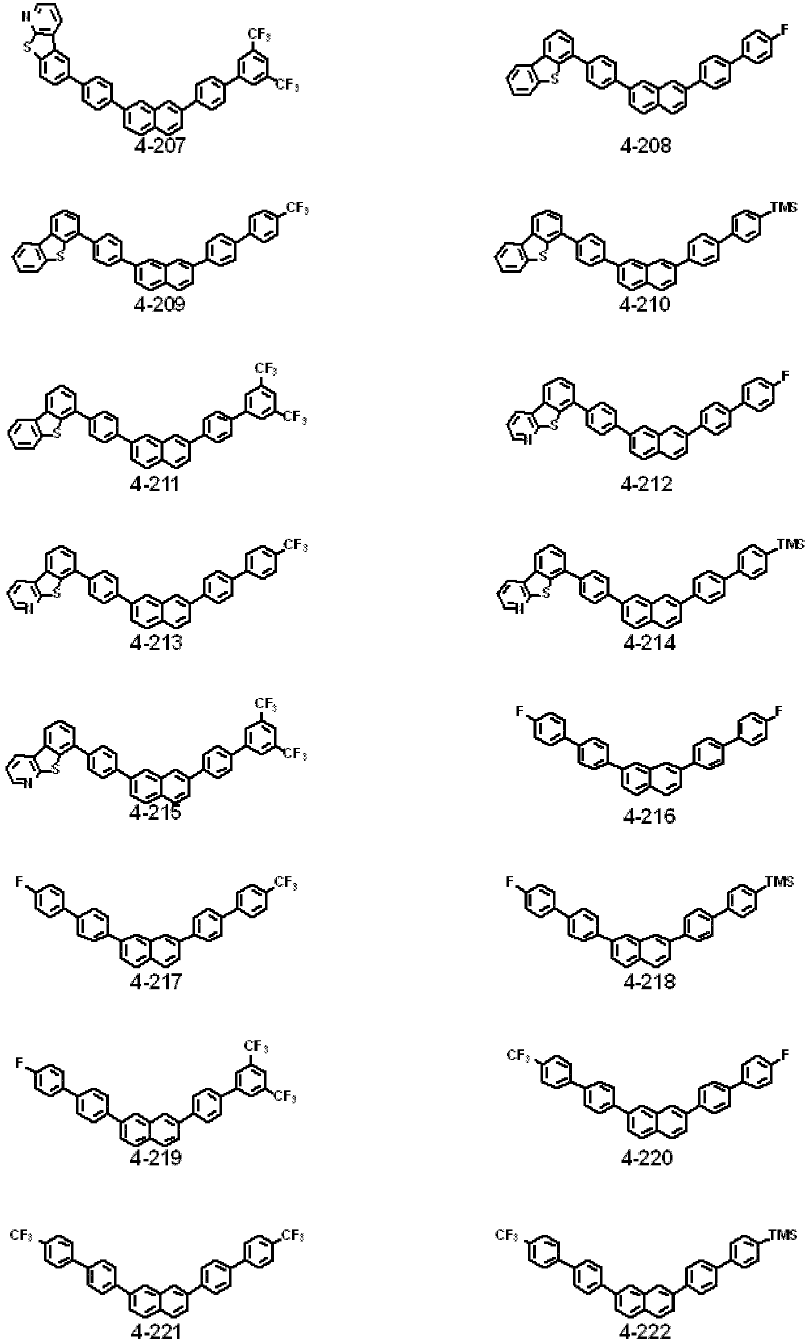

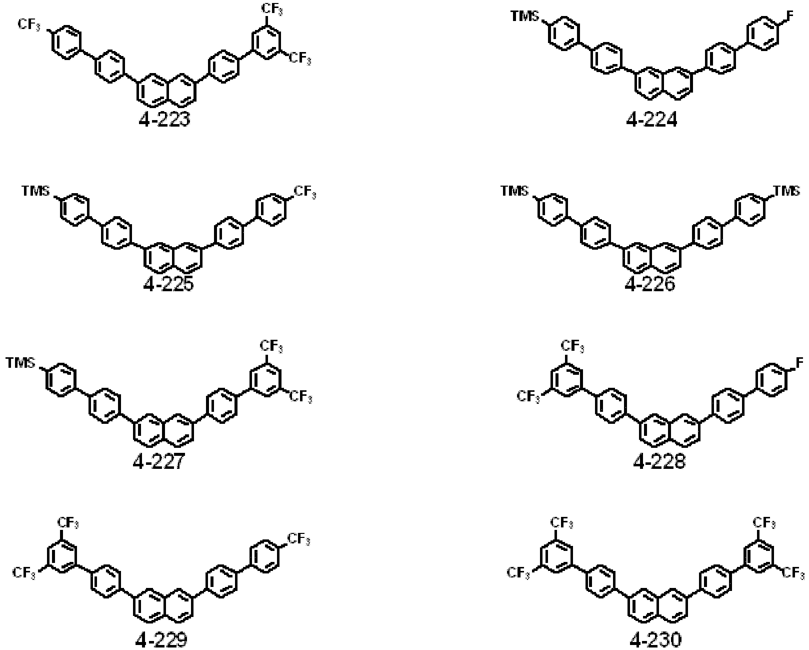

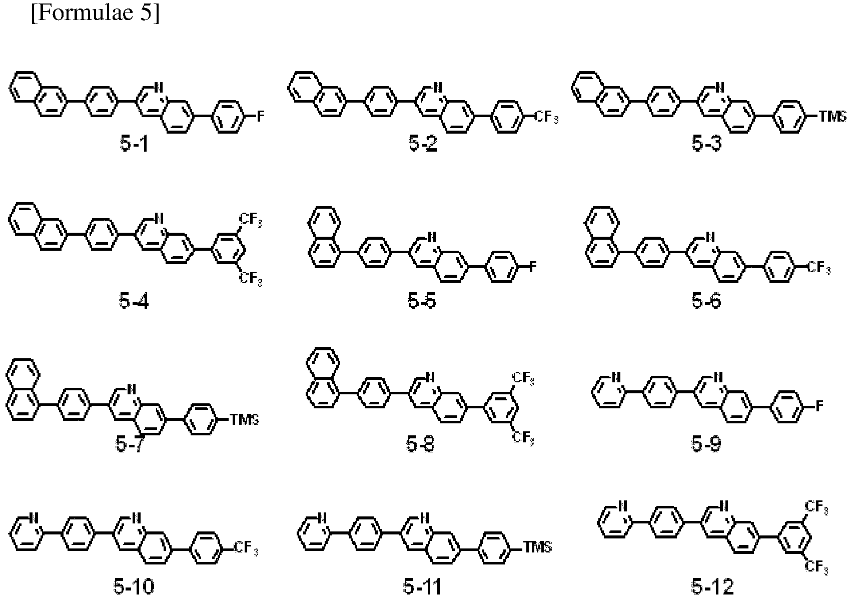

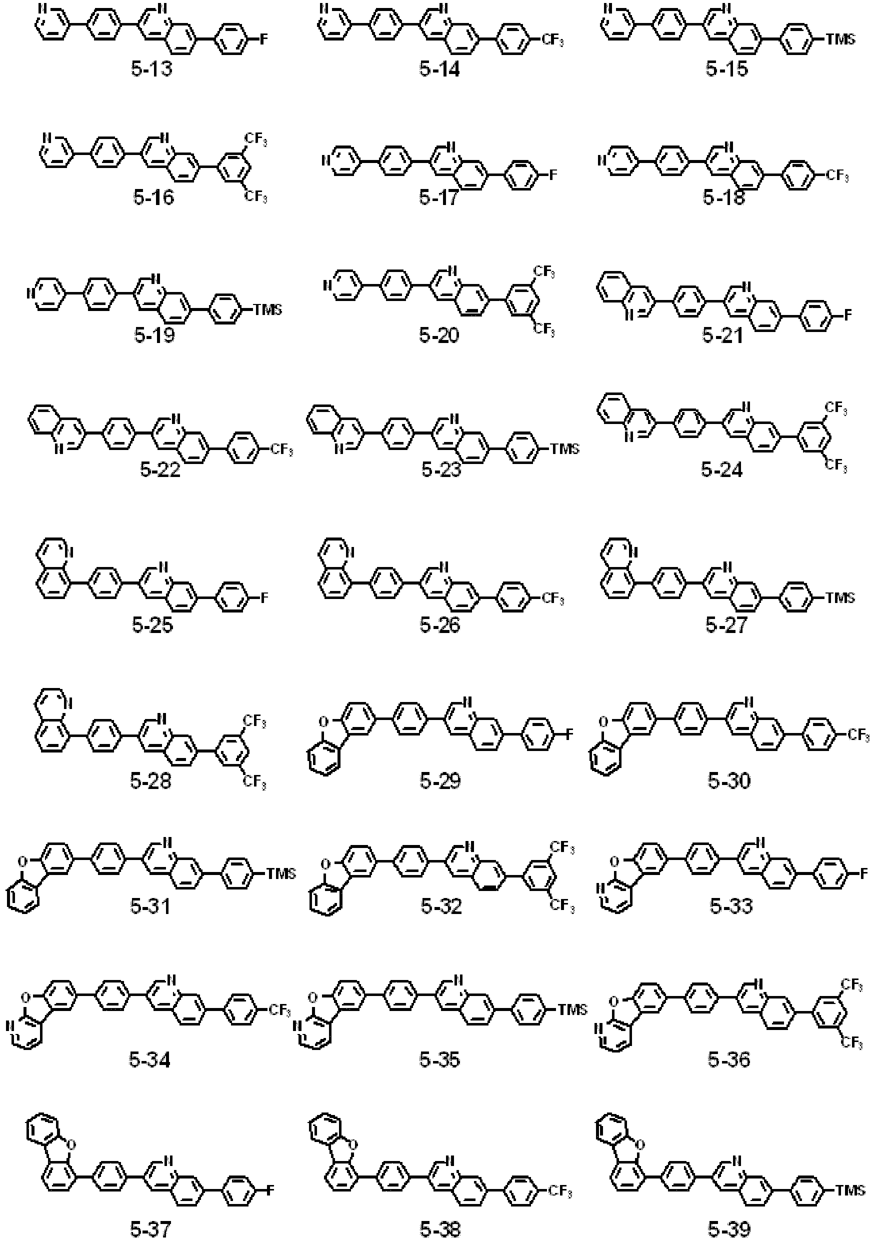

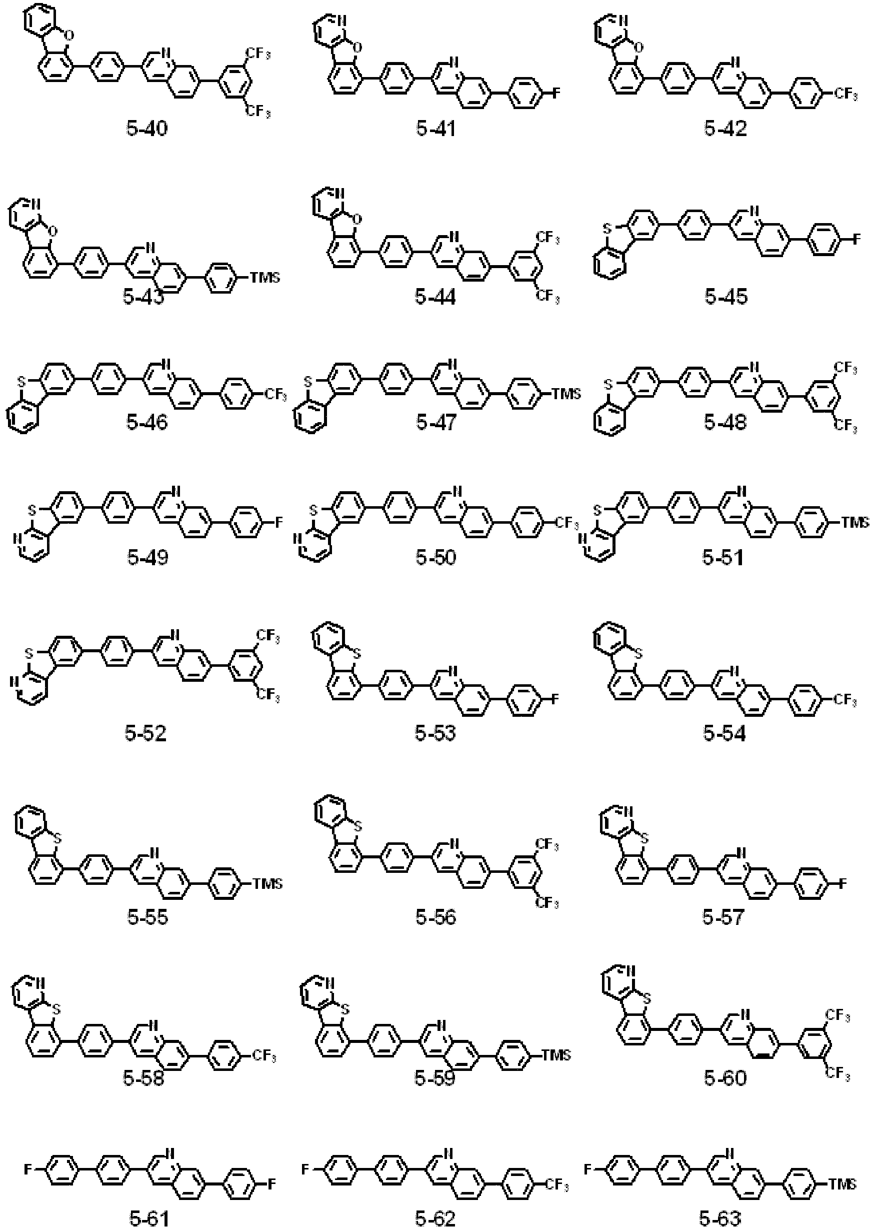

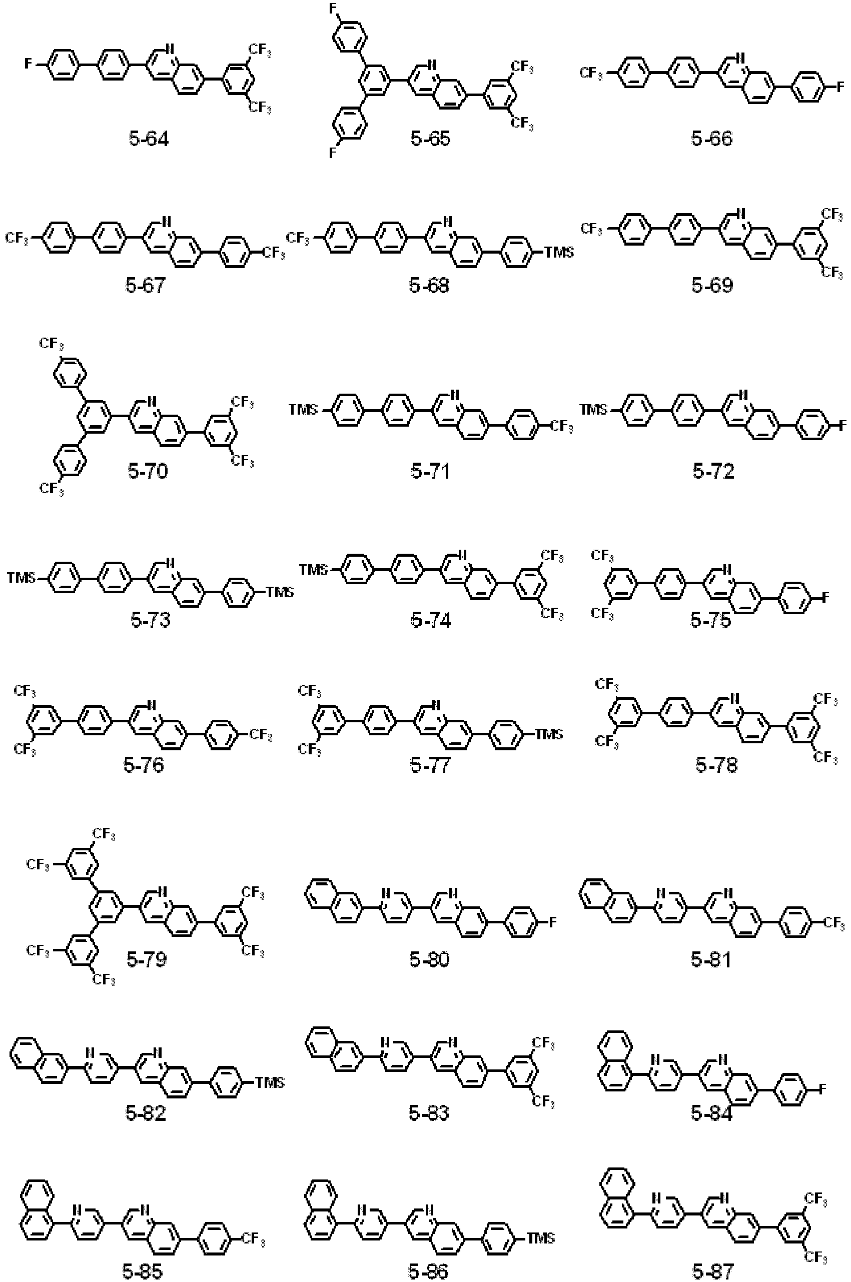

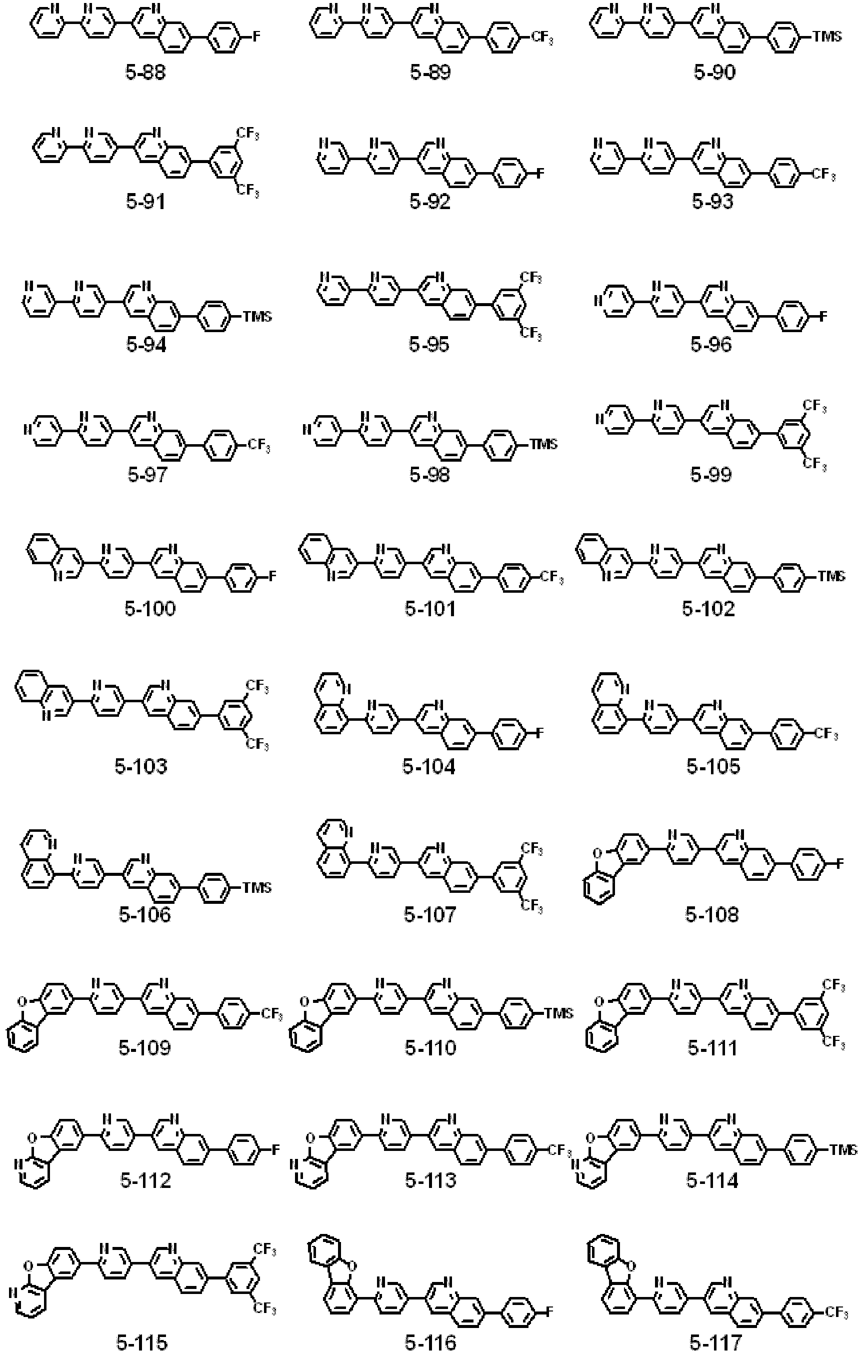

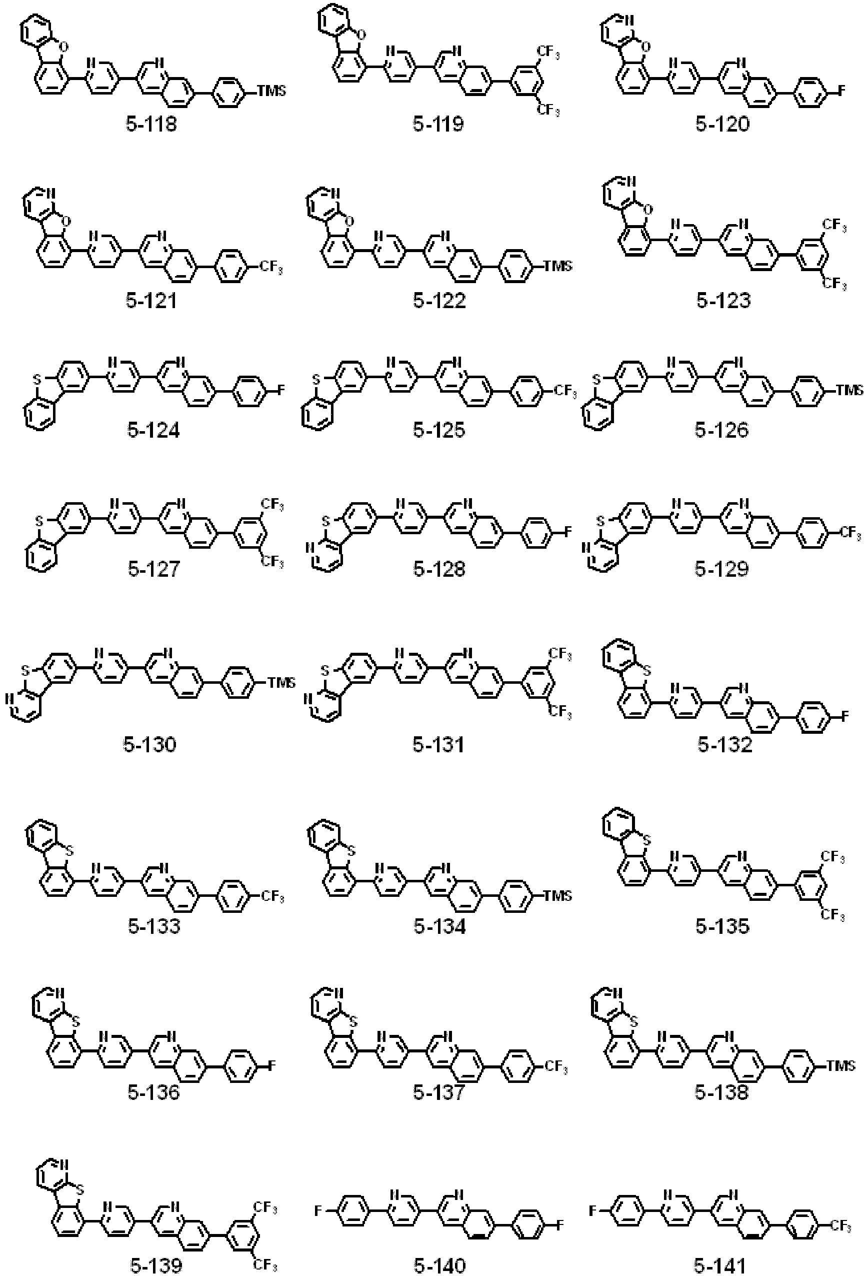

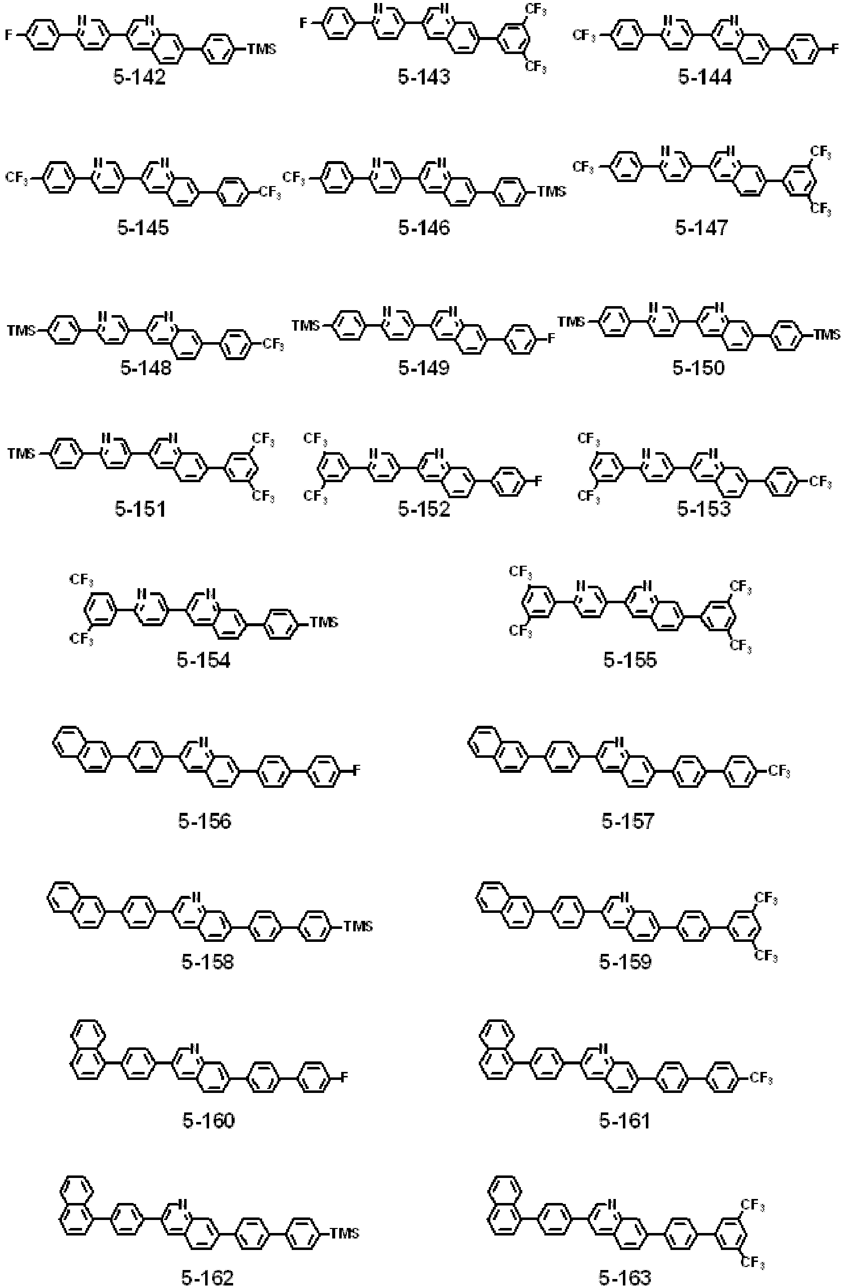

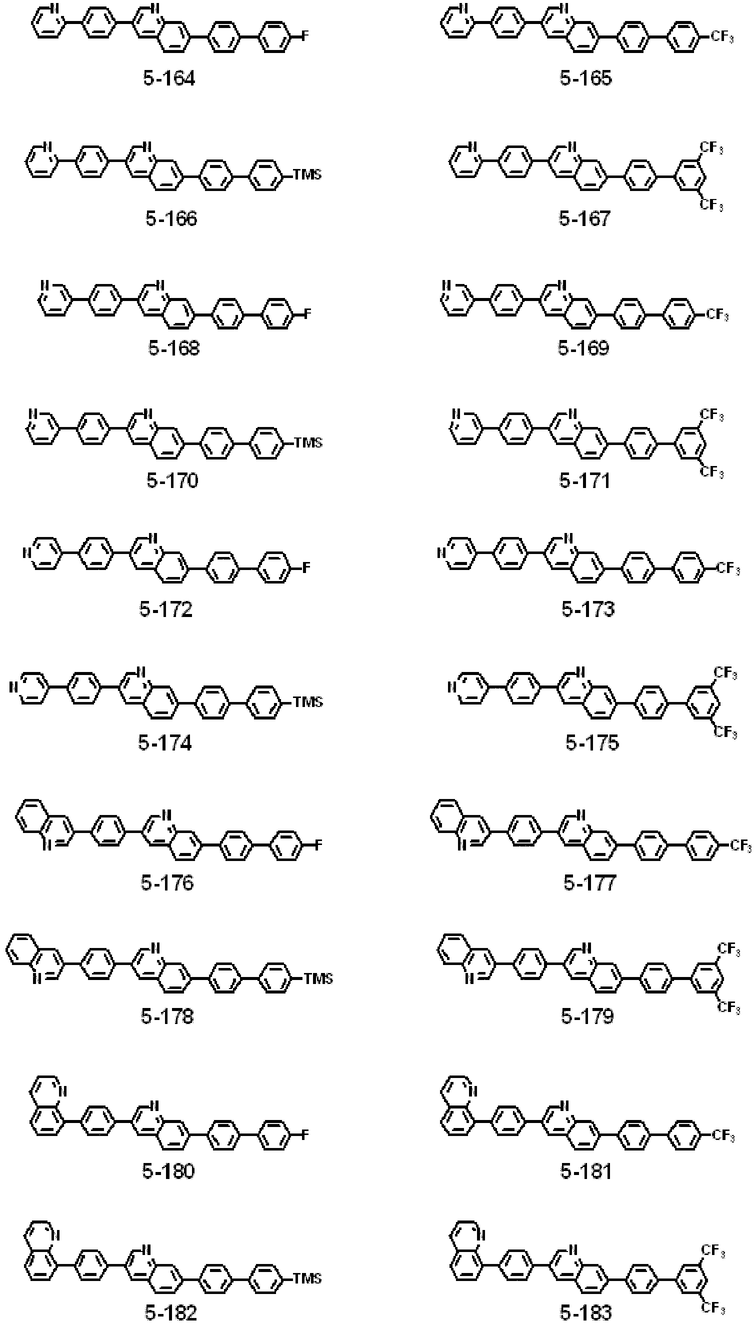

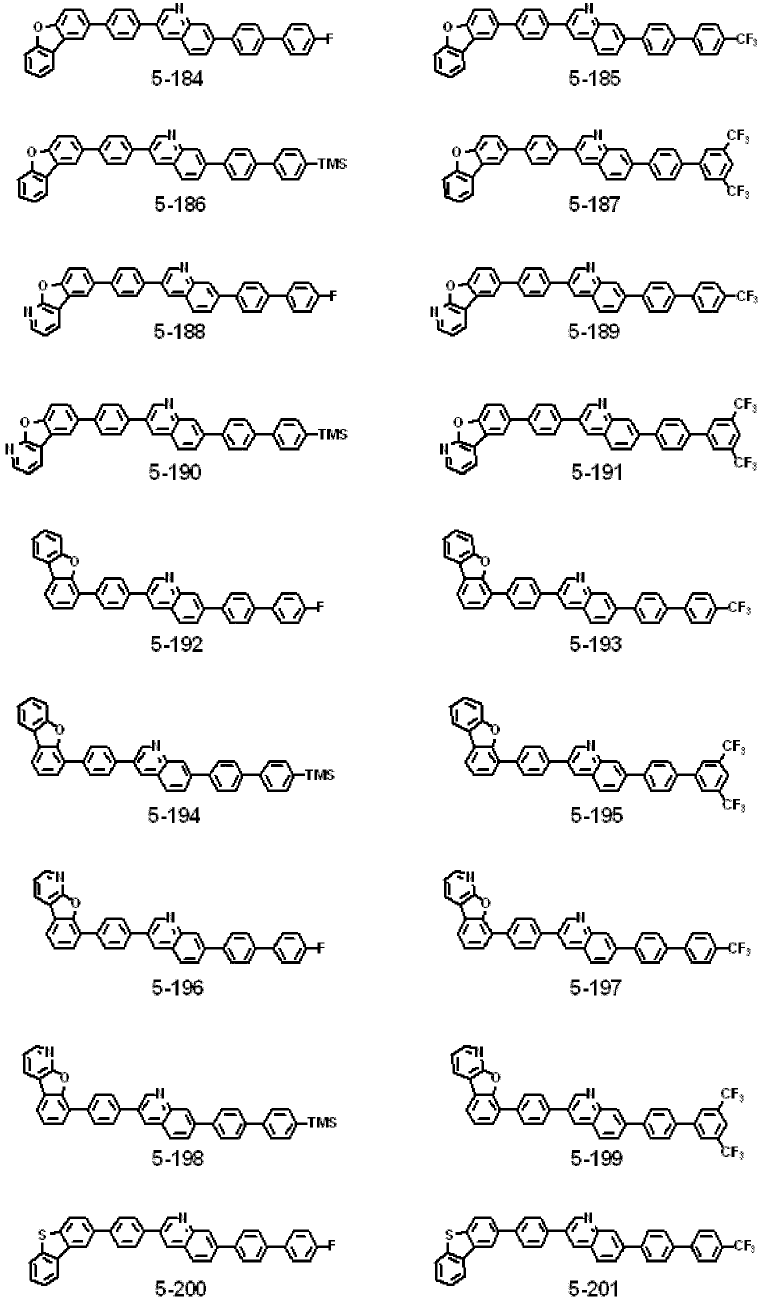

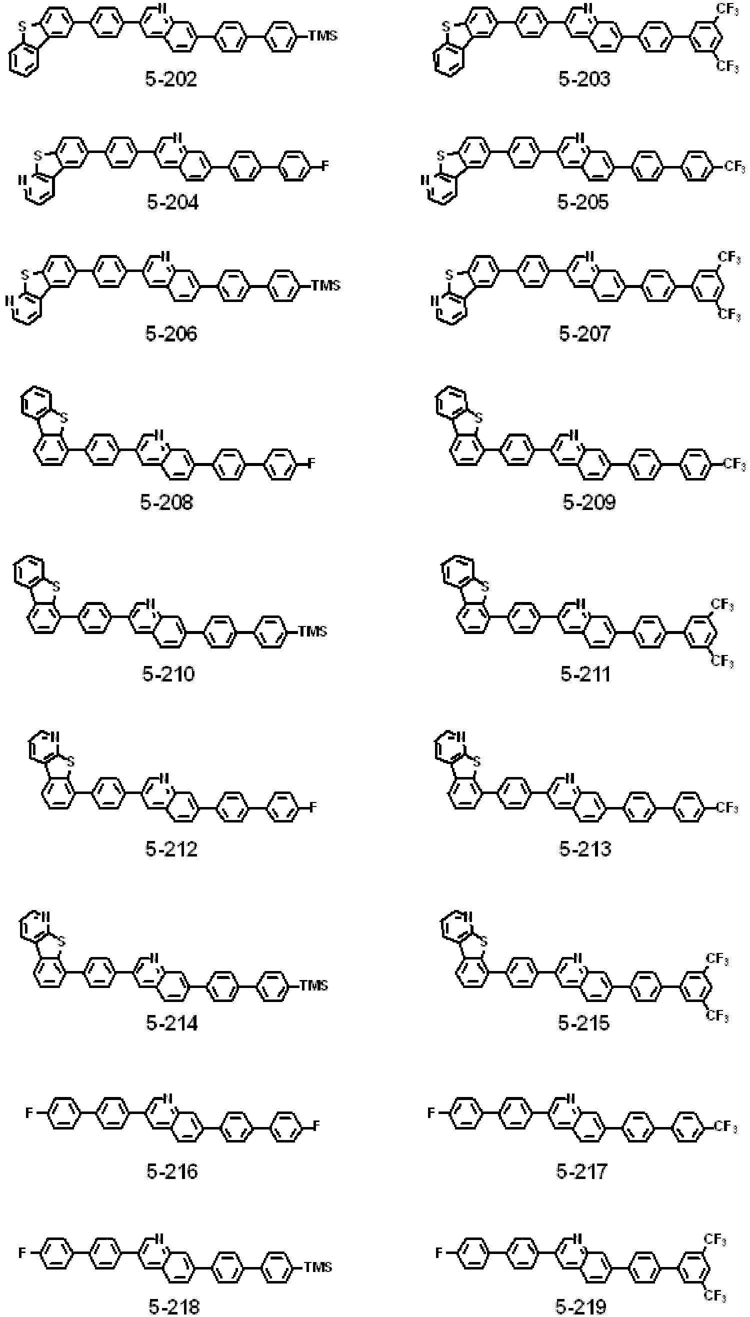

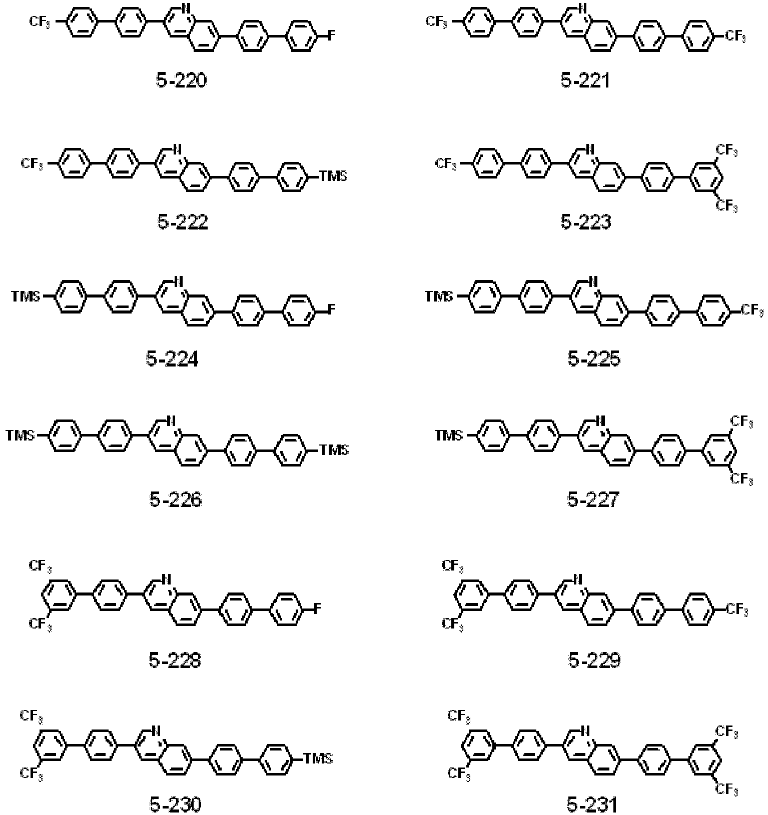

The compounds represented by Formulae 3 to 5 may be further substituted.

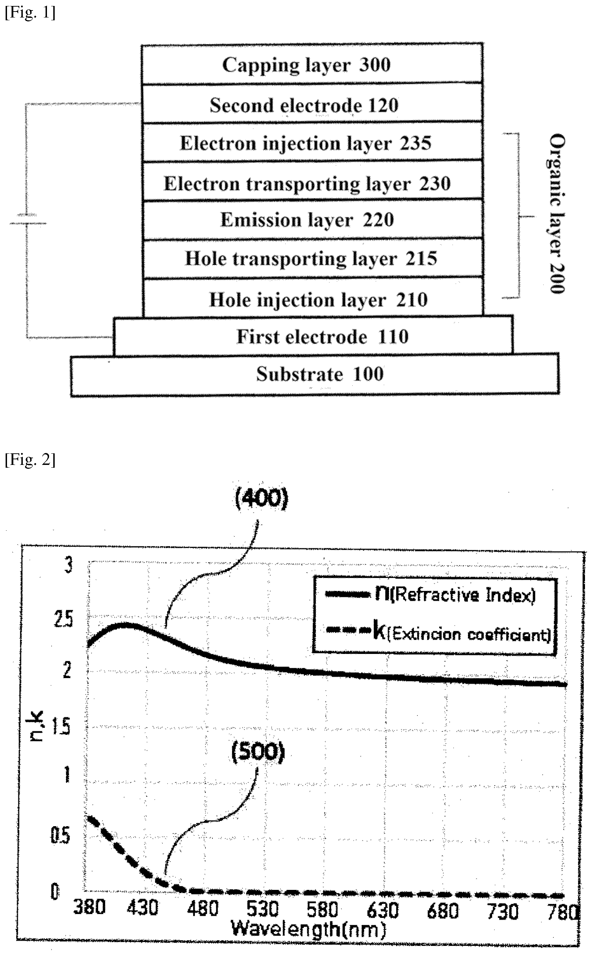

Exemplary embodiments of the present invention will now be described with reference to Figs. 1 and 2.

Fig. 1 is a schematic cross-sectional view of an organic light emitting device according to one embodiment of the present invention. Referring to Fig. 1, the organic light emitting device includes a first electrode 110, a hole injection layer 210, a hole transporting layer 215, an emission layer 220, an electron transporting layer 230, an electron injection layer 235, a second electrode 120, and a capping layer 300 stacked in this order on a substrate 100.

The first electrode 110 and the second electrode 120 are arranged opposite to each other and an organic layer 200 is arranged therebetween. The hole injection layer 210, the hole transporting layer 215, the emission layer 220, the electron transporting layer 230, and the electron injection layer 235 together form the organic layer 200.

The capping layer 300 is a feature of the present invention. The capping layer 300 is a functional layer deposited on the second electrode 120 and includes an organic material represented by Formula 1.

In the organic light emitting device illustrated in Fig. 1, the first electrode 110 is a conductive electrode and may be made of a metal alloy or a conductive material. The first electrode 110 is generally an anode but its function as an electrode is not limited.

The first electrode 110 may be prepared by depositing an electrode material on the substrate 100. Alternatively, electron beam evaporation or sputtering may be used instead of deposition. The material for the first electrode 110 may be selected from high work function materials that facilitate the injection of holes into the organic light emitting device.

The capping layer 300 is provided when the organic light emitting device is of a top emission type. In this case, a reflective electrode is used as the first electrode 110. Suitable materials for the first electrode include metals and alloys rather than oxides, for example, magnesium (Mg), aluminum (Al), aluminum-lithium (Al-Li), calcium (Ca), magnesium-indium (Mg-In), and magnesium-silver (Mg-Ag). Carbon-based flexible materials such as carbon nanotubes (CNTs) and graphene have also been used in recent years.

The organic layer 200 may consist of a plurality of layers. In this case, the organic layer 200 may include a hole transporting region 210-215 arranged on the first electrode 110, an emission layer 220 arranged on the hole transporting region, and an electron transporting region 230-235 arranged on the emission layer 220.

According to one embodiment, the capping layer 300 may include an organic compound represented by Formula 1, which will be described below.

The hole transporting region 210-215 is provided on the first electrode 110. The hole transporting region 210-215 may include a hole injection layer 210, a hole transporting layer 215, a hole buffer layer and/or an electron blocking layer (EBL). The hole transporting region 210-215 serves to ensure smooth hole injection and transport into the emission layer. The hole transporting region 210-215 is generally larger in thickness than the electron transporting region because the hole mobility is larger than the electron mobility.

The hole transporting region 210-215 may have a monolayer structure composed of a single material, a monolayer structure composed of different materials or a multilayer structure consisting of a plurality of layers composed of different materials.

For example, the hole transporting region 210-215 may include a hole injection layer 210 and/or a hole transporting layer 215. Alternatively, the hole transporting region 210-215 may have a monolayer structure composed of a hole injection material and a hole transporting material. Alternatively, the hole transporting region 210-215 may have a monolayer structure composed of a plurality of different materials. Alternatively, the hole transporting region 210-215 may have a multilayer structure consisting of a hole injection layer 210 and a hole transporting layer 215 sequentially stacked on the first electrode 110. Alternatively, the hole transporting region 210-215 may have a multilayer structure consisting of a hole injection layer 210, a hole transporting layer 215, and a hole buffer layer sequentially stacked on the first electrode 110. Alternatively, the hole transporting region 210-215 may have a multilayer structure consisting of a hole injection layer 210, a hole buffer layer, a hole transporting layer 215, and a hole buffer layer sequentially stacked on the first electrode 110. Alternatively, the hole transporting region 210-215 may have a multilayer structure consisting of a hole injection layer 210, a hole transporting layer 215, and an electron blocking layer (EBL) sequentially stacked on the first electrode 110. However, the hole transporting region is not limited to the above structures.

The hole injection layer 210 of the hole transporting region 210-215 may be formed on the anode by any suitable method such as vacuum deposition, spin coating, casting or LB deposition. For example, vacuum deposition for the formation of the hole injection layer 210 may be performed at a rate of about 1 Å/s at 100 to 500 °C. The vacuum deposition conditions are not particularly limited and can be freely controlled depending on the kind of a material for the hole injection layer 210 and the desired structure and thermal properties of the hole injection layer 210. Alternatively, the hole injection layer 210 may be formed by spin coating. The coating conditions may vary depending on the kind of a material for the hole injection layer 210 and the characteristics of layers forming interfaces with the hole injection layer 210. An appropriate coating speed and a suitable thermal process for solvent removal after coating are required to make the hole injection layer 210 even.

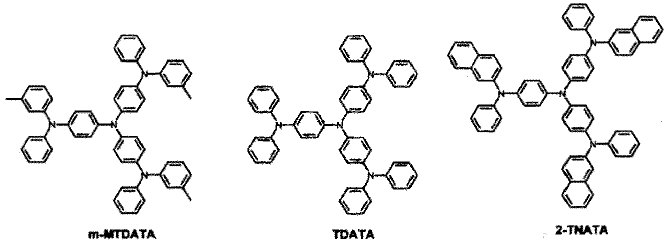

For example, the hole transporting region 210-215 may include one or more of m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline/poly(4-styrenesulfonate) (PANI/PSS).

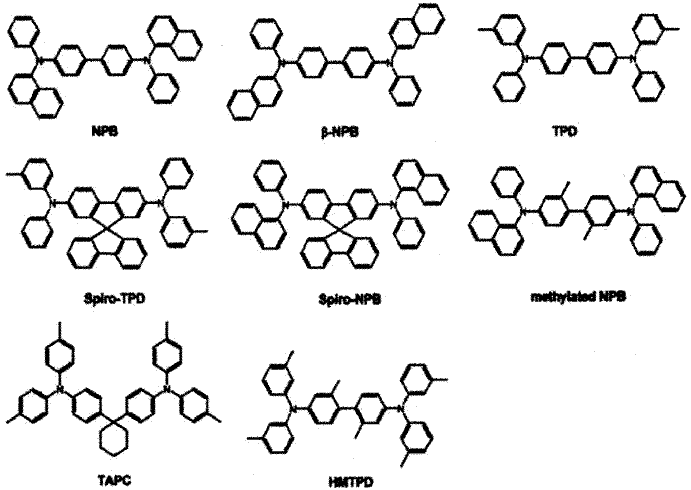

The hole transporting region 210-215 may have a thickness of 100 to 10,000 Å and the constituent organic layers of the hole transporting region 210-215 do not need to have the same thickness. For example, the hole injection layer 210 may have a thickness of 50 Å, the hole transporting layer 215 may have a thickness of 1000 Å, and the electron blocking layer may have a thickness of 500 Å. The thickness of the hole transporting region 210-215 can be determined to such a degree that the efficiency and lifetime of the organic light emitting device are satisfactory without an excessive increase in the driving voltage of the organic light emitting device. The organic layer 200 may include one or more layers selected from the group consisting of a hole injection layer 210, a hole transporting layer 215, a functional layer having functions of injecting and transporting holes, a buffer layer, an electron blocking layer, an emission layer 220, a hole blocking layer, an electron transporting layer 230, an electron injection layer 235, and a functional layer having functions of transporting and injecting electrons.

The hole transporting region 210-215 may be doped with a charge generating material to improve the characteristics (e.g., electrical properties) of the organic light emitting device, like the emission layer 220. This doping into the hole transporting region 210-215 can improve the electrical properties of the organic light emitting device.

The charge generating material is generally a material with very low HOMO and LUMO energy levels. For example, the LUMO energy level of the charge generating material is similar to the HOMO energy level of a material for the hole transporting layer 215. The low LUMO energy level facilitates hole transfer to the adjacent hole transporting layer 215 based on the electron vacancy in the LUMO, achieving improved electrical properties.

The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

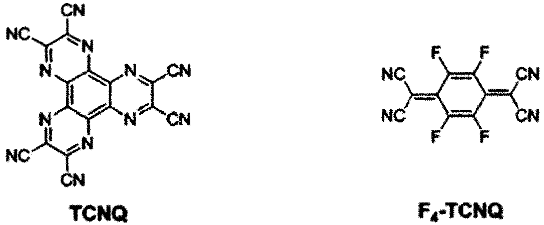

The hole transporting region 210-215 may further include a charge generating material to achieve improved conductivity, in addition to the aforementioned materials. The charge generating material may be uniformly or non-uniformly dispersed in the hole transporting region 210-215. The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

As described above, the hole transporting region 210-215 may further include a hole buffer layer and/or an electron blocking layer, in addition to the hole injection layer 210 and the hole transporting layer 215. The hole buffer layer may compensate for a resonance distance of light emitted from the emission layer 220 depending on the wavelength of the light to enhance the efficiency of light emission. The hole buffer layer may include the same material as the hole transporting region 210-215.

The electron blocking layer serves to prevent the injection of electrons from the electron transporting region 230-235 into the hole transporting region 210-215. The electron blocking layer may be formed using a material having a high T1 value that can not only block the migration of electrons to the hole transporting region but also can prevent excitons formed in the emission layer 220 from diffusing into the hole transporting region 210-215. For example, a host with a high T₁ value for the emission layer 220 is usually used as a material for the electron blocking layer.

The emission layer 220 is provided on the hole transporting region 210-215. For example, the emission layer 220 may have a thickness of about 100 Å to about 1000 Å or about 100 Å to about 300 Å. The emission layer 220 may have a monolayer structure composed of a single material, a monolayer structure composed of different materials or a multilayer structure consisting of a plurality of layers composed of different materials.

The emission layer 220 is a region where holes recombine with electrons to form excitons. Materials for the emission layer 220 should have appropriate energy band gaps such that excellent luminescent properties are obtained and a desired color of light is emitted. The materials for the emission layer 220 are typically a host and a dopant but are not limited thereto.

The host may include at least one of TPBi, TBADN, ADN (also called "DNA"), CBP, CDBP, TCP, and mCP with appropriate characteristics but is not limited thereto.

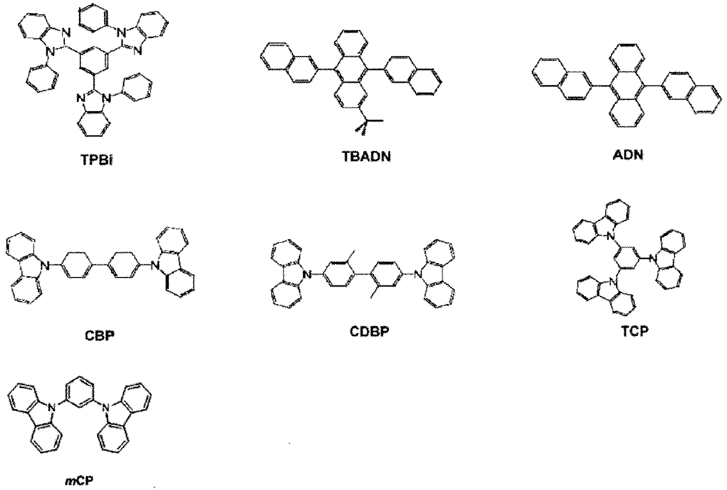

In one embodiment, the dopant may be an organometallic complex. The content of the dopant may generally be selected from 0.01 to 20% but is not necessarily limited thereto.

The electron transporting region 230-235 is provided on the emission layer 220. The electron transporting region 230-235 may include a hole blocking layer, an electron transporting layer 230, and/or an electron injection layer 235 but is not limited thereto.

The electron transporting region 230-235 may have a monolayer structure composed of a single material, a monolayer structure composed of different materials or a multilayer structure consisting of a plurality of layers composed of different materials. For example, the electron transporting region 230-235 may include an electron injection layer 235 and/or an electron transporting layer 230. Alternatively, the electron transporting region 230-235 may have a monolayer structure composed of an electron injection material and an electron transporting material. Alternatively, the electron transporting region 230-235 may have a monolayer structure composed of a plurality of different materials. Alternatively, the electron transporting region 230-235 may have a multilayer structure consisting of an electron transporting layer 230 and an electron injection layer 235 sequentially stacked on the emission layer 220. Alternatively, the electron transporting region 230-235 may have a multilayer structure consisting of a hole blocking layer, an electron transporting layer 230, and an electron injection layer 235 sequentially stacked on the emission electrode 220. However, the electron transporting region is not limited to the above structures. The thickness of the electron transporting region 230-235 may be, for example, about 1000 Å to about 1500 Å.

The electron transporting region 230-235 may be formed by any suitable method such as vacuum deposition, spin coating, casting, Langmuir-Blodgett (LB) deposition, inkjet printing, laser printing or laser induced thermal imaging (LITI).

The electron transporting layer 230 of the electron transporting region 230-235 may include an anthracene-based compound. Non-limiting examples of suitable materials for the electron transporting layer 230 include, but are not limited to, tris(8-hydroxyquinolinato)aluminum (Alq3), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-ylphenyl)-9,10-dinaphthylanthracene, 1,3,5-tri(1-phenyl-1H-benzo[d]imidazol-2-yl)phenyl) (TPBi), 2,9-dimethyl-4,7-diphenyl-1,10- phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), beryllium bis(benzoquinolin-10-olate) (Bebq2), 9,10-di(naphthalene-2-yl)anthracene (ADN). These materials may be used alone or as a mixture thereof.

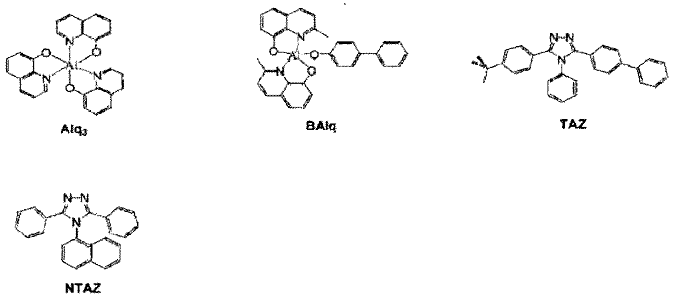

The material for the electron transporting layer 230 may be selected from materials with either high or low electron mobility. The selection varies depending on the structure of the organic light emitting device. The electron transporting layer 230 may be optionally doped with Liq or Li.

The thickness of the electron transporting layer 230 may be in the range of about 100 Å to about 1000 Å, for example, about 150 Å to about 500 Å. Within this range, satisfactory electron transporting properties can be obtained without a substantial increase in driving voltage.

The electron injection layer 235 of the electron transporting region 230-235 may include a metal material that facilitates electron injection. Examples of suitable metal materials for the electron injection layer 235 include, but are not limited to, LiF, lithium quinolate (LiQ), Li₂O, BaO, NaCl, CsF, lanthanide metals such as Yb, and metal halides such as RbCl and RbI.

The electron injection layer 235 may be formed of a mixture of an electron transporting material and an insulating organometallic salt. The organometallic salt may be a material having an energy band gap of approximately 4 eV or more. Specific examples of suitable organometallic salts for the electron injection layer include metal acetates, metal benzoates, metal acetoacetates, metal acetylacetonates, and metal stearates. The thickness of the electron injection layer 235 is in the range of about 1 Å to about 100 Å, preferably about 3 Å to about 90 Å. Within this range, satisfactory electron injection properties can be obtained without a substantial increase in driving voltage.

As mentioned previously, the electron transporting region 230-235 may include a hole blocking layer. For example, the hole blocking layer may include at least of 2,9-dimethyl-4,7-diphenyl-1,10-phenanthroline (BCP), 4,7-diphenyl-1,10-phenanthroline (Bphen), and Balq. However, the material for the hole blocking layer is not limited.

The second electrode 120 is provided on the electron transporting region 230-235. The second electrode 120 may be a common electrode or a cathode. The second electrode 120 may be a transmissive or transflective electrode. Unlike the first electrode 110, the second electrode 120 may be made of a combination of a relatively low work function metal, an electrically conductive compound, an alloy, etc.

The second electrode 120 is a transflective or reflective electrode. The second electrode 120 may include lithium (Li), magnesium (Mg), aluminum (Al), aluminum-lithium (Al-Li), calcium (Ca), magnesium-indium (Mg-In), magnesium-silver (Mg-Ag), or a compound or mixture containing the metal or alloy (e.g., a mixture of Ag and Mg). Alternatively, the second electrode 120 may have a multilayer structure consisting of a reflective or transflective film and a transparent conductive film. The material for the reflective or transflective film is the same as that described above for the transflective or reflective electrode. For example, the transparent conductive film may be formed of indium tin oxide (ITO), indium zinc oxide (IZO), zinc oxide (ZnO) or indium tin zinc oxide (ITZO).

Although not shown, the second electrode 120 may be connected to an auxiliary electrode. This connection can reduce the resistance of the second electrode 120.

The substrate 100 on which the electrodes and the organic layer are disposed may be made of a rigid or flexible material. For example, the rigid material may be soda-lime glass, alkali-free glass or aluminosilicate glass and the flexible material may be polycarbonate (PC), polyethersulfone (PES), a cyclic olefin copolymer (CEC), polyethylene terephthalate (PET) or polyethylene naphthalate (PEN).

When a voltage is applied to the first and second electrodes 110 and 120 of the organic light emitting device, holes are injected from the first electrode 110 and migrate to the emission layer 220 through the hole transporting region 210-215 and electrons are injected from the second electrode 120 and migrate to the emission layer 220 through the electron transporting region 230-235. The electrons recombine with the holes in the emission layer 220 to form excitons, which fall from the excited state to the ground state to emit light.

The path of light generated in the emission layer 220 tends to vary depending on the refractive indices of the organic/inorganic materials constituting the organic light emitting device. Only rays of light incident on the second electrode 120 at angles smaller than the critical angle of the second electrode 120 can pass through the second electrode 120. Rays of light coming into contact with the second electrode 120 at angles larger than the critical angle of the second electrode 120 are totally reflected or reflected, and as a result, they cannot be emitted outside the organic light emitting device.

A high refractive index of the capping layer 300 reduces the total reflection or reflection of light entering the capping layer 300, contributing to an improvement in luminescent efficiency. An appropriate thickness of the capping layer 300 maximizes the number of micro-cavities, contributing to high efficiency and improved color purity.

The capping layer 300 is placed at the outermost position of the organic light emitting device and has a great influence on the characteristics of the device without affecting the driving of the device at all. Therefore, the capping layer 300 is important from both viewpoints of protecting the inside of the organic light emitting device and improving the characteristics of the device. The organic materials absorb light energy in specific wavelength ranges depending on their energy band gaps. When the energy band gaps are adjusted such that light in the UV region capable of affecting the organic materials present in the organic light emitting device is absorbed, the capping layer 300 can be used to improve the optical properties of the organic light emitting device while protecting the organic light emitting device.

The organic light emitting device of the present invention may be of a top emission type, a bottom emission type or a dual emission type depending on materials used.

### Mode for Carrying out the Invention

The present invention will be specifically explained with reference to exemplary embodiments. However, these embodiments may be modified in various different forms and the scope of the present invention is not to be construed as being limited thereto. The embodiments are provided to more fully explain the present invention to those of ordinary skill in the art.

### [SYNTHESIS EXAMPLES]

### Synthesis Example 1: Synthesis of Intermediate 3

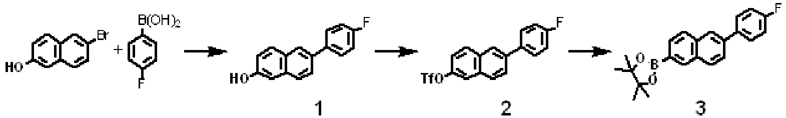

### (Synthesis of Intermediate 1)

10.0 g (44.8 mmol) of 6-bromonaphthalen-2-ol, 6.3 g (44.8 mmol) of (4-fluorophenyl)boronic acid, 1.6 g (1.3 mmol) of Pd(PPh₃)₄, 28.6 g (134.5 mmol) of K₃PO₄, 150 mL of toluene, 30 mL of ethanol, and 30 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 8.1 g (yield: 76.0%) of Intermediate **1** as a white solid.

### (Synthesis of Intermediate 2)

8.1 g (34.1 mmol) of Intermediate **1** was dissolved in 170 mL of dichloromethane (DCM) and 8.2 mL (102.2 mmol) of pyridine was added dropwise thereto. The temperature of the mixture was lowered to 0 °C. After slow dropwise addition of 6.9 mL (40.9 mmol) of trifluoromethanesulfonic anhydride (Tf₂O), the temperature was raised to room temperature. The reaction was allowed to proceed for 12 h. The reaction mixture was washed with 100 mL of water. The organic layer was separated, dried over anhydrous sodium sulfate, filtered, concentrated, and purified by column chromatography (CHCl₃) to afford 12.6 g (yield: 100%) of Intermediate **2** as a yellow liquid.

### (Synthesis of Intermediate 3)

12.6 g (34.0 mmol) of Intermediate **2,** 13.0 g (51.0 mmol) of bis(pinacolato)diboron, 556 mg (680.5 µmol) of Pd(dppf)Cl₂·CH₂Cl₂, 10.0 g (102.1 mmol) of KOAc, and 170 mL of 1,4-dioxane were mixed together in a 500 mL one-neck flask. The mixture was stirred at 100 °C for 12 h. After completion of the reaction, the reaction mixture was cooled to room temperature, passed through a pad of celite, concentrated under reduced pressure, and purified by silica gel column chromatography (CHCl₃) to afford 7.6 g (yield: 63.7%) of Intermediate **3** as a white solid.

### Synthesis Example 2: Synthesis of Intermediate 6

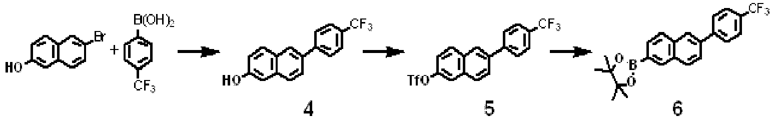

### (Synthesis of Intermediate 4)

10.0 g (44.8 mmol) of 6-bromonaphthalen-2-ol, 8.5 g (44.8 mmol) of 4-(trifluoromethyl)phenylboronic acid, 1.6 g (1.5 mmol) of Pd(PPh₃)₄, 17.6 g (89.7 mmol) of K₃PO₄, 150 mL of toluene, 30 mL of ethanol, and 30 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 9.2 g (yield: 71.2%) of Intermediate **4** as a white solid.

### (Synthesis of Intermediate 5)

9.2 g (31.9 mmol) of Intermediate **4** was dissolved in 160 mL of dichloromethane (DCM) and 7.7 mL (95.7 mmol) of pyridine was added dropwise thereto. The temperature of the mixture was lowered to 0 °C. After slow dropwise addition of 6.4 mL (38.3 mmol) of trifluoromethanesulfonic anhydride (Tf₂O), the temperature was raised to room temperature. The reaction was allowed to proceed for 12 h. The reaction mixture was washed with 100 mL of water. The organic layer was separated, dried over anhydrous sodium sulfate, filtered, concentrated, and purified by column chromatography (CHCl₃) to afford 13.2 g (yield: 98.4%) of Intermediate **5** as a yellow liquid.

### (Synthesis of Intermediate 6)

13.2 g (31.4 mmol) of Intermediate **5,** 9.6 g (37.7 mmol) of bis(pinacolato)diboron, 769.4 mg (942.1 µmol) of Pd(dppl)Cl₂·CH₂Cl₂, 6.2 g (62.8 mmol) of KOAc, and 160 mL of 1,4-dioxane were mixed together in a 500 mL one-neck flask. The mixture was stirred at 100 °C for 12 h. After completion of the reaction, the reaction mixture was cooled to room temperature, passed through a pad of celite, concentrated under reduced pressure, and purified by silica gel column chromatography (CHC1₃) to afford 7.2 g (yield: 57.6%) of Intermediate **6** as a white solid.

### Synthesis Example 3: Synthesis of Intermediate 9

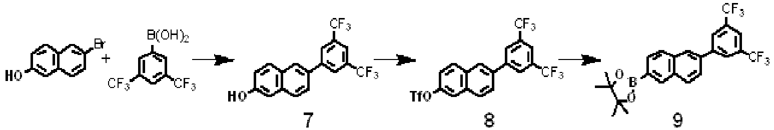

### (Synthesis of Intermediate 7)

50.0 g (224.2 mmol) of 6-bromonaphthalen-2-ol, 57.8 g (224.2 mmol) of 3,5-bis(trifluoromethyl)phenylboronic acid, 7.8 g (6.7 mmol) of Pd(PPh₃)₄, 142.7 g (672.5 mmol) of K₃PO₄, 600 mL of toluene, 200 mL of ethanol, and 200 mL of distilled water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 57.9 g (yield: 72.5%) of Intermediate **7** as a white solid.

### (Synthesis of Intermediate 8)

57.9 g (162.5 mmol) of Intermediate **7** was dissolved in 800 mL of DCM and 40.0 mL (487.6 mmol) of pyridine was added dropwise thereto. The temperature of the mixture was lowered to 0 °C. After slow dropwise addition of 33.0 mL (195.0 mmol) of trifluoromethanesulfonic anhydride (Tf₂O), the temperature was raised to room temperature. The reaction was allowed to proceed for 12 h. The reaction mixture was washed with water (500 mL). The organic layer was separated, dried over anhydrous sodium sulfate, filtered, concentrated, and purified by column chromatography (CHCl₃) to afford 79.4 g (yield: 100%) of Intermediate **8** as a yellow solid.

### (Synthesis of Intermediate 9)

79.4 g (162.5 mmol) of Intermediate **8,** 61.9 g (243.8 mmol) of bis(pinacolato)diboron, 2.7 g (3.3 mmol) of Pd(dppf)Cl₂·CH₂Cl₂, 47.9 g (487.6 mmol) of KOAc, and 800 mL of 1,4-dioxane were mixed together in a 2 L one-neck flask. The mixture was stirred at 100 °C for 12 h. After completion of the reaction, the reaction mixture was cooled to room temperature, passed through a pad of celite, concentrated under reduced pressure, and purified by silica gel column chromatography (CHC1₃) to afford 66.6 g (yield: 87.9%) of Intermediate **9** as a white solid.

### Synthesis Example 4: Synthesis of Intermediate 10

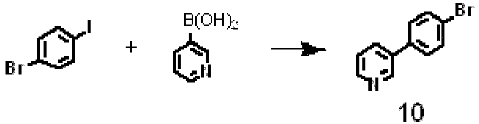

69.0 g (244.0 mmol) of 1-bromo-4-iodobenzene, 30.0 g (244.0 mmol) of 3-pyridinylboronic acid, 8.4 g (7.3 mmol) of Pd(PPh₃)₄, 83.4 g (603.4 mmol) of K₂CO₃, 500 mL of toluene, 250 mL of ethanol, and 250 mL of distilled water were mixed together in a 1 L one-neck flask. The mixture was stirred under reflux for 19 h. After completion of the reaction, the reaction mixture was cooled to room temperature and washed with CHCl₃ and water. The resulting mixture was purified by silica gel column chromatography (DCM:EA) to afford 41.1 g (yield: 71.9%) of Intermediate **10** as a yellow oil.

### Synthesis Example 5: Synthesis of Intermediate 12

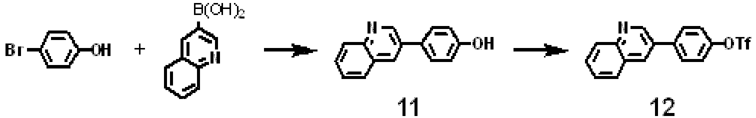

### (Synthesis of Intermediate 11)

50.0 g (289.0 mmol) of 4-bromophenol, 65.0 g (375.7 mmol) of quinolin-3-ylboronic acid, 10.0 g (8.7 mmol) of Pd(PPh₃)₄, 119.8 g (867.0 mmol) of K₂CO₃, 1500 mL of toluene, 360 mL of ethanol, and 360 mL of distilled water were mixed together in a 3 L two-neck flask. The mixture was allowed to react at 90 °C for 16 h. After completion of the reaction, the reaction mixture was cooled to room temperature, washed with 500 mL of water, and extracted with ethyl acetate. The organic layer was separated, concentrated, and solidified with a mixture of acetone/MeOH to afford 55.2 g (yield: 86.3%) of Intermediate **11** as a pale brown solid.

### (Synthesis of Intermediate 12)

54.2 g (245.0 mmol) of Intermediate **11** was dissolved in 1.2 L of DCM in a 2 L one-neck flask and 39.6 mL (490.0 mmol) of pyridine was added dropwise thereto. The temperature of the mixture was lowered to 0 °C. After slow dropwise addition of 53.6 mL (318.5 mmol) of trifluoromethanesulfonic anhydride (Tf₂O), the temperature was raised to room temperature. The reaction was allowed to proceed for 3 h. The reaction mixture was washed with 1 L of water and extracted with DCM. The organic layer was separated, dried over anhydrous magnesium sulfate, filtered, concentrated, purified by silica gel column chromatography (Hex:EA), solidified with a mixture of Hex/EA, and filtered to afford 59.3 g (yield: 68.5%) of Intermediate **12** as a pale yellow solid.

### Synthesis Example 6: Synthesis of Intermediate 14

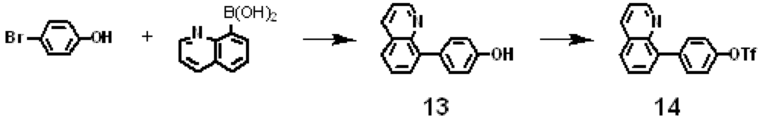

### (Synthesis of Intermediate 13)

20.0 g (115.6 mmol) of 4-bromophenol, 31.6 g (138.7 mmol) of quinolin-8-ylboronic acid, 4.0 g (3.5 mmol) of Pd(PPh₃)₄, 47.9 g (346.8 mmol) of K₂CO₃, 600 mL of toluene, 150 mL of ethanol, and 150 mL of distilled water were mixed together in a 2 L two-neck flask. The mixture was allowed to react at 90 °C for 4 h. After completion of the reaction, the reaction mixture was cooled to room temperature, washed with 200 mL of water, and extracted with ethyl acetate. The organic layer was separated, dried over anhydrous magnesium sulfate, filtered, and distilled under reduced pressure. The resulting solid mixture was dissolved in hot toluene, filtered through a pad of silica, and solidified with toluene to afford 6.9 g (yield: 27.0%) of Intermediate **13** as a white solid.

### (Synthesis of Intermediate 14)

6.9 g (31.2 mmol) of Intermediate **13** was dissolved in 160 mL of DCM in a 500 mL one-neck flask and 5.0 mL (62.4 mmol) of pyridine was added dropwise thereto. The temperature of the mixture was lowered to 0 °C. After slow dropwise addition of 7.9 mL (46.8 mmol) of trifluoromethanesulfonic anhydride (Tf₂O), the temperature was raised to room temperature. The reaction was allowed to proceed for 5 h. The reaction mixture was washed with 200 mL of water and extracted with DCM. The organic layer was separated, dried over anhydrous magnesium sulfate, filtered, concentrated, dissolved in DCM, and filtered through a pad of silica (Hex:EA) to afford 8.1 g (yield: 73.5%) of Intermediate **14** as a white solid.

### Synthesis Example 7: Synthesis of Intermediate 16

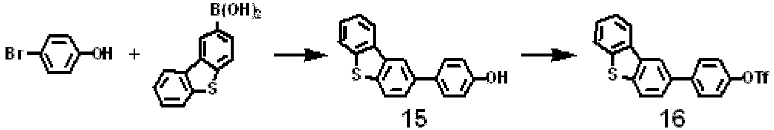

### (Synthesis of Intermediate 15)

20.0 g (115.6 mmol) of 4-bromophenol, 31.6 g (138.7 mmol) of dibenzo[b,d]thiophen-2-ylboronic acid, 4.0 g (3.5 mmol) of Pd(PPh₃)₄, 47.9 g (346.8 mmol) of K₂CO₃, 600 mL of toluene, 150 mL of ethanol, and 150 mL of distilled water were mixed together in a 2 L two-neck flask. The mixture was allowed to react at 90 °C for one day. After completion of the reaction, the reaction mixture was cooled to room temperature, washed with 200 mL of water, and extracted with ethyl acetate. The organic layer was separated, dried over anhydrous magnesium sulfate, filtered, distilled under reduced pressure, and solidified with a mixture of DCM/Hex to afford 16.1 g (yield: 62.9%) of Intermediate **15** as a pink solid.

### (Synthesis of Intermediate 16)

16.1 g (58.3 mmol) of Intermediate **15** was dissolved in 300 mL of dichloromethane in a 500 mL one-neck flask and 9.4 mL (116.5 mmol) of pyridine was added dropwise thereto. The temperature of the mixture was lowered to 0 °C. After slow dropwise addition of 14.7 mL (87.4 mmol) of trifluoromethanesulfonic anhydride (Tf₂O), the temperature was raised to room temperature. The reaction was allowed to proceed for 6 h. The reaction mixture was washed with 200 mL of water and extracted with dichloromethane. The organic layer was separated, dried over anhydrous magnesium sulfate, filtered, concentrated, dissolved in dichloromethane, and filtered through a pad of silica (Hex:EA=10:1) to afford 23.0 g (yield: 96.7%) of Intermediate **16** as a yellow oil.

### Synthesis Example 8: Synthesis of Intermediate 18

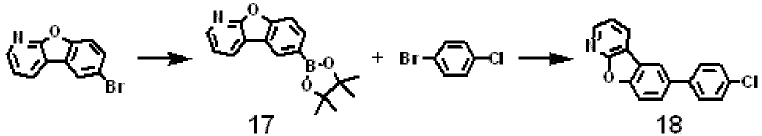

### (Synthesis of intermediate 17)

10.0 g (40.3 mmol) of 6-bromobenzofuro[2,3-b]pyridine, 12.3 g (48.4 mmol) of bis(pinacolato)diboron, 987.6 mg (1.2 mmol) of Pd(dppf)Cl₂·CH₂Cl₂, 7.9 g (80.6 mmol) of KOAc, and 200 mL of 1,4-dioxane were mixed together. The mixture was stirred at 100 °C for 12 h. After completion of the reaction, the reaction mixture was cooled to room temperature, passed through a pad of celite, concentrated under reduced pressure, and purified by silica gel column chromatography (CHCl₃) to afford 6.0 g (yield: 50.4%) of Intermediate **17** as a white solid.

### (Synthesis of Intermediate 18)

6.0 g (20.3 mmol) of Intermediate **17,** 3.9 g (20.3 mmol) of 1-bromo-4-chlorobenzene, 1.2 g (0.1 mmol) of Pd(PPh₃)₄, 5.6 g (40.6 mmol) of K₂CO₃, 72 mL of toluene, 36 mL of purified water, and 24 mL of ethanol were mixed together in a 250 mL two-neck flask. The mixture was allowed to react at 100 °C for one day. After completion of the reaction, the reaction mixture was cooled to room temperature, added with purified water, and extracted with ethyl acetate. The solvent was removed under reduced pressure. The resulting reaction mixture was dissolved in chloroform, filtered through silica gel, concentrated under reduced pressure to remove the solvent, and solidified with methanol/hexane to afford 3.5 g (yield: 62.1%) of Intermediate **18** as a white solid.

### Synthesis Example 9: Synthesis of Intermediate 20

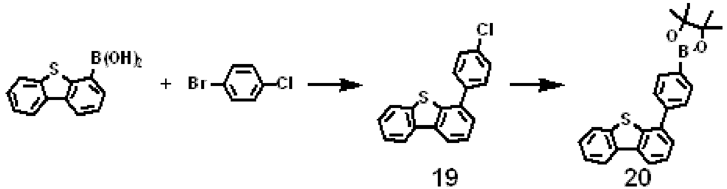

### (Synthesis of Intermediate 19)

20.0 g (104.4 mmol) of 4-dibenzothiophenylboronic acid, 23.8 g (104.4 mmol) of 1-bromo-4-chlorobenzene, 6.0 g (5.2 mmol) of Pd(PPh₃)₄, 43.3 g (313.4 mmol) of K₂CO₃, 240 mL of toluene, 140 mL of purified water, and 120 mL of ethanol were mixed together in a 2000 mL two-neck flask. The mixture was allowed to react at 110 °C for 5 h. After completion of the reaction, the reaction mixture was cooled to room temperature, added with purified water, and extracted with ethyl acetate. The solvent was removed under reduced pressure. The resulting reaction mixture was dissolved in chloroform, filtered through silica gel, concentrated under reduced pressure to remove the solvent, and solidified with methanol/hexane to afford 25.8 g (yield: 83.9%) of Intermediate **19** as a white solid.

### (Synthesis of Intermediate 20)

10.0 g (33.9 mmol) of Intermediate **19,** 12.9 g (50.8 mmol) of bis(pinacolato)diboron, 1.9 g (3.4 mmol) of Pd(dba)₂, 3.2 g (6.7 mmol) of X-Phos, 9.9 g (101.7 mmol) of KOAc, and 120 mL of toluene were mixed together in a 250 mL two-neck flask. The mixture was stirred under reflux at 110 °C for 5 h. After completion of the reaction, the reaction mixture was cooled to room temperature, added with purified water, and extracted with ethyl acetate. The solvent was removed under reduced pressure. The resulting reaction mixture was purified by silica gel column chromatography (Hex:CH₂Cl₂) and solidified with methanol/hexane to afford 10.3 g (yield: 78.7%) of Intermediate **20** as a white solid.

### Synthesis Example 10: Synthesis of Intermediate 23

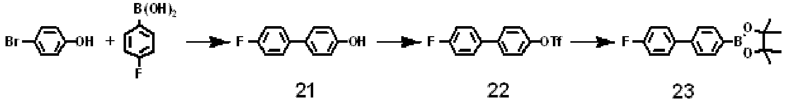

### (Synthesis of Intermediate 21)

50.0 g (289.0 mmol) of 4-bromophenol, 67.3 g (346.8 mmol) of (4-fluorophenyl)boronic acid, 10.0 g (8.7 mmol) of Pd(PPh₃)₄, 119.8 g (867.0 mmol) of K₂CO₃, 1.5 L of toluene, 400 mL of ethanol, and 400 mL of water were mixed together in a 3 L four-neck flask. The mixture was stirred at 80 °C for 16 h. After completion of the reaction, the reaction mixture was cooled to room temperature and extracted with ethyl acetate. The organic layer was separated, dried over anhydrous magnesium sulfate, filtered, and distilled under reduced pressure. The resulting solid mixture was dissolved in ethyl acetate, filtered through a pad of celite, and solidified with a mixture of Hex/EA to afford 48.1 g (yield: 88.4%) of Intermediate **21** as an ivory solid.

### (Synthesis of Intermediate 22)

48.1 g (255.6 mmol) of Intermediate **21** was dissolved in 1.2 L of DCM in a 2 L one-neck flask and 41.3 mL (511.2 mmol) of pyridine was added dropwise thereto. The temperature of the mixture was lowered to 0 °C. After slow dropwise addition of 64.5 mL (383.4 mmol) of trifluoromethanesulfonic anhydride (Tf₂O), the temperature was raised to room temperature. The reaction was allowed to proceed for 4 h. The reaction mixture was added with purified water, extracted with DCM, and dried over anhydrous magnesium sulfate. The solvent was removed under reduced pressure. The resulting reaction mixture was solidified with a mixture of DCM/MeOH to afford 47.0 g (yield: 57.4%) of Intermediate **22** as a colorless oil.

### (Synthesis of Intermediate 23)

40.0 g (124.9 mmol) of Intermediate **22,** 38.1 g (149.9 mmol) of bis(pinacolato)diboron, 5.1 g (6.2 mmol) of Pd(dppf)Cl₂·CH₂Cl₂, 36.8 g (374.7 mmol) of KOAc, and 600 mL of 1,4-dioxane were mixed together in a 2 L two-neck flask. The mixture was stirred under reflux for 16 h. After completion of the reaction, the reaction mixture was cooled to room temperature, added with purified water, extracted with ethyl acetate, and dried over anhydrous magnesium sulfate. The solvent was removed under reduced pressure. The resulting reaction mixture was purified by silica gel column chromatography (toluene) and solidified with hexane at a low temperature to afford 26.3 g (yield: 70.5%) of Intermediate **23** as a white solid.

### Synthesis Example 11: Synthesis of Intermediate 24

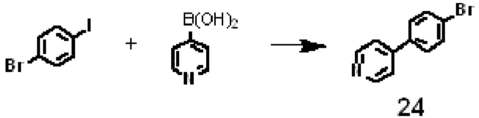

69.0 g (244.0 mmol) of 1-bromo-4-iodobenzene, 30.0 g (244.0 mmol) of 4-pyridinylboronic acid, 8.4 g (7.3 mmol) of Pd(PPh₃)₄, 83.4 g (603.4 mmol) of K₂CO₃, 500 mL of toluene, 250 mL of ethanol, and 250 mL of distilled water were mixed together in a 1 L one-neck flask. The mixture was stirred under reflux for 19 h. After completion of the reaction, the reaction mixture was cooled to room temperature and washed with CHCl₃ and water. The resulting mixture was purified by silica gel column chromatography (DCM:EA) to afford 41.1 g (yield: 71.9%) of Intermediate **24** as a yellow oil.

### Synthesis Example 12: Synthesis of Intermediate 26

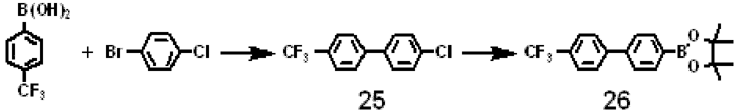

### (Synthesis of Intermediate 25)

10.0 g (52.7 mmol) of 4-(trifluoromethyl)phenylboronic acid, 10.1 g (52.7 mmol) of 1-bromo-4-chlorobenzene, 1.8 g (1.6 mmol) of Pd(PPh₃)₄, 14.6 g (105.3 mmol) of K₂CO₃, 120 mL of toluene, 70 mL of purified water, and 70 mL of ethanol were mixed together in a 1000 mL two-neck flask The mixture was allowed to react at 100 °C for 5 h. After completion of the reaction, the reaction mixture was cooled to room temperature, added with purified water, and extracted with ethyl acetate. The solvent was removed under reduced pressure. The resulting mixture was dissolved in chloroform, filtered through silica gel, and concentrated under reduced pressure to remove the solvent. The resulting reaction mixture was solidified with methanol/hexane to afford 10.1 g (yield: 74.7%) of Intermediate **25** as a white solid.

### (Synthesis of Intermediate 26)

10.1 g (39.4 mmol) of Intermediate **25,** 12.0 g (47.2 mmol) of bis(pinacolato)diboron, 1.9 g (3.4 mmol) of Pd(dba)₂, 3.2 g (6.7 mmol) of X-Phos, 7.7 g (78.7 mmol) of KOAc, and 120 mL of toluene were mixed together in a 250 mL two-neck flask. The mixture was stirred under reflux at 110 °C for 5 h. After completion of the reaction, the reaction mixture was cooled to room temperature, added with purified water, and extracted with ethyl acetate. The solvent was removed under reduced pressure. The resulting reaction mixture was purified by silica gel column chromatography (Hex:CH₂Cl₂) and solidified with methanol/hexane to afford 10.2 g (yield: 74.4%) of Intermediate **26** as a white solid.

### Synthesis Example 13: Synthesis of Intermediate 27

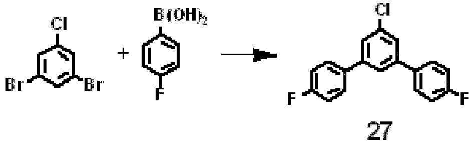

10.0 g (37.0 mmol) of 1,3-dibromo-5-chlorobenzene, 10.6 g (75.8 mmol) of (4-fluorophenyl)boronic acid, 2.6 g (2.2 mmol) of Pd(PPh₃)₄, 30.7 g (221.9 mmol) of K₂CO₃, 120 mL of toluene, 30 mL of water, and 30 mL of ethanol were mixed together in a 1 L one-neck flask acid. The mixture was stirred under reflux for 12 h. After completion of the reaction, the reaction mixture was cooled to room temperature, added with water, and extracted with chloroform. The solvent was removed under reduced pressure. The resulting reaction mixture was purified by silica gel column chromatography (Hex:DCM=10:1) and solidified with methanol to afford 3.6 g (yield: 32.7%) of Intermediate **27** as a white solid.

### Synthesis Example 14: Synthesis of Intermediate 29

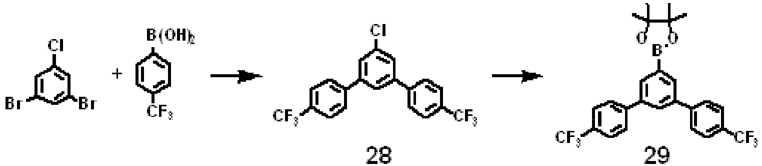

### (Synthesis of Intermediate 28)

15.0 g (55.4 mmol) of 1,3-dibromo-5-chlorobenzene, 22.1 g (116.5 mmol) of 4-(trifluoromethyl)phenylboronic acid, 6.4 g (5.5 mmol) of Pd(PPh₃)₄, 23.0 g (166.4 mmol) of K₂CO₃, 180 mL of toluene, 100 mL of purified water, and 90 mL of ethanol were mixed together in a 250 mL two-neck flask. The mixture was allowed to react at 80 °C for one day. After completion of the reaction, the reaction mixture was cooled to room temperature, added with purified water, and extracted with ethyl acetate. The solvent was removed under reduced pressure. The resulting reaction mixture was dissolved in chloroform, filtered through silica gel, and concentrated under reduced pressure to remove the solvent. The resulting reaction mixture was solidified with dichloromethane/methanol to afford 16.2 g (yield: 73.1%) of Intermediate **28** as a white solid.

### (Synthesis of Intermediate 29)

7.0 g (17.4 mmol) of Intermediate **28,** 6.6 g (26.2 mmol) of bis(pinacolato)diboron, 1.0 g (0.2 mmol) of Pd(dba)₂, 1.7 g (0.3 mmol) of X-Phos, 5.1 g (52.4 mmol) of KOAc, and 84 mL of toluene were mixed together in a 250 mL two-neck flask. The mixture was allowed to react at 110 °C for 5 h. After completion of the reaction, the reaction mixture was cooled to room temperature, added with purified water, and extracted with ethyl acetate. The solvent was removed under reduced pressure. The resulting reaction mixture was purified by silica gel column chromatography (Hex:CH₂Cl₂) and solidified with methanol to afford 4.6 g (yield: 53.4%) of Intermediate **29** as a white solid.

### Synthesis Example 15: Synthesis of Intermediate 30

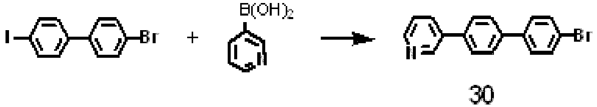

17.4 g (48.3 mmol) of 4-bromo-4'-iodo-1,1'-biphenyl, 5.9 g (48.3 mmol) of 3-pyridinylboronic acid, 1.7 g (1.4 mmol) of Pd(PPh₃)₄, 16.7 g (120.8 mmol) of K₂CO₃, 200 mL of toluene, 100 mL of ethanol, and 100 mL of water were mixed together in a 1 L one-neck flask. The mixture was stirred under reflux for 18 h. After completion of the reaction, the reaction mixture was cooled to room temperature, washed with DCM and water, purified by silica gel column chromatography (DCM:EA), and solidified with MeOH to afford 11.8 g (yield: 78.5%) of Intermediate **30** as a yellow solid.

### Synthesis Example 16: Synthesis of Intermediate 33

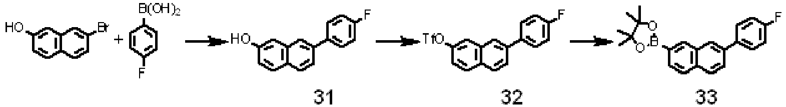

### (Synthesis of Intermediate 31)

10.0 g (44.8 mmol) of 7-bromonaphthalen-2-ol, 6.3 g (44.8 mmol) of (4-fluorophenyl)boronic acid, 1.6 g (1.3 mmol) of Pd(PPh₃)₄, 28.6 g (134.5 mmol) of K₃PO₄, 150 mL of toluene, 30 mL of ethanol, and 30 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 8.1 g (yield: 76.0%) of Intermediate **31** as a white solid.

### (Synthesis of Intermediate 32)

8.1 g (34.1 mmol) of Intermediate **31** was dissolved in 170 mL of dichloromethane (DCM) and 8.2 mL (102.2 mmol) of pyridine was added dropwise thereto. The temperature of the mixture was lowered to 0 °C. After slow dropwise addition of 6.9 mL (40.9 mmol) of trifluoromethanesulfonic anhydride (Tf₂O), the temperature was raised to room temperature. The reaction was allowed to proceed for 12 h. The reaction mixture was washed with 100 mL of water. The organic layer was separated, dried over anhydrous sodium sulfate, filtered, concentrated, and purified by column chromatography (CHC1₃) to afford 12.6 g (yield: 100%) of Intermediate **32** as a yellow liquid.

### (Synthesis of Intermediate 33)

12.6 g (34.0 mmol) of Intermediate **32,** 13.0 g (51.0 mmol) of bis(pinacolato)diboron, 556 mg (680.5 µmol) of Pd(dppf)Cl₂·CH₂Cl₂, 10.0 g (102.1 mmol) of KOAc, and 170 mL of 1,4-dioxane were mixed together in a 500 mL one-neck flask. The mixture was stirred at 100 °C for 12 h. After completion of the reaction, the reaction mixture was cooled to room temperature, passed through a pad of celite, concentrated under reduced pressure, and purified by silica gel column chromatography (CHC1₃) to afford 7.55 g (yield: 63.7%) of Intermediate **33** as a white solid.

### Synthesis Example 17: Synthesis of Intermediate 36

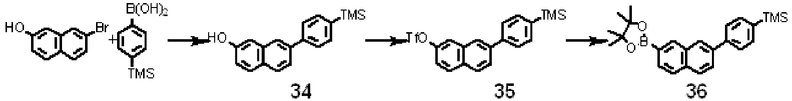

### (Synthesis of Intermediate 34)

10.0 g (44.8 mmol) of 7-bromonaphthalen-2-ol, 10.4 g (53.8 mmol) of (4-fluorophenyl)boronic acid, 2.6 g (2.2 mmol) of Pd(PPh₃)₄, 12.4 g (89.7 mmol) of K₂CO₃, 150 mL of toluene, 30 mL of ethanol, and 30 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 7.3 g (yield: 55.7%) of Intermediate **34** as a white solid.

### (Synthesis of Intermediate 35)

7.3 g (25.0 mmol) of Intermediate **34** was dissolved in 125 mL of dichloromethane (DCM) and 6.0 mL (74.9 mmol) of pyridine was added dropwise thereto. The temperature of the mixture was lowered to 0 °C. After slow dropwise addition of 5.0 mL (30.0 mmol) of trifluoromethanesulfonic anhydride (Tf₂O), the temperature was raised to room temperature. The reaction was allowed to proceed for 12 h. The reaction mixture was washed with 100 mL of water. The organic layer was separated, dried over anhydrous sodium sulfate, filtered, concentrated, and purified by column chromatography (CHC1₃) to afford 10.0 g (yield: 94.4%) of Intermediate **35** as a yellow liquid.

### (Synthesis of Intermediate 36)

10.0 g (23.6 mmol) of Intermediate **35,** 9.0 g (35.3 mmol) of bis(pinacolato)diboron, 962 mg (1.2 mmol) of Pd(dppf)Cl₂·CH₂Cl₂, 4.6 g (47.1 mmol) of KOAc, and 120 mL of 1,4-dioxane were mixed together in a 500 mL one-neck flask. The mixture was stirred at 100 °C for 12 h. After completion of the reaction, the reaction mixture was cooled to room temperature, passed through a pad of celite, concentrated under reduced pressure, and purified by silica gel column chromatography (CHC1₃) to afford 6.2 g (yield: 65.4%) of Intermediate **36** as a white solid.

### Synthesis Example 18: Synthesis of Intermediate 39

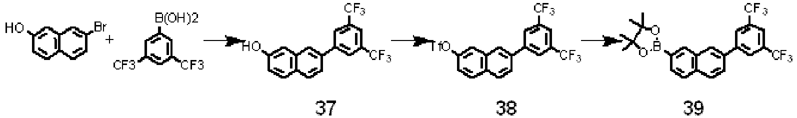

### (Synthesis of Intermediate 37)

50.0 g (224.2 mmol) of 7-bromonaphthalen-2-ol, 57.8 g (224.2 mmol) of 3,5-bis(trifluoromethyl)phenylboronic acid, 7.8 g (6.7 mmol) of Pd(PPh₃)₄, 142.7 g (672.5 mmol) of K₃PO₄, 500 mL of toluene, 150 mL of ethanol, and 150 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃→CHCl₃:EA) to afford 78.0 g (yield: 97.7%) of Intermediate **37** as a yellow solid.

### (Synthesis of Intermediate 38)

78.0 g (218.9 mmol) of Intermediate **37** was dissolved in 800 mL of dichloromethane (DCM) and 52.9 mL (656.8 mmol) of pyridine was added dropwise thereto. The temperature of the mixture was lowered to 0 °C. After slow dropwise addition of 44.2 mL (262.7 mmol) of Tf₂O, the temperature was raised to room temperature. The reaction was allowed to proceed for 12 h. The reaction mixture was washed with water (500 mL). The organic layer was separated, dried over anhydrous sodium sulfate, filtered, concentrated, and purified by column chromatography (CHCl₃) to afford 106.9 g (yield: 100%) of Intermediate **38** as a yellow liquid.

### (Synthesis of Intermediate 39)

106.9 g (218.9 mmol) of Intermediate **38,** 83.4 g (328.4 mmol) of bis(pinacolato)diboron, 3.6 g (4.4 mmol) of Pd(dppf)Cl₂·CH₂Cl₂, 64.4 g (656.7 mmol) of KOAc, and 700 mL of 1,4-dioxane were mixed together in a 2 L one-neck flask. The mixture was stirred at 100 °C for 12 h. After completion of the reaction, the reaction mixture was cooled to room temperature, passed through a pad of celite, concentrated under reduced pressure, and purified by silica gel column chromatography (Hex: CHCl₃) to afford 80.7 g (yield: 79.1%) of Intermediate **39** as a yellow liquid.

### Synthesis Example 19: Synthesis of Intermediate 40

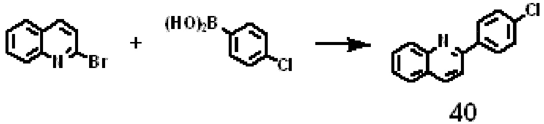

10.0 g (48.1 mmol) of 2-bromoquinoline, 7.5 g (48.1 mmol) of (4-chlorophenyl)boronic acid, 1.7 g (1.4 mmol) of Pd(PPh₃)₄, 13.3 g (96.1 mmol) of potassium carbonate, 150 mL of toluene, 30 mL of ethanol, and 30 mL of water were mixed together. The mixture was stirred under reflux for 12 h. After completion of the reaction, the reaction mixture was cooled to room temperature, added with water, and extracted with ethyl acetate. The solvent was removed under reduced pressure. The resulting reaction mixture was purified by silica gel column chromatography (CHCl₃) and solidified with a mixture of DCM/Hex to afford 8.2 g (yield: 71.2%) of Intermediate **40** as a white solid.

### Synthesis Example 20: Synthesis of Intermediate 41

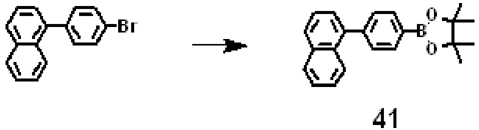

20.0 g (70.6 mmol) of 1-(4-bromophenyl)naphthalene, 21.5 g (84.8 mmol) of bis(pinacolato)diboron, 1.7 g (2.1 mmol) of Pd(dppf)Cl₂·CH₂Cl₂, 12.5 g (127.1 mmol) of KOAc, and 350 mL of 1,4-dioxane were mixed together. The mixture was stirred at 100 °C for 12 h. After completion of the reaction, the reaction mixture was cooled to room temperature, passed through a pad of celite, concentrated under reduced pressure, and purified by silica gel column chromatography (Hex:CHCl₃) to afford 19.8 g (yield: 85.0%) of Intermediate **41** as a yellow liquid.

### Synthesis Example 21: Synthesis of Intermediate 44

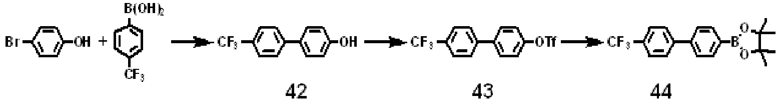

### (Synthesis of Intermediate 42)

40.0 g (231.2 mmol) of 4-bromophenol, 48.3 g (254.3 mmol) of 4-(trifluoromethyl)phenylboronic acid, 800 mL of toluene, and 400 mL of ethanol were placed in a 2 L one-neck flask, and 13.4 g (11.6 mmol) of Pd(PPh₃)₄ and 405 mL (809.2 mmol) of a 2 M K₂CO₃ solution were added thereto. The mixture was stirred at 90 °C for 5 h. After completion of the reaction, the reaction mixture was extracted with ethyl acetate and distilled water. The organic layer was separated, dried over magnesium sulfate, concentrated under reduced pressure to remove the solvent, purified by silica gel column chromatography (EA:Hex), and solidified with dichloromethane and hexane to afford 20.1 g (yield: 36.5%) of Intermediate **42** as a white solid.

### (Synthesis of Intermediate 43)

20.1 g (84.4 mmol) of Intermediate **42** and 422 mL of dichloromethane were placed in a 1 L one-neck flask. The mixture was cooled to 0 °C. After addition of 20.5 mL (253.2 mmol) of pyridine, 17.1 mL (101.3 mmol) of trifluoromethanesulfonic anhydride was slowly added dropwise. The resulting mixture was stirred at 0 °C for 10 min. The reaction mixture was heated to room temperature, followed by stirring for one day. After completion of the reaction, the reaction mixture was extracted with dichloromethane and distilled water. The organic layer was separated, dried over magnesium sulfate, concentrated under reduced pressure to remove the solvent, purified by silica gel column chromatography (DCM), and solidified with hexane to afford 25.6 g (yield: 82.2%) of Intermediate **43** as a white solid.

### (Synthesis of Intermediate 44)

25.6 g (69.1 mmol) of Intermediate **43,** 26.3 g (103.7 mmol) of bis(pinacolato)diboron, 2.8 g (3.5 mmol) of Pd(dppf)Cl₂·CH₂Cl₂, 20.4 g (207.4 mmol) of KOAc, and 461 mL of 1,4-dioxane were placed in a 1 L one-neck flask. The mixture was stirred at 100 °C for one day. After completion of the reaction, the reaction mixture was cooled to room temperature, filtered through celite with dichloromethane, concentrated under reduced pressure to remove the solvent, purified by silica gel column chromatography (DCM:Hex), and solidified with hexane to afford 18.9 g (yield: 78.7%) of Intermediate **44** as a white solid.

### Synthesis Example 22: Synthesis of Intermediate 45

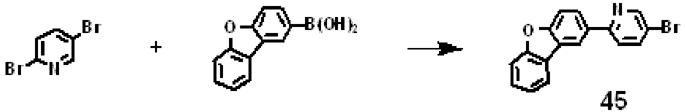

10.0 g (42.2 mmol) of 4-dibenzofuran boronic acid, 9.0 g (42.2 mmol) of 2,5-dibromopyridine, 1.5 g (1.3 mmol) of Pd(PPh₃)₄, 11.7 g (84.4 mmol) of K₂CO₃, 120 mL of toluene, 60 mL of purified water, and 60 mL of ethanol were mixed tougher. The mixture was allowed to react at 110 °C for 5 h. After completion of the reaction, the reaction mixture was cooled to room temperature, added with purified water, and extracted with ethyl acetate. The solvent was removed under reduced pressure. The resulting reaction mixture was dissolved in chloroform, filtered through silica gel, concentrated under reduced pressure to remove the solvent, and solidified with methanol/hexane to afford 8.3 g (yield: 60.7%) of Intermediate **45** as a white solid.

The synthesized intermediates were used to prepare various organic compounds as follows.

### Example 1: Synthesis of Compound 3-1 (LT20-35-203)

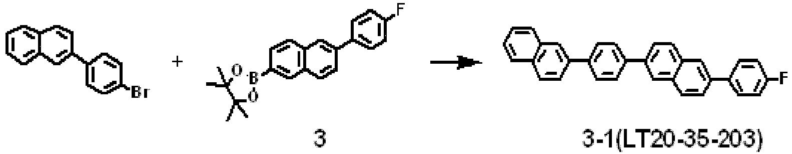

3.0 g (10.6 mmol) of 2-(4-bromophenyl)naphthalene, 3.7 g (10.6 mmol) of Intermediate **3,** 367.3 mg (317.8 µmol) of Pd(PPh₃)₄, 4.2 g (21.2 mmol) of K₃PO₄, 40 mL of toluene, 10 mL of ethanol, and 10 mL of water were mixed together. The mixture was stirred under reflux for 4 h. After completion of the reaction, the reaction mixture was cooled to room temperature. The solid was collected by filtration, washed with water and ethanol, and dried. The resulting solid mixture was purified by silica gel column chromatography (CHCl₃) and solidified with chloroform to give 3.1 g (yield: 64.9%) of Compound **3-1** (LT20-35-203) as a white solid.

### Example 2: Synthesis of Compound 3-4 (LT20-30-101)

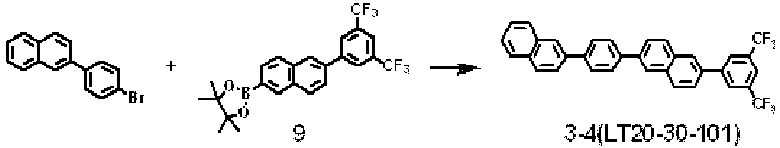

2.5 g (8.8 mmol) of 2-(4-bromophenyl)naphthalene, 4.9 g (8.8 mmol) of Intermediate **9,** 305.0 mg (263.9 µmol) of Pd(PPh₃)₄, 3.0 g (21.4 mmol) of K₂CO₃, 100 mL of toluene, 50 mL of ethanol, and 50 mL of distilled water were mixed together in a 250 mL one-neck flask. The mixture was stirred under reflux for 18 h. After completion of the reaction, the solid was collected by filtration, washed with water and methanol, dried, added with toluene, filtered under heating, dissolved in monochlorobenzene (MCB), filtered through a pad of silica, and solidified with MeOH to give 3.1 g (yield: 64.6%) of Compound **3-4** (LT20-30-101) as a white solid.

### Example 3: Synthesis of Compound 3-8 (LT19-30-186)

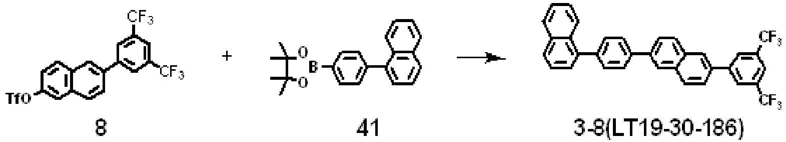

2.5 g (5.12 mmol) of Intermediate **8,** 1.78 g (5.38 mmol) of Intermediate **41,** 0.2 g (0.2 mmol) of Pd(PPh₃)₄, 6.0 mL (12.0 mmol) of 2 M K₂CO₃, 15 mL of toluene, and 6 mL of ethanol were mixed together. The mixture was stirred under reflux for 12 h. After the reaction mixture was cooled to room temperature, the resulting precipitate was collected by filtration under reduced pressure, washed with toluene, water, and methanol, dissolved in 24 mL of chloroform, filtered through a pad of celite, and washed with 16 mL of chloroform. To the filtrate was added 40 mL of methanol. Thereafter, the mixture was stirred for 2 h. The resulting precipitate was collected by filtration, washed with methanol, and dried under reduced pressure to give 1.30 g (yield: 47.0%) of Compound **3-8** (LT19-30-186) as a white solid.

### Example 4: Synthesis of Compound 3-16 (LT20-30-079)

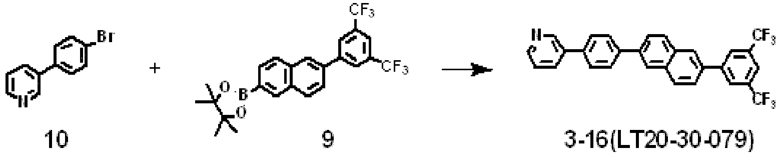

2.0 g (8.6 mmol) of Intermediate **10,** 4.0 g (8.6 mmol) of Intermediate **9,** 297.0 mg (257.0 µmol) of Pd(PPh₃)₄, 3.0 g (21.4 mmol) of K₂CO₃, 80 mL of toluene, 40 mL of ethanol, and 40 mL of distilled water were mixed together in a 250 mL one-neck flask. The mixture was stirred under reflux for 19 h. After completion of the reaction, the reaction mixture was cooled to room temperature. The solid was collected by filtration, washed with water and methanol, dried, dissolved in chloroform (CHCl₃), filtered through a pad of silica, and solidified with MeOH to give 1.7 g (yield: 40.2%) of Compound **3-16** (LT20-30-079) as a white solid.

### Example 5: Synthesis of Compound 3-20 (LT20-30-111)

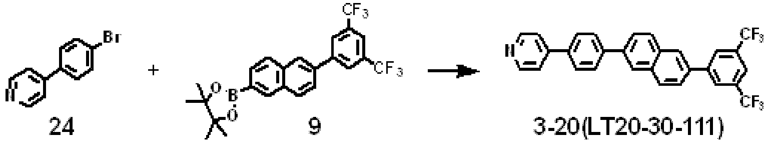

2.3 g (9.8 mmol) of Intermediate **24,** 4.5 g (9.8 mmol) of Intermediate **9,** 0.5 g (0.5 mmol) of Pd(PPh₃)₄, 2.7 g (19.6 mmol) of K₂CO₃, 30 mL of toluene, 15 mL of purified water, and 12 mL of ethanol were mixed together in a 250 mL two-neck flask. The mixture was allowed to react at 110 °C for 4 h. After completion of the reaction, the reaction mixture was cooled to room temperature, filtered, dissolved in chloroform, filtered through silica gel, and solidified with chloroform/methanol to give 2.2 g (yield: 46.1%) of Compound **3-20** (LT20-30-111) as a white solid.

### Example 6: Synthesis of Compound 3-23 (LT20-30-165)

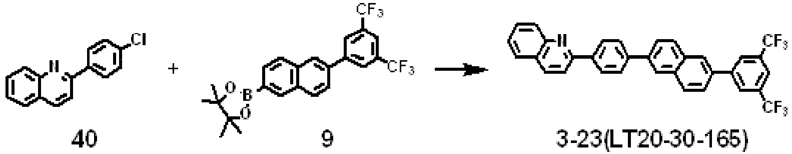

3.0 g (12.5 mmol) of Intermediate **40,** 5.8 g (12.5 mmol) of Intermediate 9, 433.9 mg (375.5 µmol) of Pd(PPh₃)₄, 4.9 g (25.0 mmol) of K₃PO₄, 40 mL of toluene, 10 mL of ethanol, and 10 mL of water were mixed together. The mixture was stirred under reflux for 4 h. After completion of the reaction, the reaction mixture was cooled to room temperature. The solid was collected by filtration, washed with water and ethanol, and dried. The resulting solid mixture was purified by silica gel column chromatography (CHCl₃) and solidified with chloroform to give 2.9 g (yield: 42.6%) of Compound **3-23** (LT20-30-165) as a white solid.

### Example 7: Synthesis of Compound 3-24 (LT20-30-081)

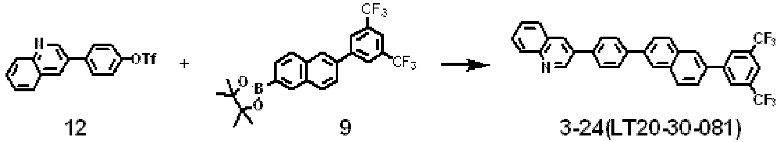

3.0 g (8.5 mmol) of Intermediate **12,** 4.8 g (10.2 mmol) of Intermediate **9,** 294.4 mg (254.7 µmol) of Pd(PPh₃)₄, 3.5 g (25.5 mmol) of K₂CO₃, 40 mL of toluene, 10 ml of ethanol, and 10 ml of distilled water were mixed together in a 250 mL two-neck flask. The mixture was allowed to react at 90 °C for 5 h. After completion of the reaction, the reaction mixture was cooled to room temperature. The solid was collected by filtration, washed with water, methanol, and hexane, and dried. The resulting solid mixture was dissolved in hot toluene, filtered through a pad of silica, and solidified with toluene to give 3.4 g (yield: 74.3%) of Compound **3-24** (LT20-30-081) as a white solid.

### Example 8: Synthesis of Compound 3-32 (LT20-30-078)

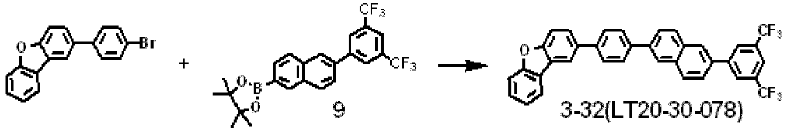

2.8 g (6.1 mmol) of Intermediate **9,** 1.9 g (6.1 mmol) of 2-(4-bromophenyl)dibenzo[b,d]furan, 211.0 mg (183.0 µmol) of Pd(PPh₃)₄, 2.1 g (15.2 mmol) of K₂CO₃, 80 mL of toluene, 40 mL of ethanol, and 40 mL of distilled water were mixed together in a 250 mL one-neck flask. The mixture was stirred under reflux for 17 h. After completion of the reaction, the reaction mixture was cooled to room temperature. The solid was collected by filtration, washed with water and methanol, dried, dissolved in chloroform (CHCl₃), filtered through a pad of silica, and solidified with a mixture of DCM/MeOH to give 2.2 g (yield: 62.4%) of Compound **3-32** (LT20-30-078) as a white solid.

### Example 9: Synthesis of Compound 3-36 (LT20-30-092)

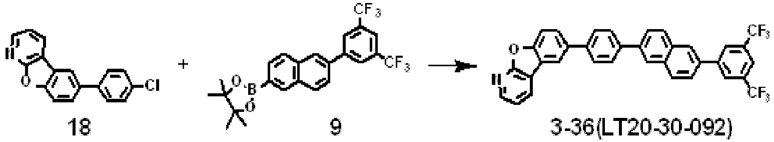

2.7 g (9.6 mmol) of Intermediate **18,** 4.5 g (9.6 mmol) of Intermediate **9,** 0.5 g (0.9 mmol) of Pd(dba)₂, 0.9 g (1.8 mmol) of X-Phos, 4.1 g (19.3 mmol) of K₃PO₄, 32 mL of toluene, and 13 mL of purified water were mixed together in a 250 mL two-neck flask. The mixture was allowed to react at 100 °C for 4 h. After completion of the reaction, the reaction mixture was cooled to room temperature, filtered, dissolved in chloroform, filtered through silica gel, and solidified with chloroform/methanol to give 3.4 g (yield: 61.4%) of Compound **3-36** (LT20-30-092) as a white solid.

### Example 10: Synthesis of Compound 3-38 (LT20-35-105)

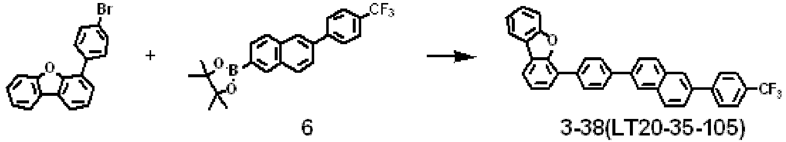

3.0 g (9.3 mmol) of 4-(4-bromophenyl)dibenzo[b,d]furan, 3.7 g (9.3 mmol) of Intermediate **6,** 321.8 mg (278.5 µmol) of Pd(PPh₃)₄, 3.6 g (18.6 mmol) of K₃PO₄, 40 mL of toluene, 10 mL of ethanol, and 10 mL of water were mixed together. The mixture was stirred under reflux for 4 h. After completion of the reaction, the reaction mixture was cooled to room temperature. The solid was collected by filtration, washed with water and ethanol, and dried. The resulting solid mixture was purified by silica gel column chromatography (CHCl₃) and solidified with chloroform to give 2.0 g (yield: 41.9%) of Compound **3-38** (LT20-35-105) as a white solid.

### Example 11: Synthesis of Compound 3-40 (LT19-30-193)

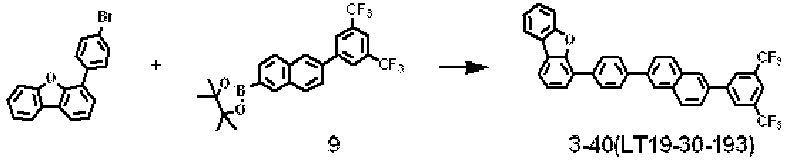

3.5 g (7.6 mmol) of Intermediate **9,** 2.4 g (8.4 mmol) of 4-(4-bromophenyl)dibenzo[b,d]furan, 0.3 g (0.2 mmol) of Pd(PPh₃)₄, 2.6 g (19 mmol) of K₂CO₃, 500 mL of toluene, 25 mL of EtOH, and 25 mL of distilled water were mixed together in a 250 mL one-neck flask. The mixture was stirred at 100 °C for 15 h. After completion of the reaction, the reaction mixture was cooled to room temperature, washed with DCM and distilled water, purified by silica gel column chromatography (DCM), and solidified with EA to give 2.6 g (yield: 59.3%) of Compound **3-40** (LT19-30-193) as a white solid.

### Example 12: Synthesis of Compound 3-48 (LT20-30-093)

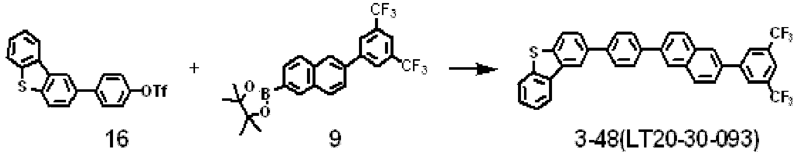

3.0 g (7.3 mmol) of Intermediate **16,** 4.1 g (8.8 mmol) of Intermediate **9,** 254.6 mg (220.4 µmol) of Pd(PPh₃)₄, 3.0 g (22.0 mmol) of K₂CO₃, 40 mL of toluene, 10 mL of ethanol, and 10 mL of distilled water were mixed together in a 250 mL two-neck flask. The mixture was allowed to react at 90 °C for one day. After completion of the reaction, the reaction mixture was cooled to room temperature. The solid was collected by filtration, washed with water, methanol, ethyl acetate, and hexane, and dried. The resulting solid mixture was dissolved in hot toluene, filtered through a pad of silica, and solidified with toluene to give 1.6 g (yield: 37.1%) of Compound **3-48** (LT20-30-093) as a white solid.

### Example 13: Synthesis of Compound 3-56 (LT20-30-090)

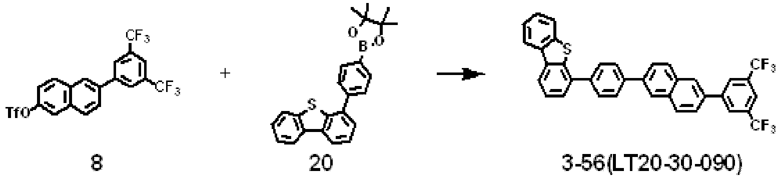

4.5 g (9.2 mmol) of Intermediate **8,** 3.6 g (9.3 mmol) of Intermediate **20,** 0.5 g (0.5 mmol) of Pd(PPh₃)₄, 2.6 g (18.4 mmol) of K₂CO₃, 54 mL of toluene, 27 mL of purified water, and 18 mL of ethanol were mixed together in a 250 mL two-neck flask. The mixture was allowed to react at 110 °C for 5 h. After completion of the reaction, the reaction mixture was cooled to room temperature, filtered, dissolved in chloroform, filtered through silica gel, and solidified with methanol to give 3.6 g (yield: 66.4%) of Compound **3-56** (LT20-30-090) as a white solid.

### Example 14: Synthesis of Compound 3-64 (LT20-30-099)

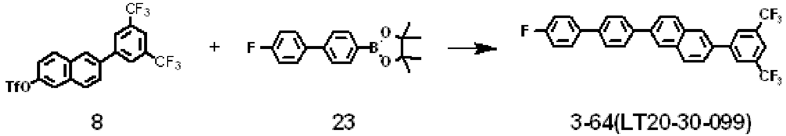

2.7 g (9.2 mmol) of Intermediate **23,** 4.5 g (9.2 mmol) of Intermediate **8,** 319.4 mg (276.4 µmol) of Pd(PPh₃)₄, 3.2 g (23.0 mmol) of K₂CO₃, 80 mL of toluene, 40 mL of ethanol, and 40 mL of distilled water were mixed together in a 250 mL one-neck flask. The mixture was stirred under reflux for 48 h. After completion of the reaction, the reaction mixture was cooled to room temperature, washed with DCM and distilled water, purified by silica gel column chromatography (DCM: n-hex), and solidified with a mixture of DCM/MeOH to give 3.3 g (yield: 70.3%) of Compound **3-64** (LT20-30-099) as a white solid.

### Example 15: Synthesis of Compound 3-65 (LT20-30-083)

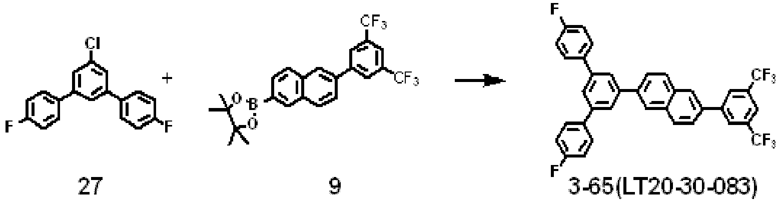

2.0 g (6.7 mmol) of Intermediate **27,** 3.7 g (8.0 mmol) of Intermediate **9,** 382.4 mg (665.0 µmol) of Pd(dba)₂, 634.0 mg (1.3 mmol) of X-Phos, 4.2 g (20.0 mmol) of K₃PO₄, and 35 mL of xylene were mixed together. The mixture was stirred under reflux for 5 h. After completion of the reaction, the reaction mixture was cooled to room temperature, added with water, and extracted with chloroform. The solvent was removed under reduced pressure. The resulting reaction mixture was purified by silica gel column chromatography (Hex:DCM) and solidified with a mixture of DCM/MeOH to give 2.0 g (yield: 50.5%) of Compound **3-65** (LT20-30-083) as a white solid.

### Example 16: Synthesis of Compound 3-69 (LT20-30-088)

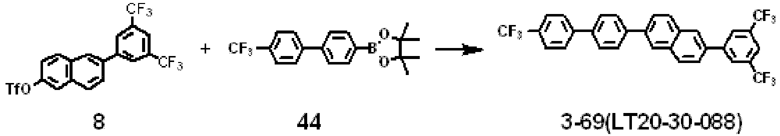

4.5 g (9.2 mmol) of Intermediate **8,** 3.2 g (9.2 mmol) of Intermediate **44,** 0.5 g (0.5 mmol) of Pd(PPh₃)₄, 2.5 g (18.4 mmol) of K₂CO₃, 54 mL of toluene, 27 mL of purified water, and 18 mL of ethanol were mixed together in a 250 mL two-neck flask. The mixture was allowed to react at 110 °C for 4 h. After completion of the reaction, the reaction mixture was cooled to room temperature, filtered, dissolved in chloroform, filtered through silica gel, and solidified with methanol to give 4.5 g (yield: 88.5%) of Compound **3-69** (LT20-30-088) as a white solid.

### Example 17: Synthesis of Compound 3-70 (LT20-30-089)

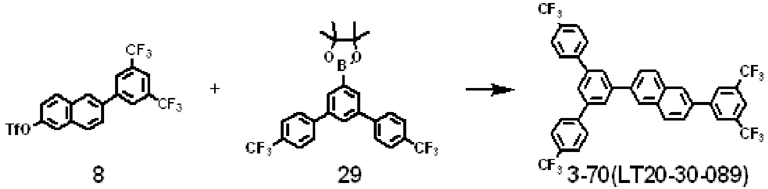

4.0 g (8.2 mmol) of Intermediate **8,** 4.1 g (8.2 mmol) of Intermediate **29,** 0.5 g (0.5 mmol) of Pd(PPh₃)₄, 2.3 g (16.4 mmol) of K₂CO₃, 48 mL of toluene, 24 mL of purified water, and 16 mL of ethanol were mixed together in a 250 mL two-neck flask. The mixture was allowed to react at 110 °C for 4 h. After completion of the reaction, the reaction mixture was cooled to room temperature, filtered, dissolved in chloroform, filtered through silica gel, and solidified with hexane to give 4.5 g (yield: 78.6%) of Compound **3-70** (LT20-30-089) as a white solid.

### Example 18: Synthesis of Compound 3-91 (LT20-30-119)

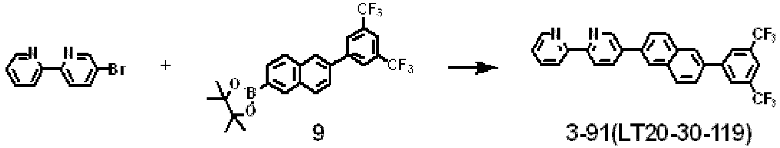

2.0 g (8.5 mmol) of 5-bromo-2,2'-bipyridine, 4.0 g (8.5 mmol) of Intermediate **9,** 491.0 mg (425.4 µmol) of Pd(PPh₃)₄, 5.4 g (25.5 mmol) of K₃PO₄, 30 mL of toluene, 10 mL of ethanol, and 10 mL of distilled water were mixed together. The mixture was stirred under reflux for 12 h. After completion of the reaction, the reaction mixture was cooled to room temperature. The solid was collected by filtration, washed with water and methanol, dried, dissolved in chloroform, purified by silica gel column chromatography (CHCl₃:EA), and solidified with a mixed solvent of DCM/MeOH to give 2.6 g (yield: 62.3%) of Compound **3-91** (LT20-30-119) as a white solid.

### Example 19: Synthesis of Compound 3-111 (LT20-30-171)

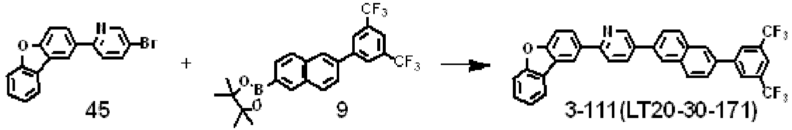

2.5 g (7.7 mmol) of Intermediate **45,** 4.0 g (8.5 mmol) of Intermediate **9,** 446.0 mg (385.6 µmol) of Pd(PPh₃)₄, 4.9 g (23.1 mmol) of K₃PO₄, 30 mL of toluene, 10 mL of ethanol, and 10 mL of water were mixed together. The mixture was stirred under reflux for 12 h. After completion of the reaction, the reaction mixture was cooled to room temperature. The solid was collected by filtration, washed with water and methanol, dried, dissolved in chloroform, purified by silica gel column chromatography (CHCl₃), and solidified with chloroform to give 4.2 g (yield: 93.3%) of Compound **3-111** (LT20-30-171) as a white solid.

### Example 20: Synthesis of Compound 4-3 (LT20-35-210)

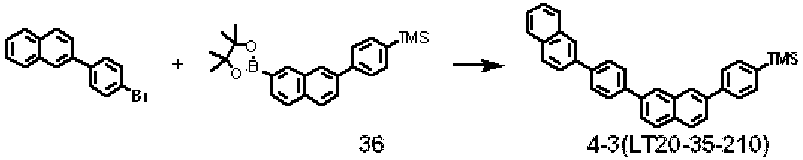

2.5 g (8.8 mmol) of 2-(4-bromophenyl)naphthalene, 4.9 g (8.8 mmol) of Intermediate **36,** 305.0 mg (263.9 µmol) of Pd(PPh₃)₄, 3.0 g (21.4 mmol) of K₂CO₃, 100 mL of toluene, 50 mL of ethanol, and 50 mL of distilled water were mixed together in a 250 mL one-neck flask. The mixture was stirred under reflux for 18 h. After completion of the reaction, the solid was collected by filtration, washed with water and methanol, dried, added with toluene, filtered under heating, dissolved in monochlorobenzene (MCB), filtered through a pad of silica (MCB), and solidified with MeOH to give 3.1 g (yield: 64.6%) of Compound **4-3** (LT20-35-210) as a white solid.

### Example 21: Synthesis of Compound 4-4 (LT20-30-178)

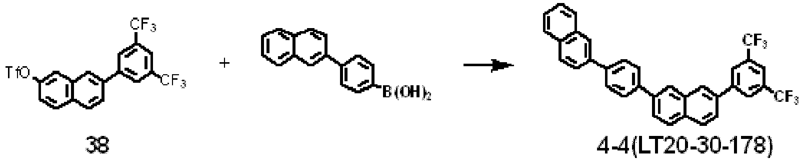

4.5 g (9.2 mmol) of Intermediate **38,** 2.3 g (9.3 mmol) of (4-(naphthalen-2-yl)phenyl)boronic acid, 0.5 g (0.5 mmol) of Pd(PPh₃)₄, 2.5 g (18.4 mmol) of K₂CO₃, 54 mL of toluene, 27 mL of purified water, and 18 mL of ethanol were mixed together in a 250 mL two-neck flask. The mixture was allowed to react at 110 °C for 4 h. After completion of the reaction, the reaction mixture was cooled to room temperature, filtered, dissolved in chloroform, filtered through silica gel, and solidified with chloroform/methanol to give 4.5 g (yield: 90.0%) of Compound **4-4** (LT20-30-178) as a white solid.

### Example 22: Synthesis of Compound 4-16 (LT20-30-162)

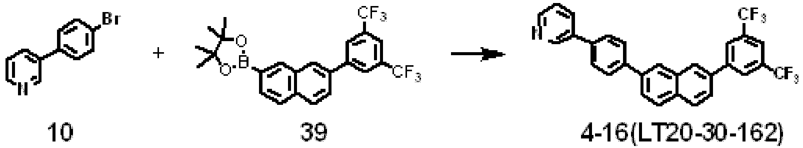

3.9 g (13.8 mmol) of Intermediate **10,** 4.5 g (9.2 mmol) of Intermediate **39,** 319.0 mg (276.0 µmol) of Pd(PPh₃)₄, 3.2 g (23.0 mmol) of K₂CO₃, 80 mL of toluene, 40 mL of ethanol, and 40 mL of water were mixed together in a 250 mL one-neck flask. The mixture was stirred under reflux for 20 h. After completion of the reaction, the reaction mixture was cooled to room temperature, washed with CHCl₃ and water, purified by silica gel column chromatography (DCM:EA), and solidified with MeOH to give 1.5 g (yield: 33.4%) of Compound **4-16** (LT20-30-162) as a white solid.

### Example 23: Synthesis of Compound 4-24 (LT20-30-174)

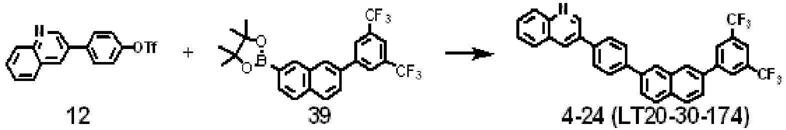

3.5 g (9.9 mmol) of Intermediate **12,** 5.0 g (10.7 mmol) of Intermediate **39,** 310.2 mg (268.4 µmol) of Pd(PPh₃)₄, 3.7 g (26.8 mmol) of K₂CO₃, 45 mL of toluene, 11 mL of ethanol, and 11 mL of distilled water were mixed together in a 250 mL two-neck flask. The mixture was allowed to react at 90 °C for one day. After completion of the reaction, the reaction mixture was cooled to room temperature, washed with 200 mL of water, and extracted with chloroform. The organic layer was separated, dried over anhydrous magnesium sulfate, filtered, and distilled under reduced pressure. The resulting solid mixture was purified by silica column (CHCl₃) and solidified with toluene to give 3.3 g (yield: 67.9%) of Compound **4-24** (LT20-30-174) as a white solid.

### Example 24: Synthesis of Compound 4-29 (LT20-35-215)

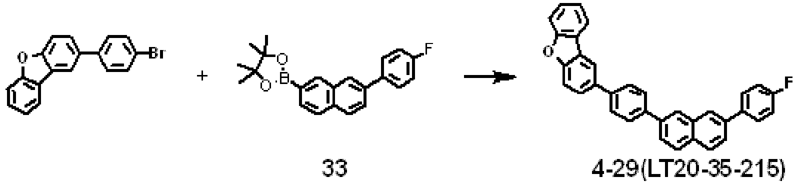

2.8 g (6.1 mmol) of Intermediate **33,** 1.9 g (6.1 mmol) of 2-(4-bromophenyl)dibenzo[b,d]furan, 211.0 mg (183.0 µmol) of Pd(PPh₃)₄, 2.1 g (15.2 mmol) of K₂CO₃, 80 mL of toluene, 40 mL of ethanol, and 40 mL of distilled water were mixed together in a 250 mL one-neck flask. The mixture was stirred under reflux for 17 h. After completion of the reaction, the reaction mixture was cooled to room temperature. The solid was collected by filtration, washed with water and methanol, dried, dissolved in chloroform (CHCl₃), filtered through a pad of silica, and solidified with a mixture of DCM/MeOH to give 2.2 g (yield: 62.4%) of Compound **4-29** (LT20-35-215) as a white solid.

### <TEST EXAMPLES>

Monolayer films for evaluating the optical properties of the inventive compounds were prepared and their refractive indices (n) and extinction coefficients (k) were measured using an ellipsometer (J.A. WOOLLAM).

Preparation of monolayer films for evaluating optical properties:
A glass substrate (0.7T) was washed with ethanol, DI water, and acetone (each for 10 min) and treated with a 125 W oxygen plasma at 2×10⁻² Torr for 2 min. Thereafter, a compound was deposited to a thickness of 800 Å on the glass substrate at a degree of vacuum of 9×10⁻⁷ Torr and a rate of 1 Å/sec to form a monolayer film, which was used to measure the optical properties of the compound.

### Comparative Test Examples 1-2:

Monolayer films for evaluating optical properties were prepared in the same manner as described above, except that Alq₃ (Comparative Test Example 1) and REF01 (Comparative Test Example 2) were used.

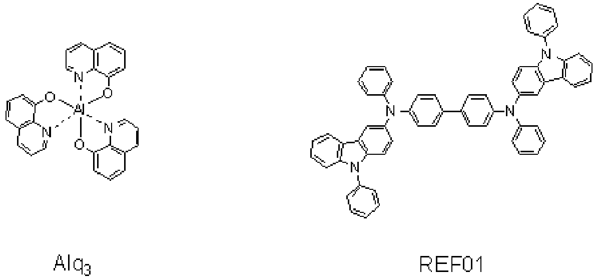

### <Test Examples 1-24>

Monolayer films for evaluating optical properties were prepared in the same manner as described above, except that the compounds shown in Table 1 were used.

The optical properties of the compounds used in Comparative Test Examples 1-2 and Test Examples 1-24 are shown in Table 1.

The optical properties were refractive indices at wavelengths of 460 nm and 620 nm.

**[Table 1]**

| | Compound | n (460 nm) | n (620 nm) |
|---|---|---|---|
| Comparative Test Example 1 | Alq₃ | 1.808 | 1.690 |
| Comparative Test Example 2 | REF01 | 1.986 | 1.846 |
| Test Example 1 | 3-1 (LT20-35-203) | 1.499 | 1.476 |
| Test Example 2 | 3-4 (LT20-30-101) | 1.635 | 1.581 |
| Test Example 3 | 3-8 (LT19-30-186) | 1.474 | 1.456 |
| Test Example 4 | 3-16 (LT20-30-079) | 1.460 | 1.443 |
| Test Example 5 | 3-20 (LT20-30-111) | 1.615 | 1.550 |
| Test Example 6 | 3-23 (LT20-30-165) | 1.446 | 1.430 |
| Test Example 7 | 3-24 (LT20-30-081) | 1.442 | 1.431 |
| Test Example 8 | 3-32 (LT20-30-078) | 1.565 | 1.525 |
| Test Example 9 | 3-36 (LT20-30-092) | 1.635 | 1.581 |
| Test Example 10 | 3-38 (LT20-35-105) | 1.499 | 1.476 |
| Test Example 11 | 3-40 (LT19-30-193) | 1.552 | 1.533 |
| Test Example 12 | 3-48 (LT20-30-093) | 1.556 | 1.506 |
| Test Example 13 | 3-56 (LT20-30-090) | 1.518 | 1.493 |
| Test Example 14 | 3-64 (LT20-30-099) | 1.615 | 1.550 |
| Test Example 15 | 3-65 (LT20-30-083) | 1.697 | 1.637 |
| Test Example 16 | 3-69 (LT20-30-088) | 1.635 | 1.581 |
| Test Example 17 | 3-70 (LT20-30-089) | 1.652 | 1.596 |
| Test Example 18 | 3-91 (LT20-30-119) | 1.499 | 1.476 |
| Test Example 19 | 3-111 (LT20-30-171) | 1.474 | 1.456 |
| Test Example 20 | 4-3 (LT20-35-210) | 1.499 | 1.476 |
| Test Example 21 | 4-4 (LT20-30-178) | 1.482 | 1.454 |
| Test Example 22 | 4-16 (LT20-30-162) | 1.486 | 1.465 |
| Test Example 23 | 4-24 (LT20-30-174) | 1.450 | 1.454 |
| Test Example 24 | 4-29 (LT20-35-215) | 1.442 | 1.431 |

As can be seen from the results in Table 1, the refractive indices (n) of Alq₃ used in Comparative Test Example 1 in the blue (460 nm) and red regions (620 nm) were 1.808 and 1.690, respectively. In contrast, most of the inventive compounds were found to have lower refractive indices (n < 1.69 at 620 nm) than Alq₃ used in Comparative Test Example 1 in the blue, green, and red regions. The refractive indices of the inventive compounds were low enough to secure wide viewing angles in the blue wavelength range.

### <EXAMPLES>

### Device fabrication

A transparent ITO electrode was used as an anode, 2-TNATA was used as a material for a hole injection layer, NPB was used as a material for a hole transporting layer, αβ-ADN and Pyene-CN were used as a host and a blue fluorescent dopant for an emission layer, respectively, Liq was used as a material for an electron injection layer, and Mg:Ag was used as a material for a cathode to fabricate a device. The structures of 2-TNATA, NPB, and αβ-ADN are as follow:

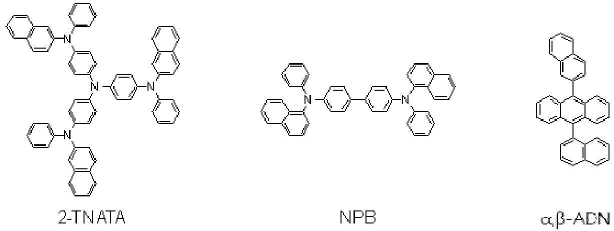

Comparative Example 1 (without capping layer): ITO/2-TNATA (60 nm)/NPB (20 nm)/αβ-ADN: 10% pyrene-CN (30 nm)/Alq₃ (30 nm)/Liq (2 nm)/Mg:Ag (1:9,10 nm)

Comparative Example 2 (with capping layer consisting of one layer): ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)/Alq₃ (80 nm)

Example 1 (with capping layer consisting of two layers): ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)/inventive compound (20 nm, low refractive index compound)/REF01 (60 nm, high refractive index compound)

ITO (180 nm), 2-TNATA (60 nm), NPB (20 nm), αβ-ADN:pyrene-CN 10% (30 nm), Alq₃ (30 nm), Liq (2 nm), Mg:Ag (1:9, 10 nm), and a capping layer were deposited in this order to fabricate a blue fluorescent organic light emitting diode.

Before deposition of the organic materials, the ITO electrode was treated with a 125 W oxygen plasma at 2 × 10⁻² Torr for 2 min. The organic materials were deposited at a degree of vacuum of 9 × 10⁻⁷ Torr. Liq was deposited at a rate of 0.1 Å/sec, αβ-ADN and pyrene-CN were deposited simultaneously at rates of 0.18 Å/sec and 0.02 Å/sec, respectively, and the other organic materials were deposited at a rate of 1 Å/sec.

The device was sealed in a glove box filled with nitrogen gas to prevent contact with air and moisture. After a barrier was formed with an adhesive tape (3M), barium oxide as a moisture absorbent was placed to remove moisture and a glass plate was attached to the barrier.

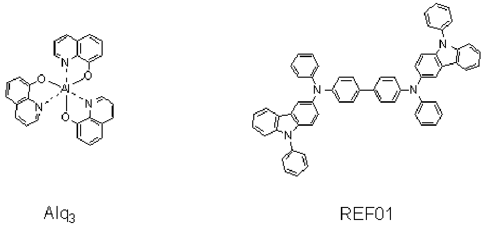

### <Examples 2-24>

An organic light emitting device was fabricated in the same manner as in Example 1, except that REF01 was used to form a high refractive index layer (60 nm), the corresponding compound shown in Table 2 was used to form a low refractive index layer (20 nm), and the high refractive index layer was formed on the low refractive index layer to form a capping layer having a multilayer structure.

The electroluminescent properties of the organic light emitting devices fabricated in Comparative Examples 1-2 and Examples 1-24 are shown in Table 2.

**[Table 2]**

| Properties | Compound | Driving voltage (V) | Efficiency (cd/A) | Lifetime (%) |
|---|---|---|---|---|
| Comparative Example 1 | - | 4.51 | 4.22 | 89.21 |
| Comparative Example 2 | Alq₃ alone | 4.50 | 5.10 | 88.92 |
| Example 1 | 3-1 (LT20-35-203) | 4.47 | 6.32 | 98.13 |
| Example 2 | 3-4 (LT20-30-101) | 4.51 | 5.31 | 95.61 |
| Example 3 | 3-8 (LT19-30-186) | 4.45 | 6.55 | 97.45 |
| Example 4 | 3-16 (LT20-30-079) | 4.38 | 6.23 | 97.40 |
| Example 5 | 3-20 (LT20-30-111) | 4.49 | 5.43 | 95.55 |
| Example 6 | 3-23 (LT20-30-165) | 4.40 | 6.00 | 98.11 |
| Example 7 | 3-24 (LT20-30-081) | 4.41 | 6.23 | 97.55 |
| Example 8 | 3-32 (LT20-30-078) | 4.45 | 6.13 | 97.45 |
| Example 9 | 3-36 (LT20-30-092) | 4.51 | 5.31 | 95.61 |
| Example 10 | 3-38 (LT20-35-105) | 4.45 | 6.13 | 97.45 |
| Example 11 | 3-40 (LT19-30-193) | 4.47 | 6.32 | 98.13 |
| Example 12 | 3-48 (LT20-30-093) | 4.49 | 5.53 | 95.61 |
| Example 13 | 3-56 (LT20-30-090) | 4.47 | 6.32 | 98.13 |
| Example 14 | 3-64 (LT20-30-099) | 4.50 | 6.15 | 97.32 |
| Example 15 | 3-65 (LT20-30-083) | 4.50 | 6.10 | 97.00 |
| Example 16 | 3-69 (LT20-30-088) | 4.48 | 6.00 | 97.01 |
| Example 17 | 3-70 (LT20-30-089) | 4.49 | 5.98 | 97.00 |
| Example 18 | 3-91 (LT20-30-119) | 4.42 | 6.11 | 97.54 |
| Example 19 | 3-111 (LT20-30-171) | 4.38 | 6.23 | 97.40 |
| Example 20 | 4-3 (LT20-35-210) | 4.40 | 6.22 | 98.11 |
| Example 21 | 4-4 (LT20-30-178) | 4.41 | 6.23 | 97.55 |
| Example 22 | 4-16 (LT20-30-162) | 4.42 | 6.11 | 97.54 |
| Example 23 | 4-24 (LT20-30-174) | 4.40 | 6.25 | 97.42 |
| Example 24 | 4-29 (LT20-35-215) | 4.42 | 6.11 | 97.54 |

The results in Table 2 reveal that the presence of the capping layer in the device of Comparative Example 2 leads to an increase in the efficiency of the device, unlike in the device without a capping layer (Comparative Example 1).

The results in Table 2 also demonstrate that the inventive organic compounds can be used as materials for low refractive index capping layers of organic electronic devices, including organic light emitting devices, and their use ensures excellent characteristics of the organic electronic devices in terms of efficiency, driving voltage, and stability.

The capping layer having a multilayer structure composed of the high refractive index compound (n > 1.69 at 620 nm) and the low refractive index compound (n < 1.69 at 620 nm) was found to increase the efficiency of the corresponding device, unlike the capping layer having a monolayer structure composed of the high refractive index compound (n > 1.69 at 620 nm). In addition, the use of the inventive compound as a material for the capping layer having a multilayer structure was found to improve the efficiency of the corresponding device compared to the use of Alq₃ as a material for the capping layer (Comparative Example 2).

These results can be explained by the refractive indices of the inventive compounds. It is obvious that the use of the capping layer having a multilayer structure composed of REF01 having a high refractive index and the inventive compound having a low refractive index ensures high efficiency of the corresponding organic electroluminescent device compared to the use of the capping layer having a monolayer structure composed of REF01.

Therefore, it can be concluded that the compound of Formula 1 has unexpectedly desirable characteristics for use as a material for a low refractive index capping layer of an OLED.

Based on its characteristics, the compound of the present invention can be used in organic electronic devices for industrial applications.

The above synthesis examples are merely illustrative and the reaction conditions may vary as required. The compounds according to exemplary embodiments of the present invention may be substituted with various substituents using suitable methods and materials known in the art. The introduction of various substituents to the core structure of the compound represented by Formula 1 allows the substituted compounds to have characteristics suitable for use in organic electroluminescent devices.

### Industrial Applicability

The organic compound of the present invention can be used as a material for a capping layer of an organic electroluminescent device to improve the quality of the device.

When the compound of the present invention is used in a capping layer consisting of a high refractive index layer and a low refractive index layer, the optical properties of the compound ensure an improvement in the lifetime of an organic electroluminescent device using the capping layer while allowing the device to exhibit its original characteristics.

## Claims

1. An organic compound for an organic electroluminescent device, represented by Formula 1: 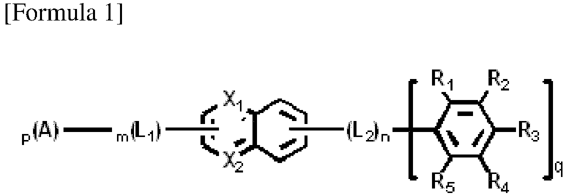 wherein X₁ and X₂ are each independently N or CH, L₁ and L₂ are each independently selected from substituted or unsubstituted phenyl groups, substituted or unsubstituted naphthyl groups, and substituted or unsubstituted pyridyl groups, R₁ to R₅ are each independently F, CF₃ or Si(CH₃)₃, p and q are each independently an integer from 1 to 5, m and n are each independently an integer from 0 to 5, provided that when m and n are 0, L₁ and L₂ are direct bonds and provided that when m and n are 2 or more, L₁ and L₂ are each independently the same as or different from each other, and A is selected from the group consisting of structures represented by the following formulae 2: 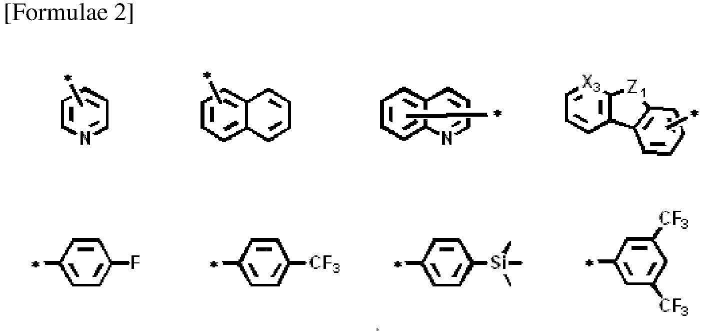 wherein Zi is O or S and X₃ is N or CH.

2. The organic compound according to claim 1, wherein the organic compound is selected from the compounds represented by Formulae 3 to 5:

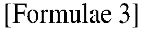

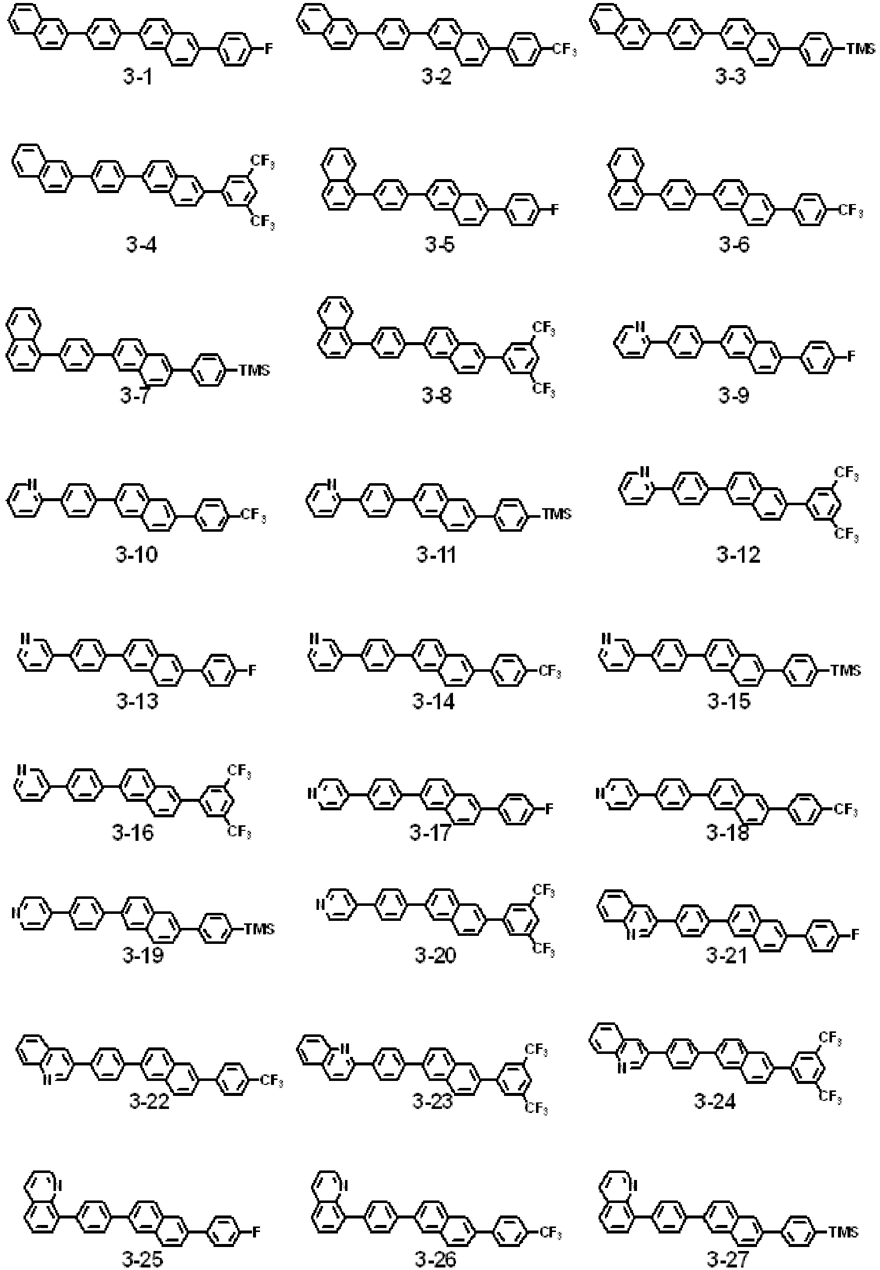

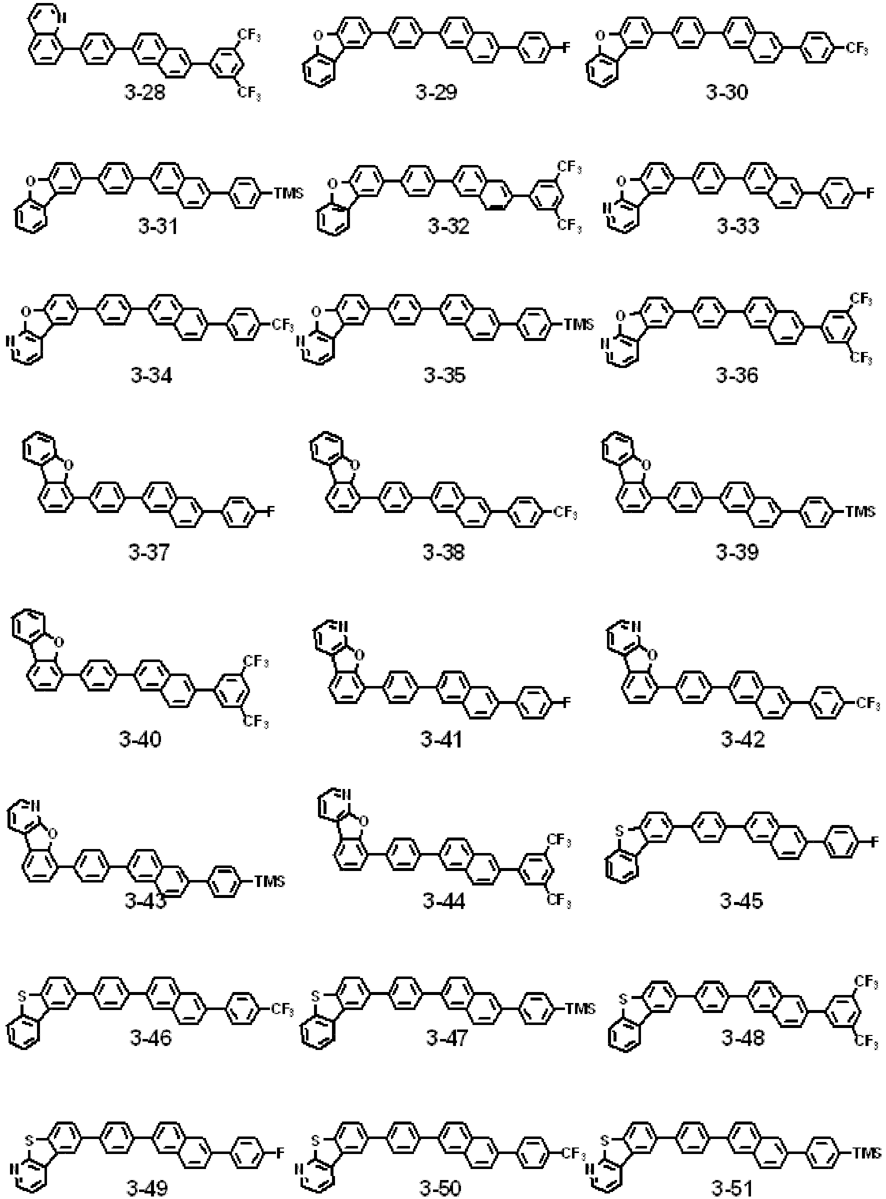

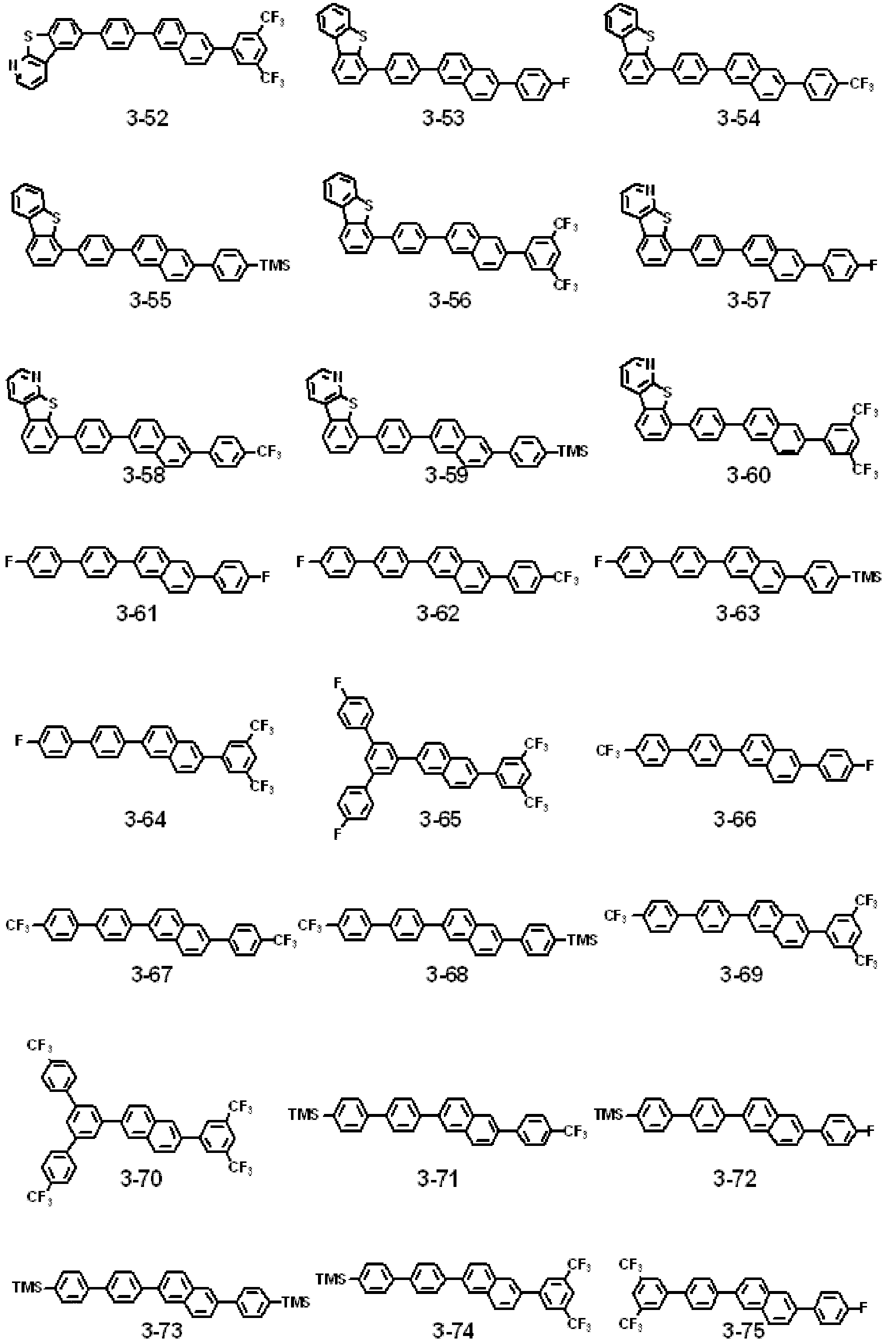

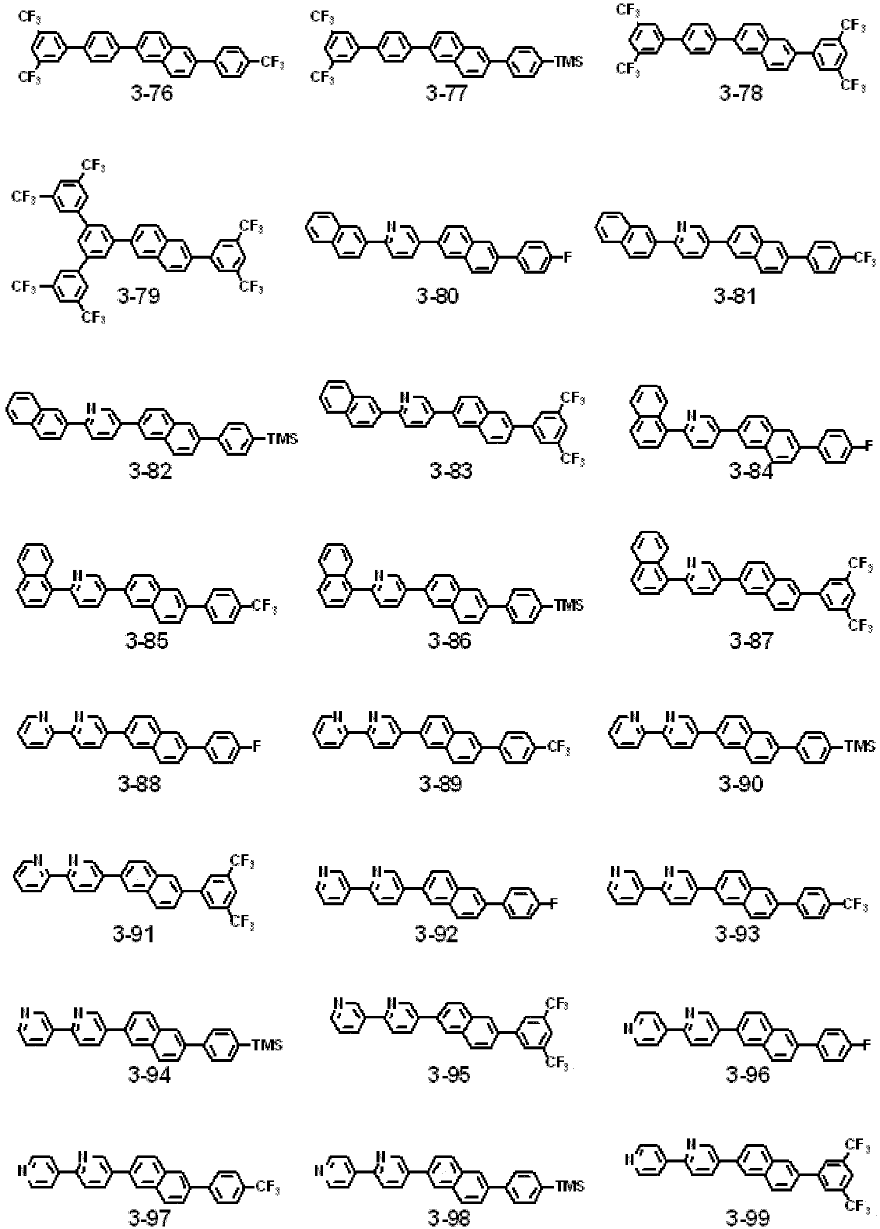

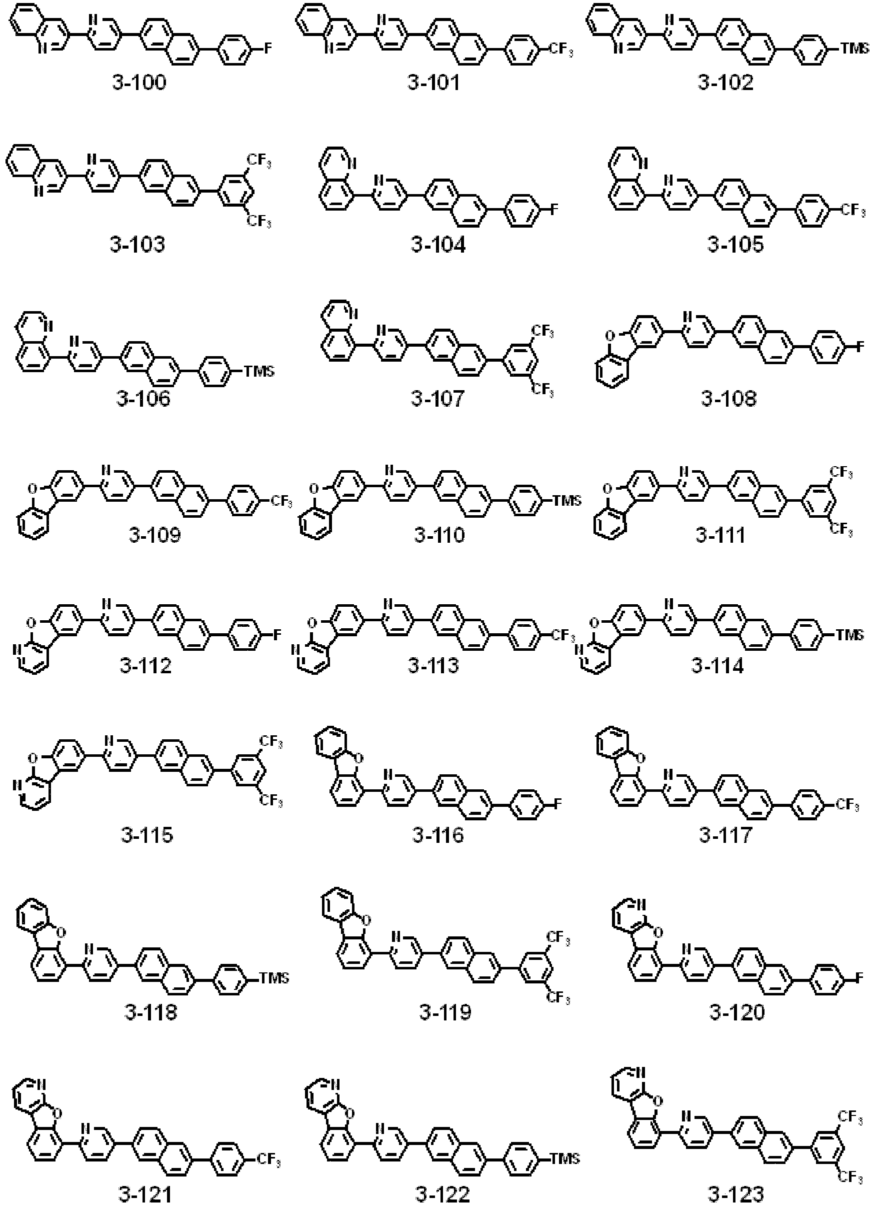

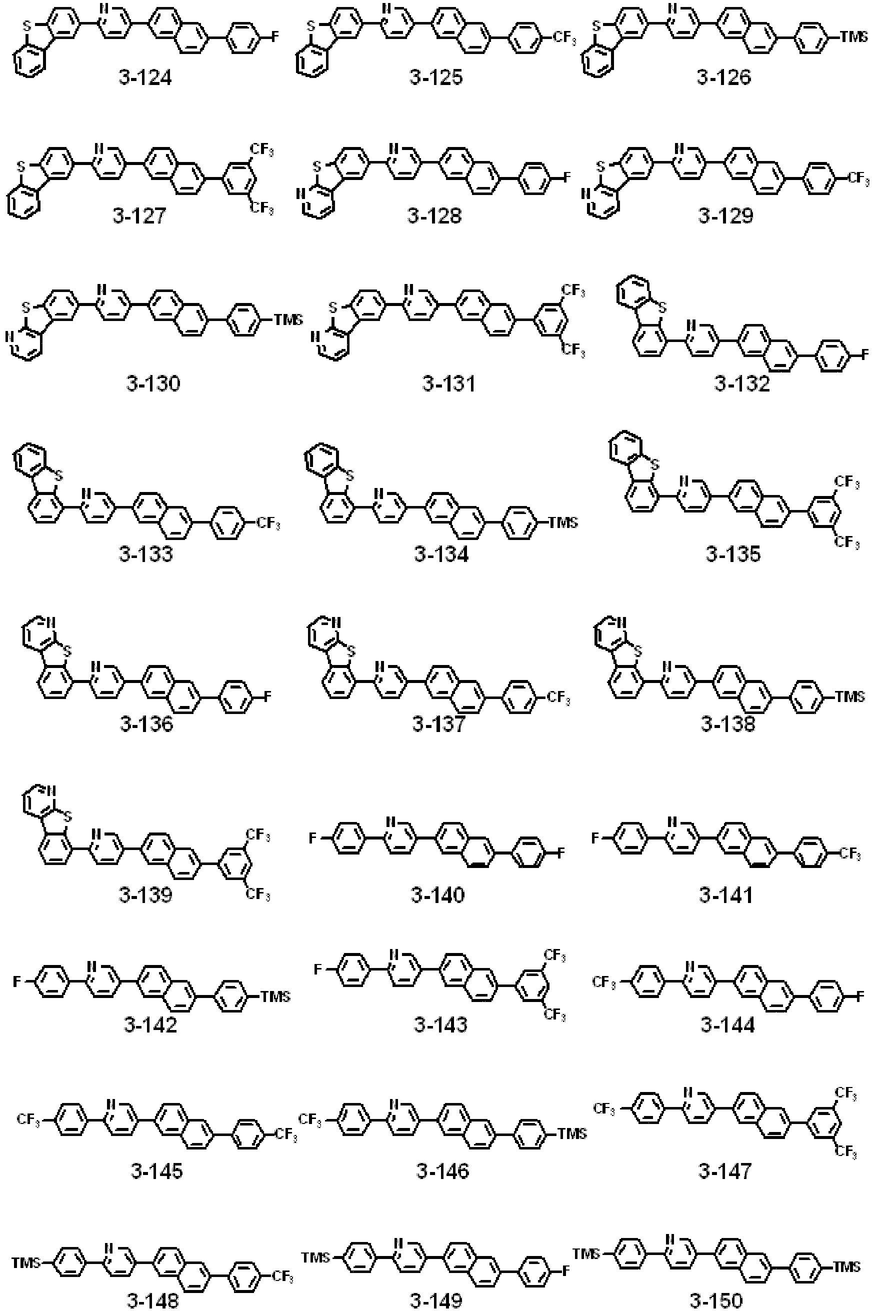

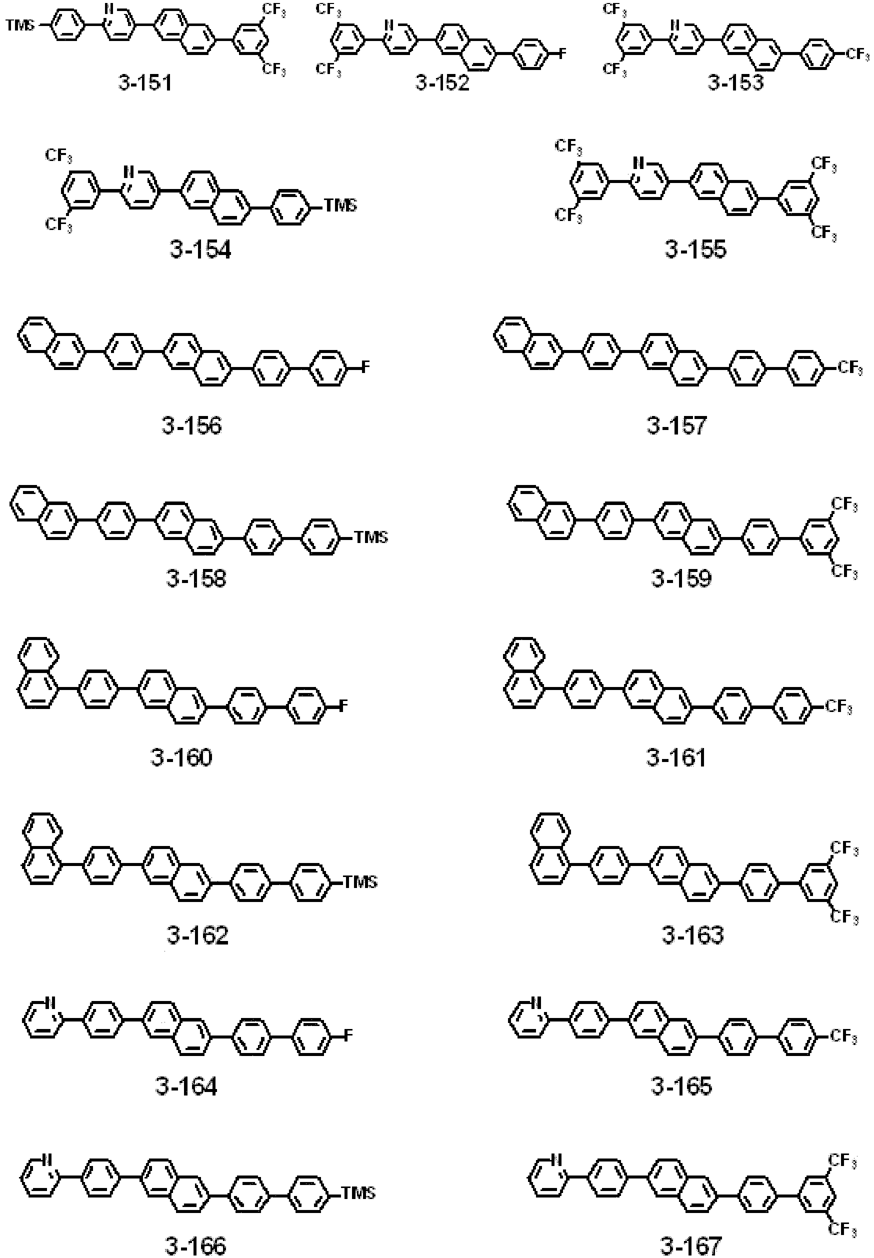

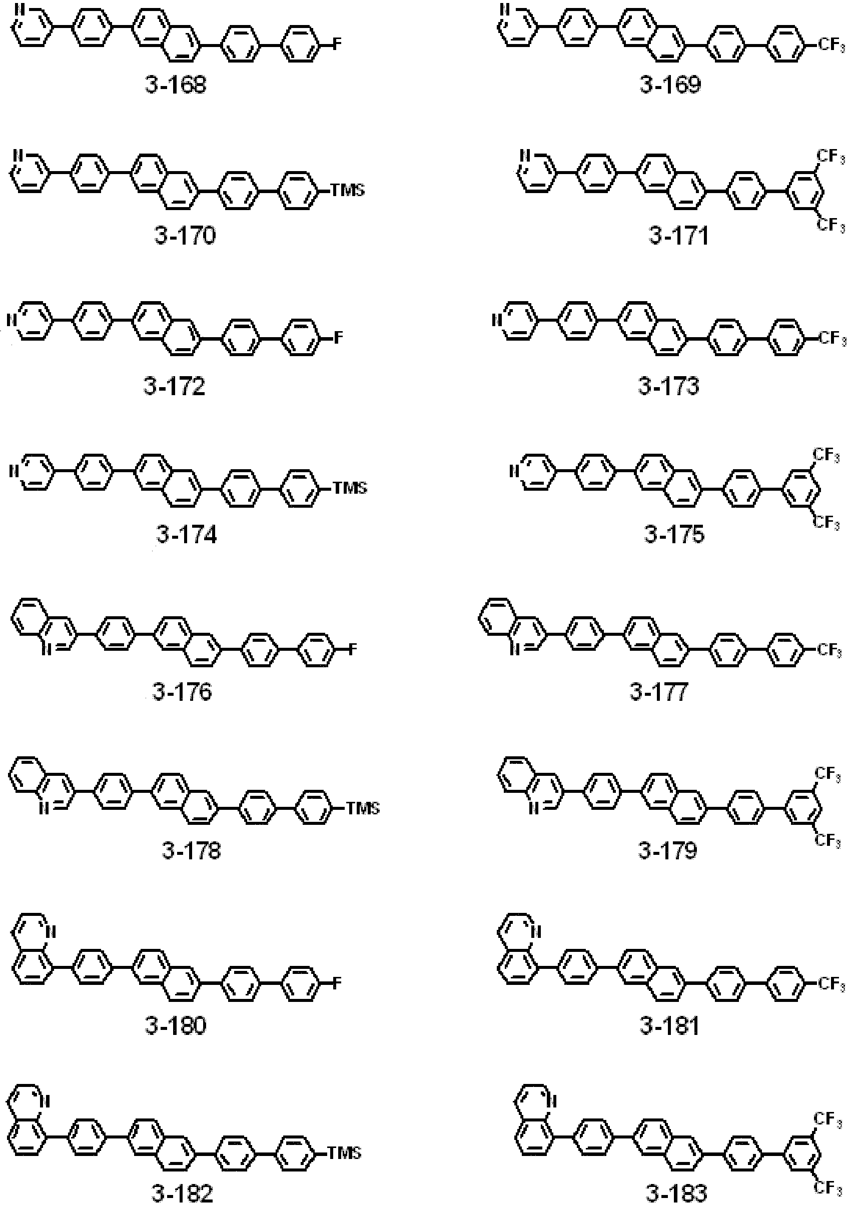

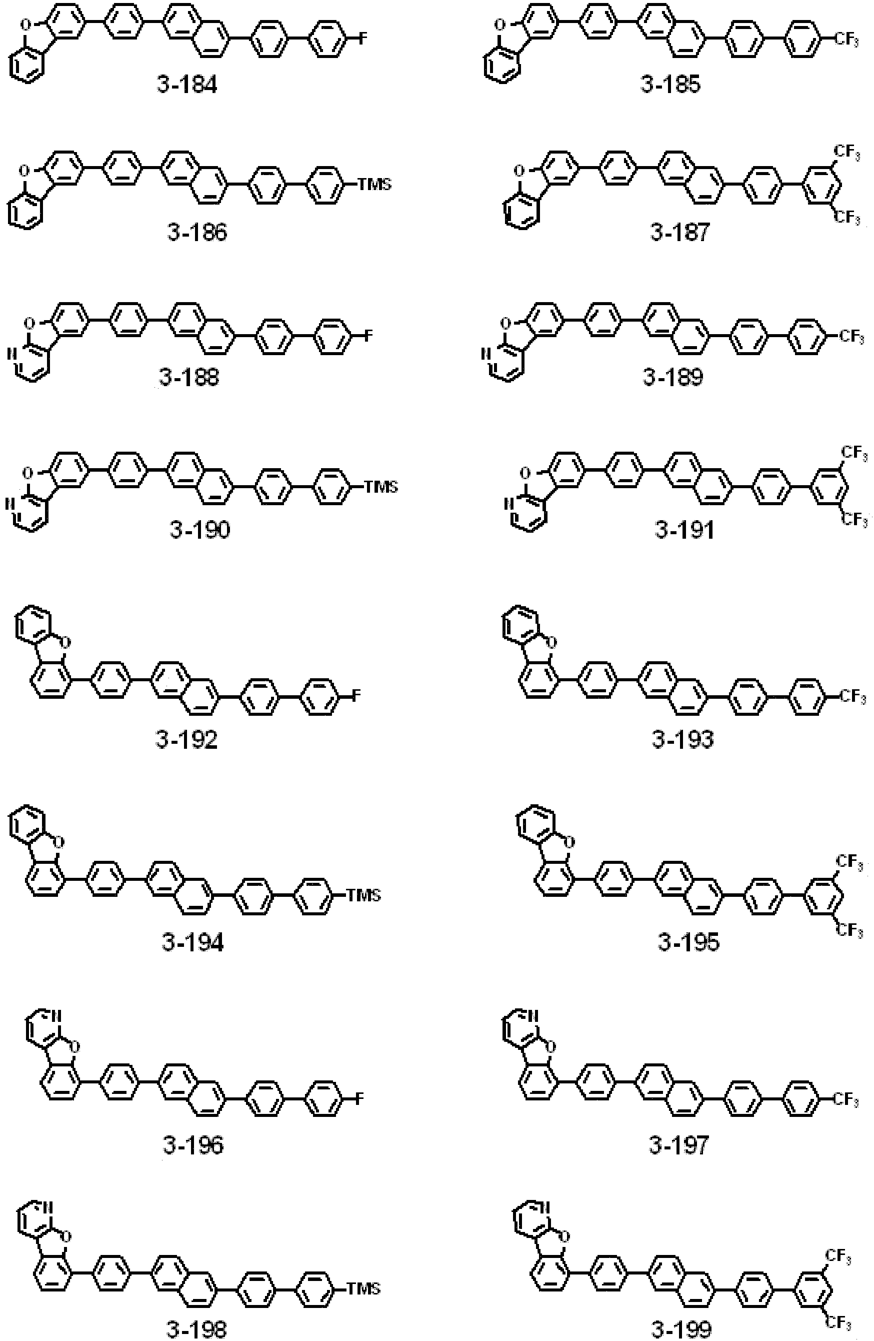

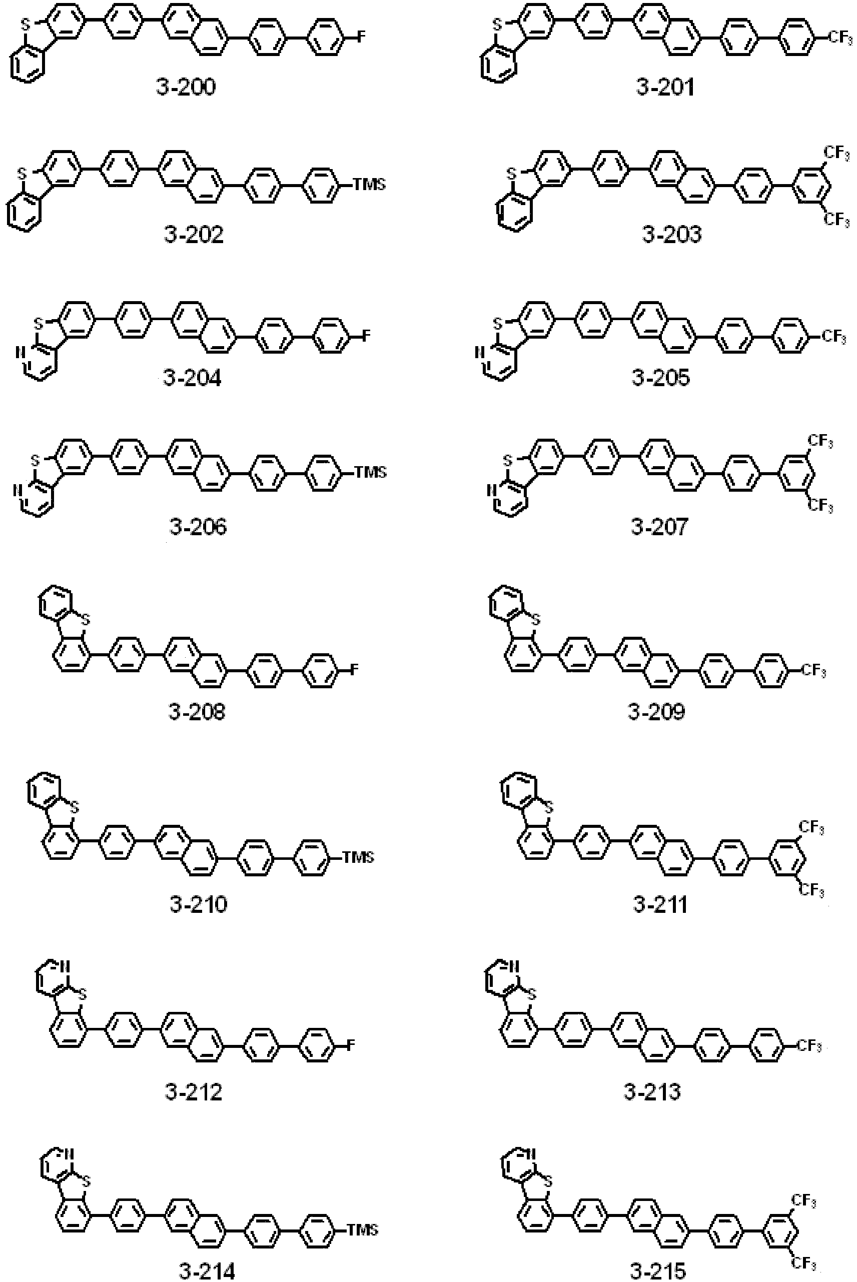

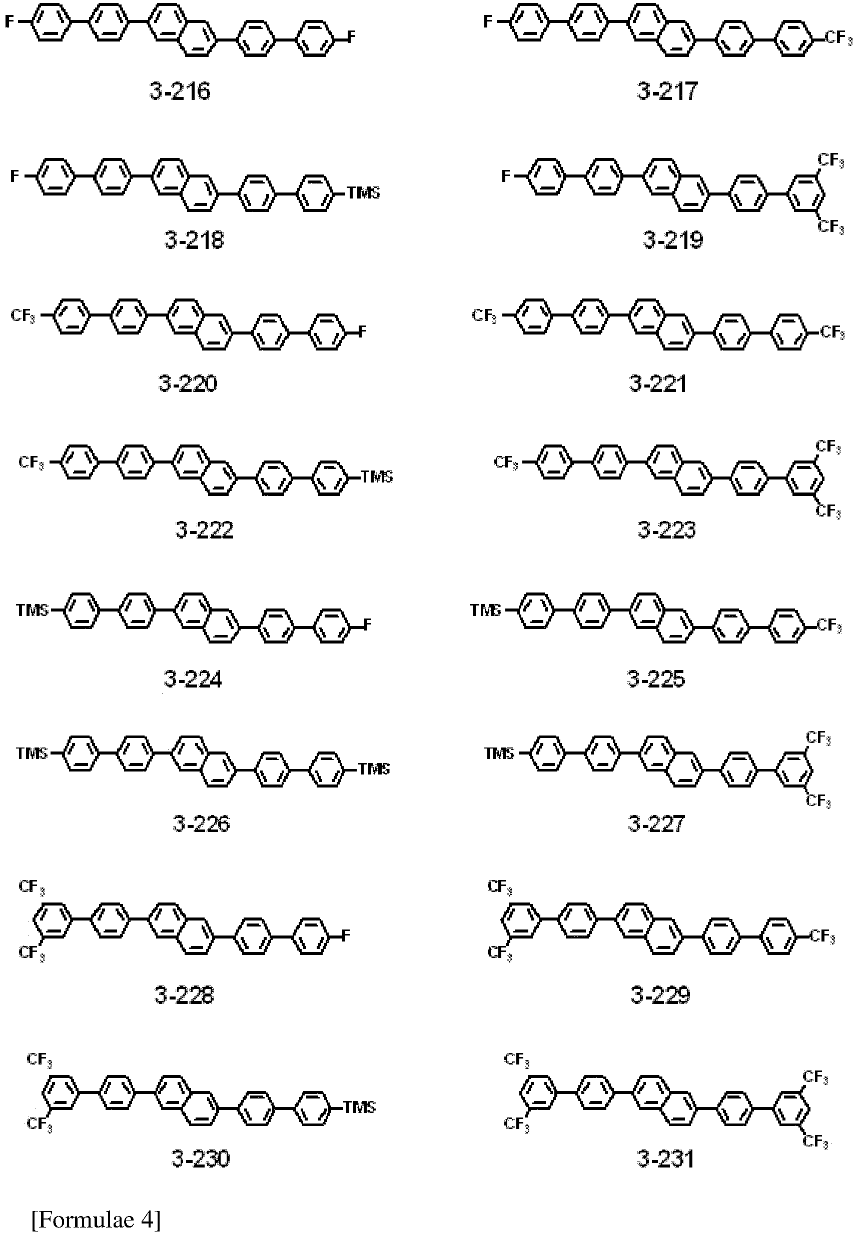

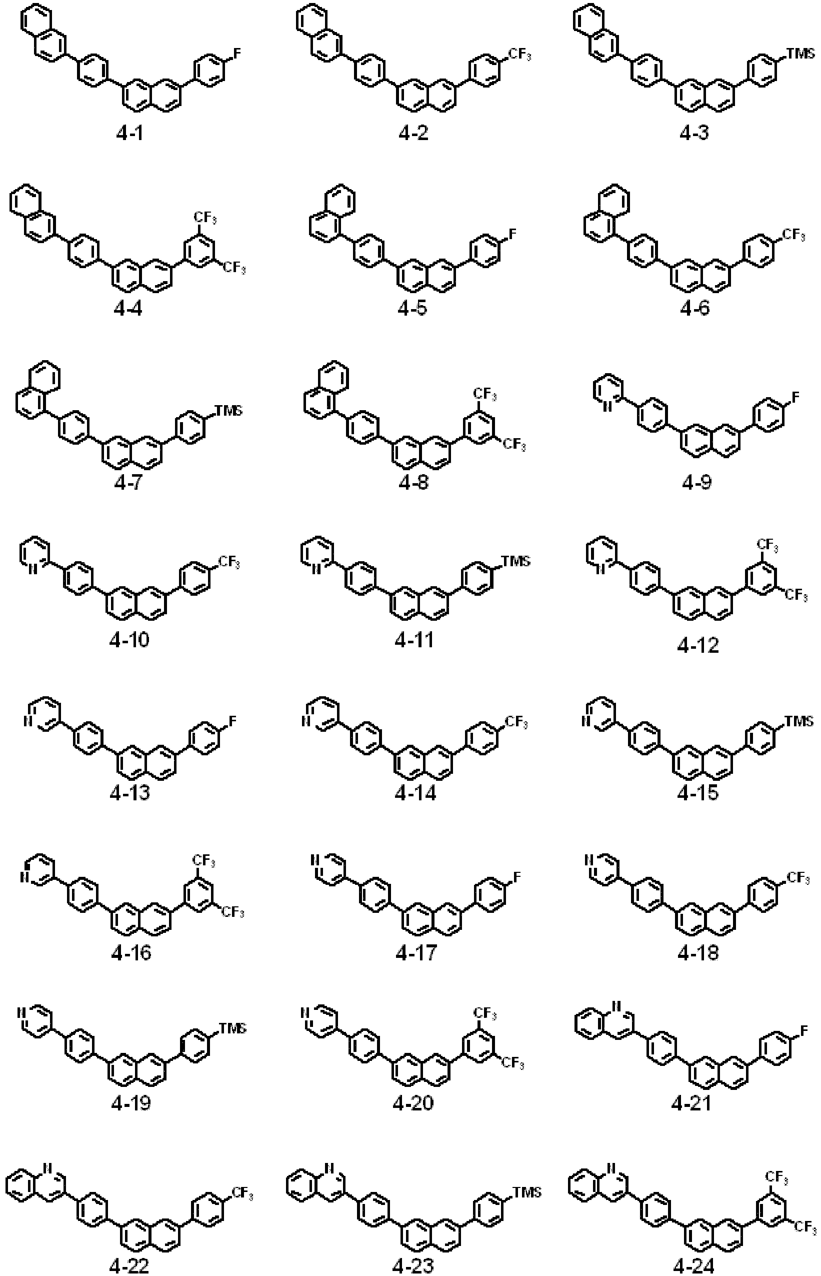

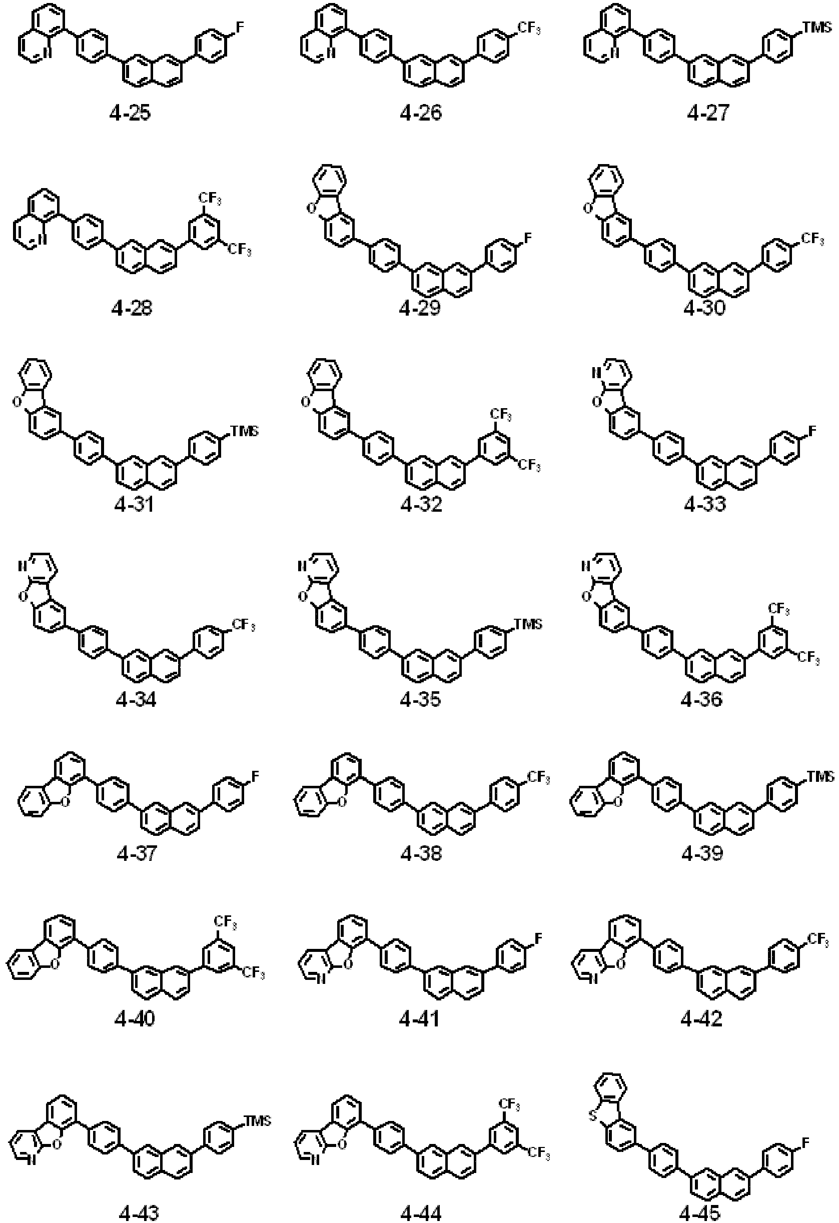

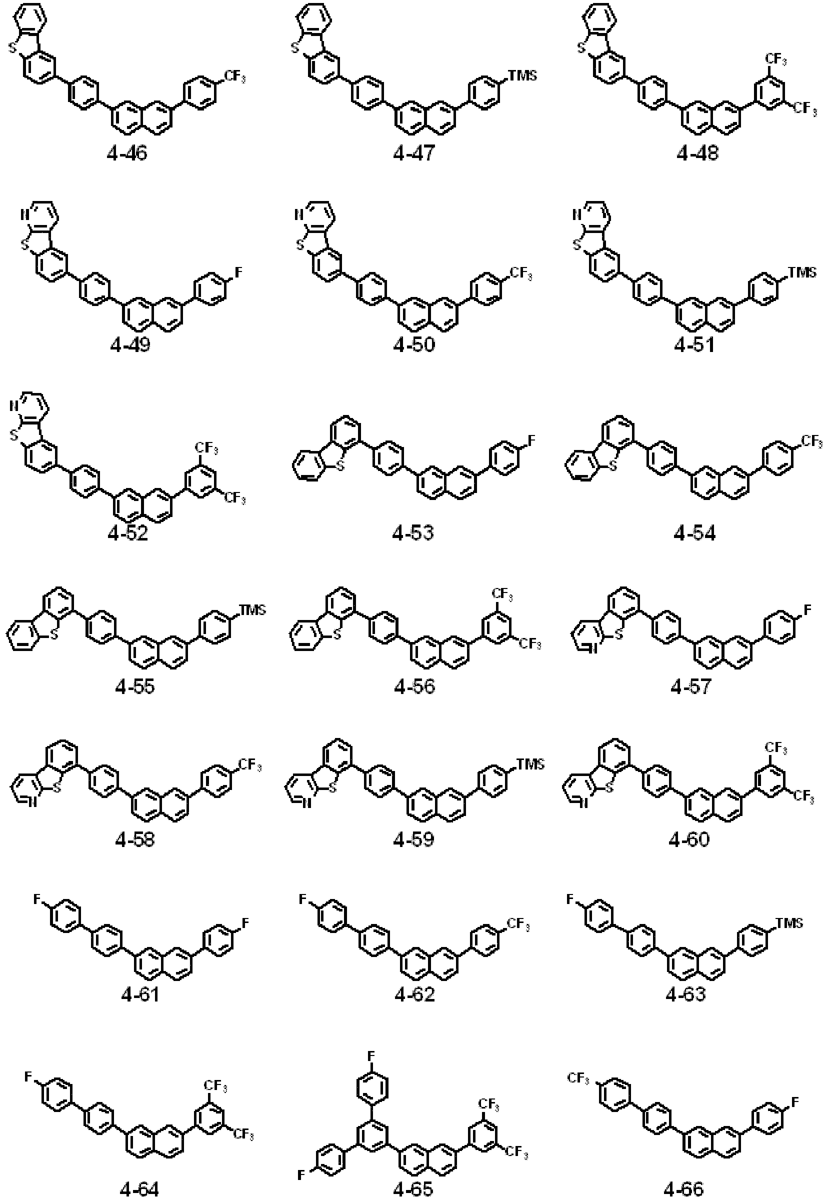

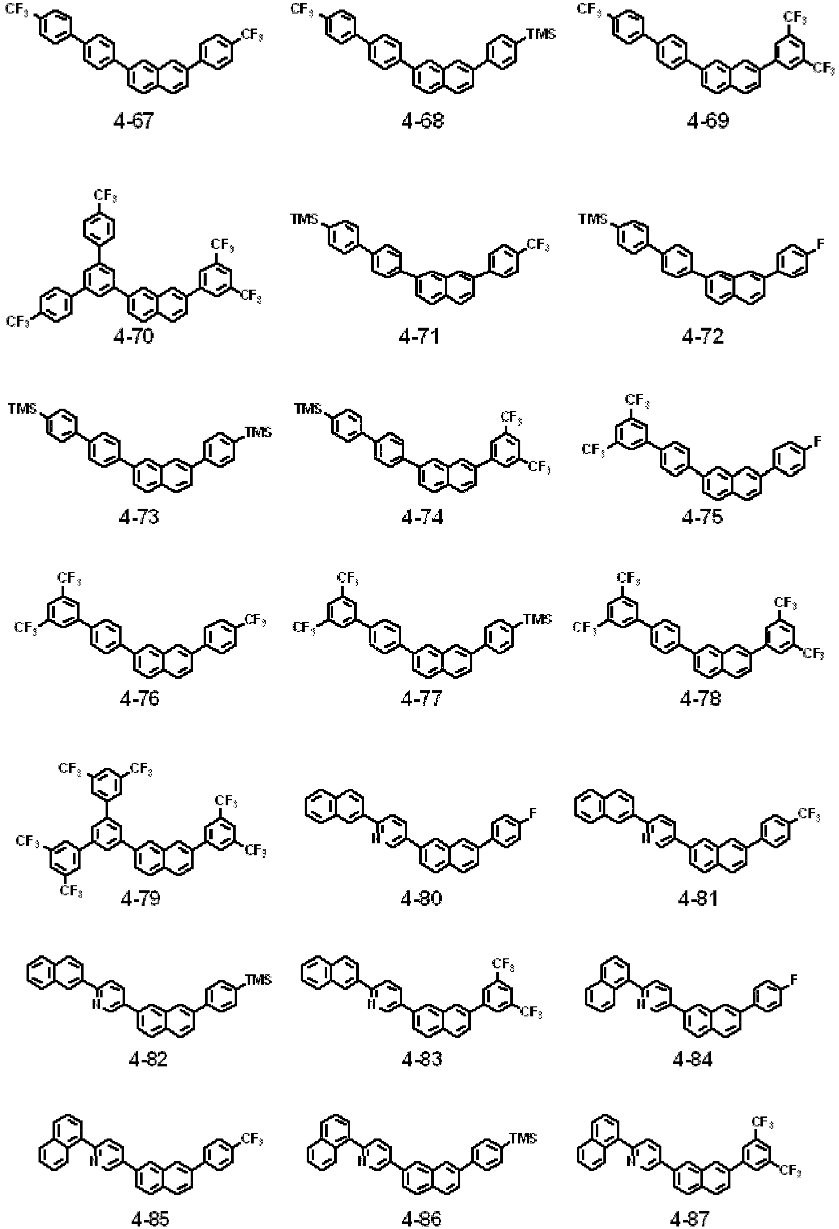

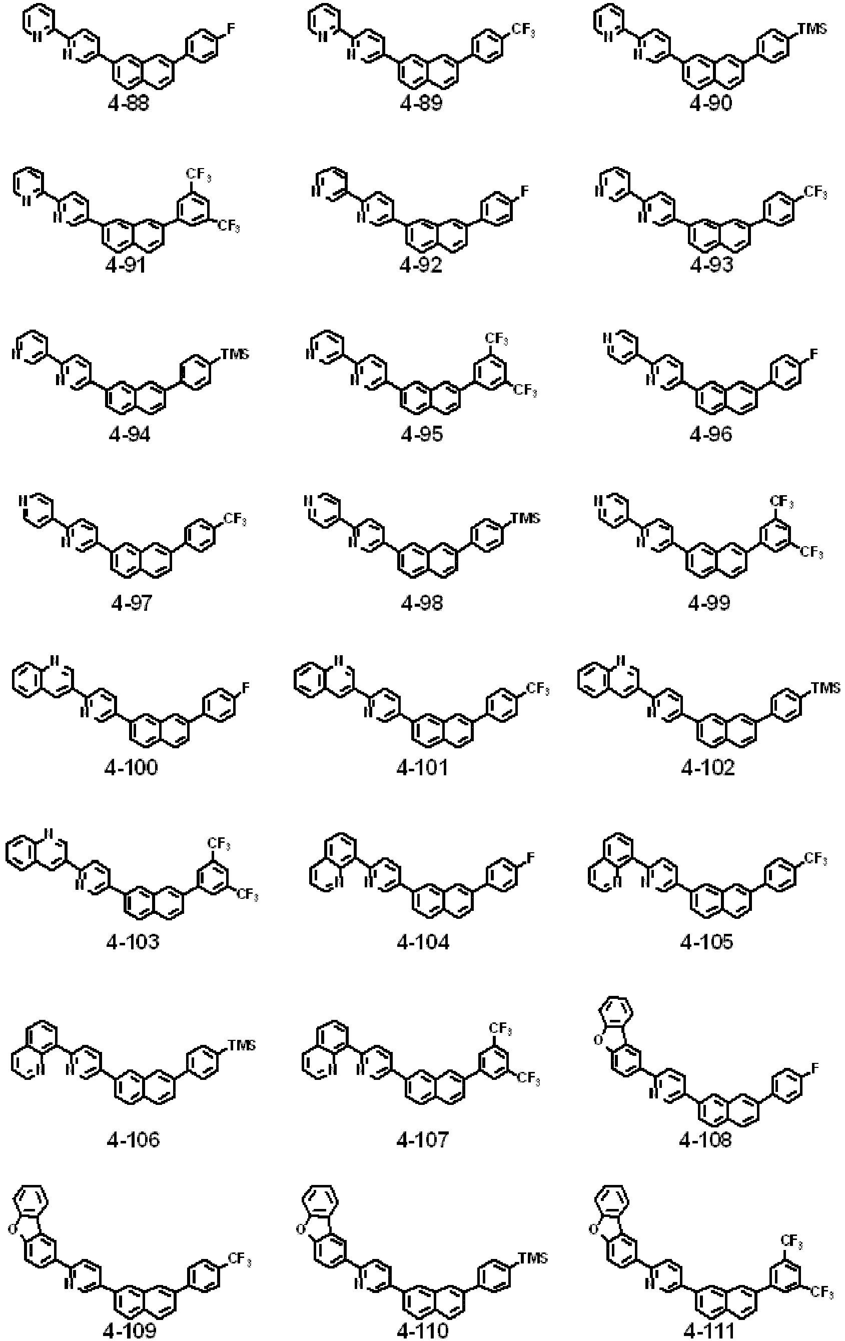

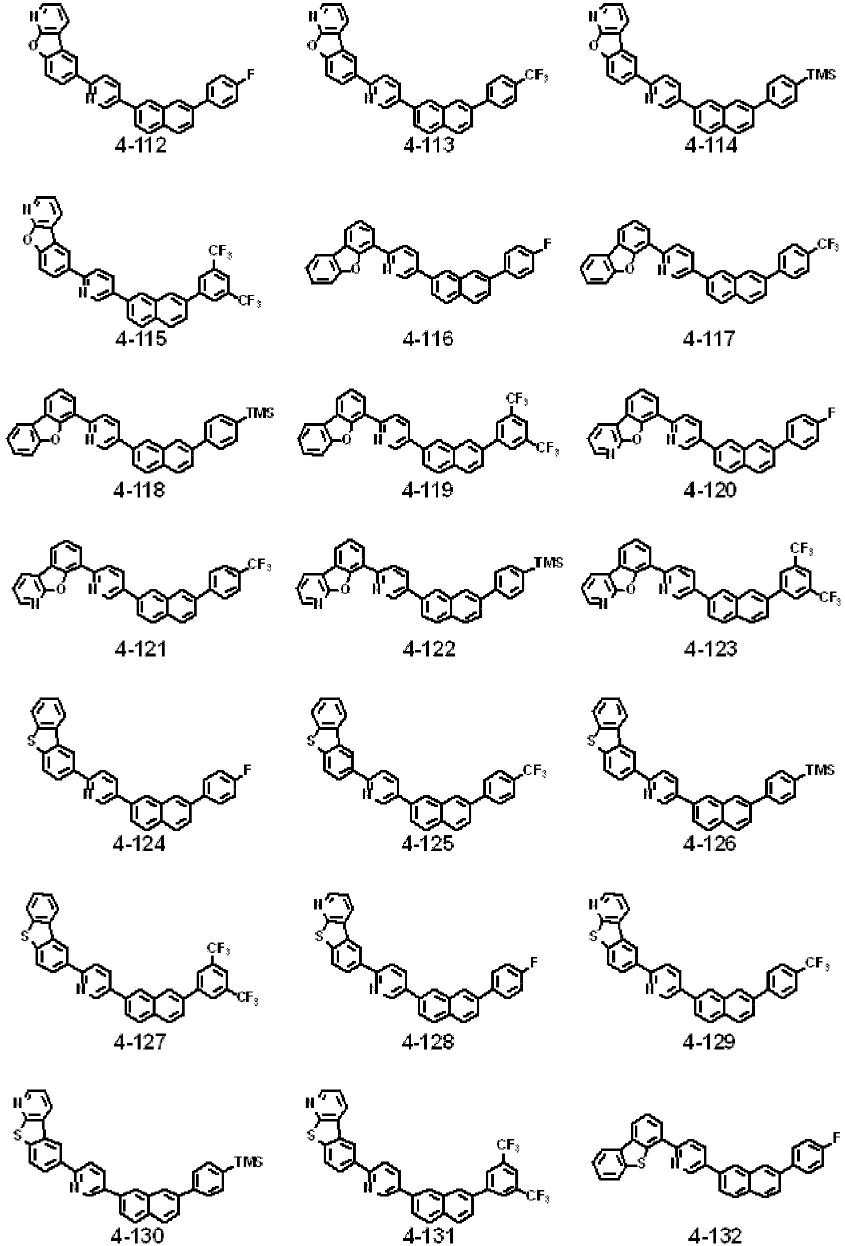

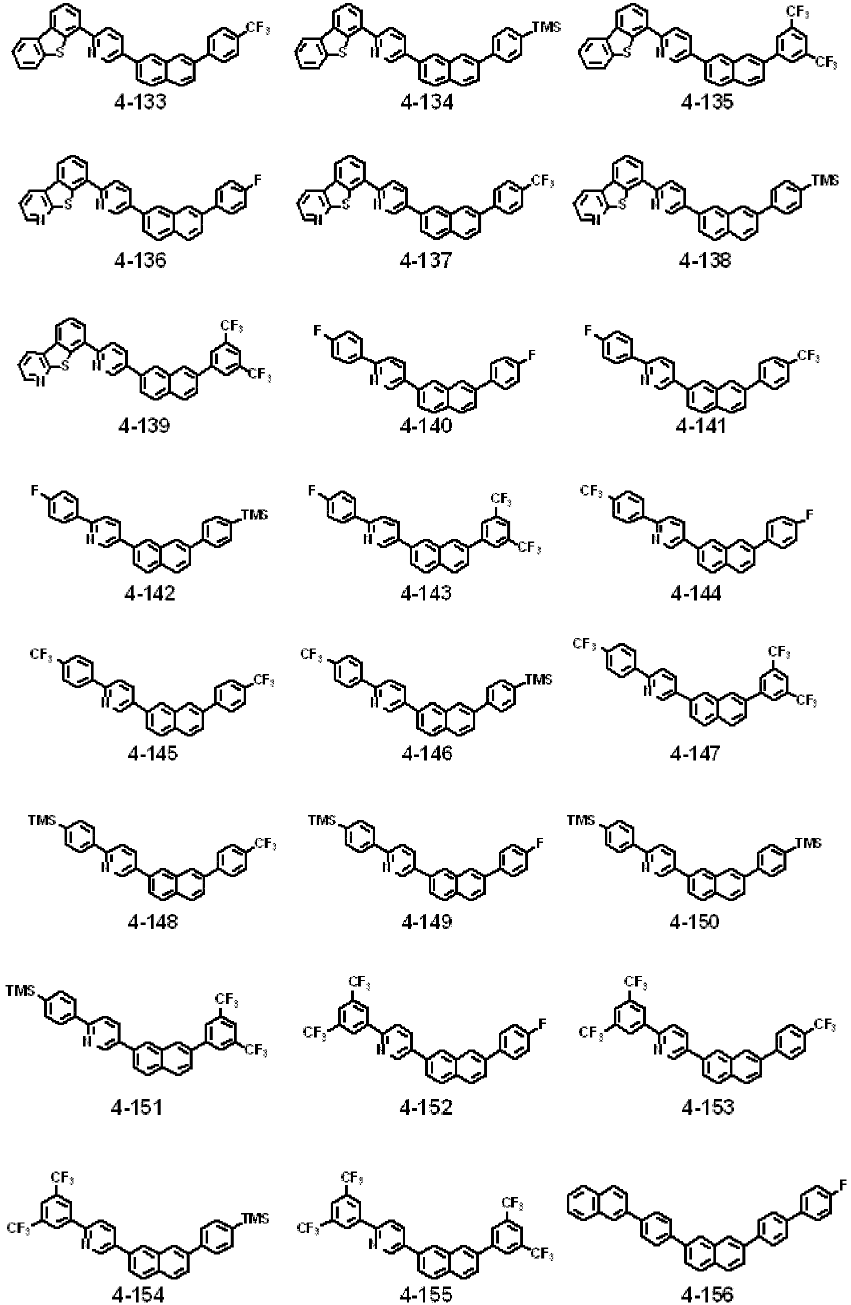

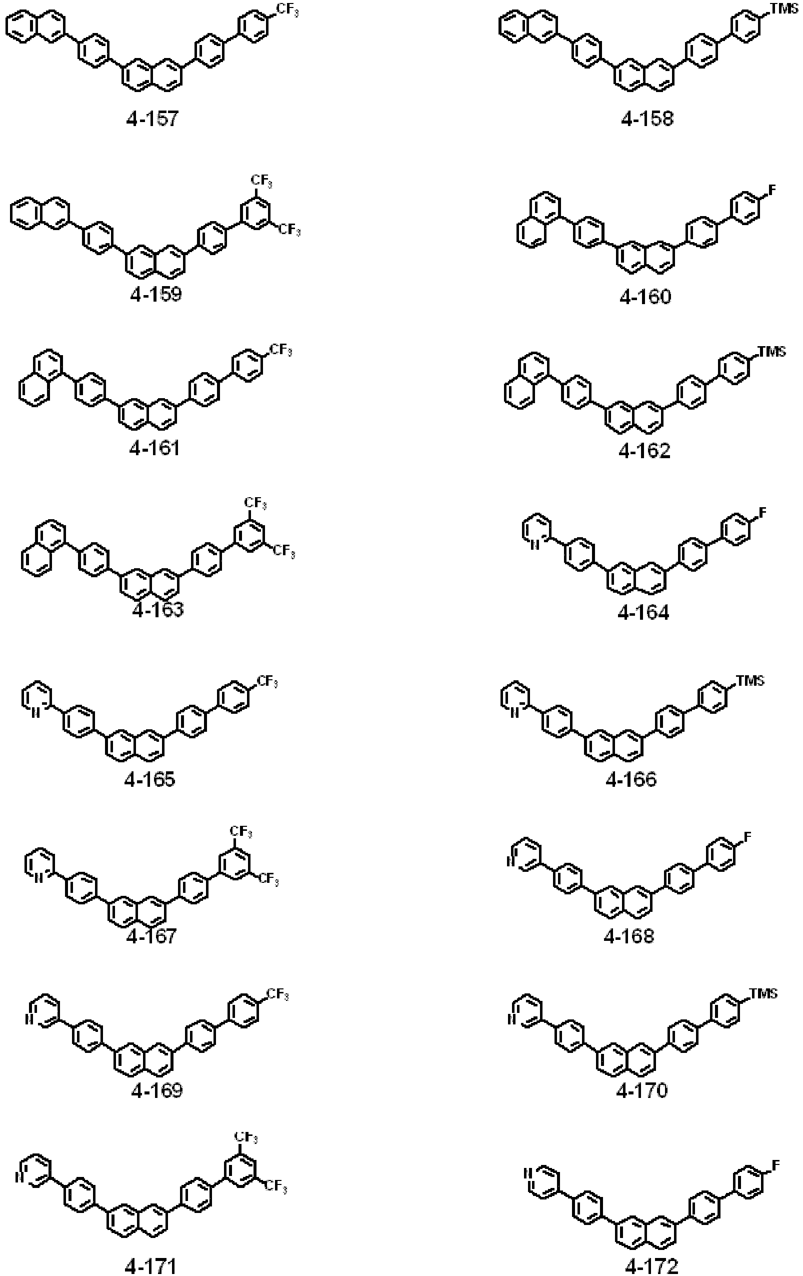

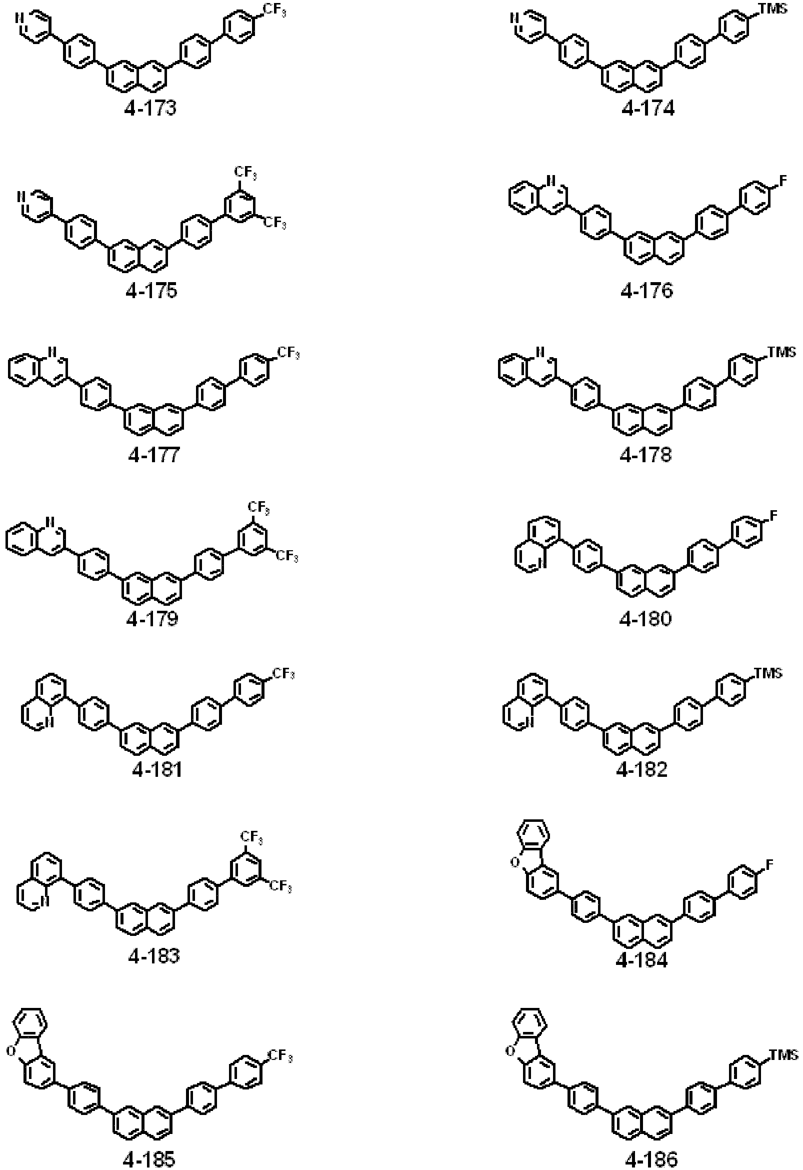

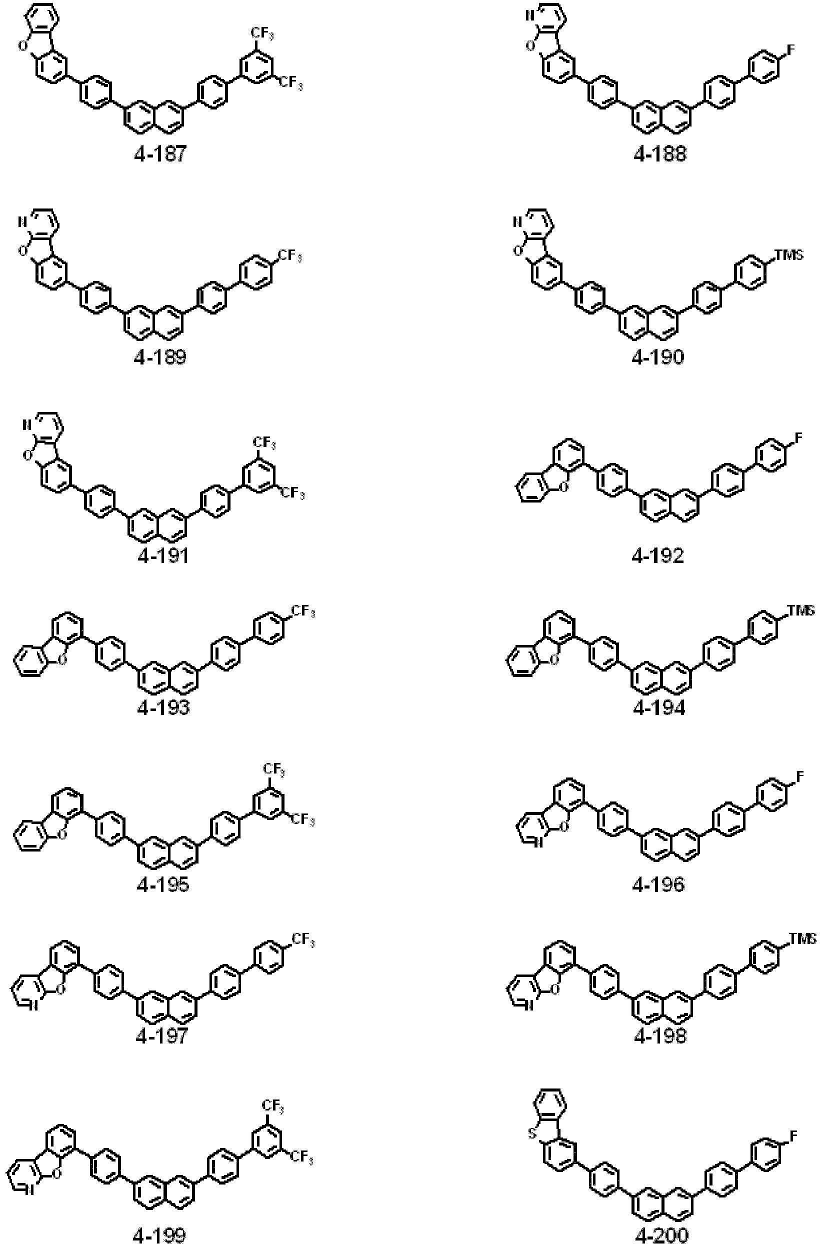

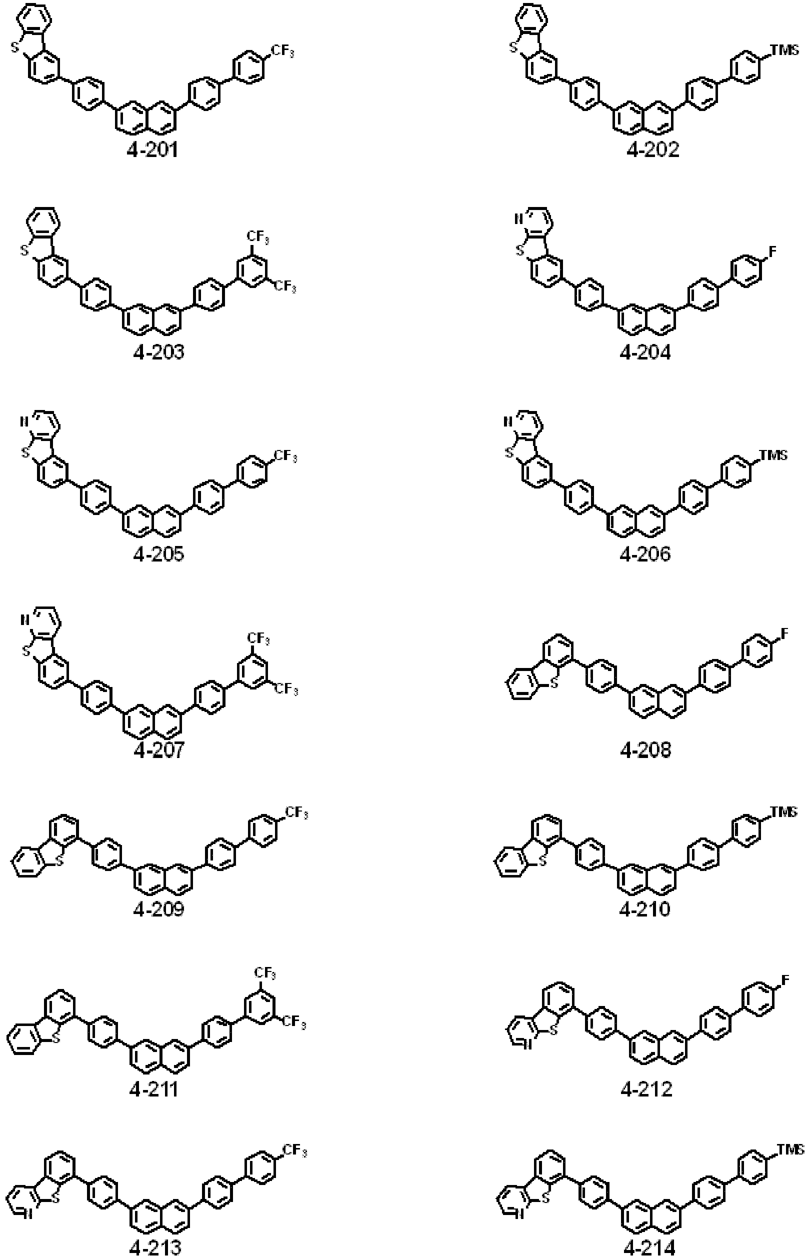

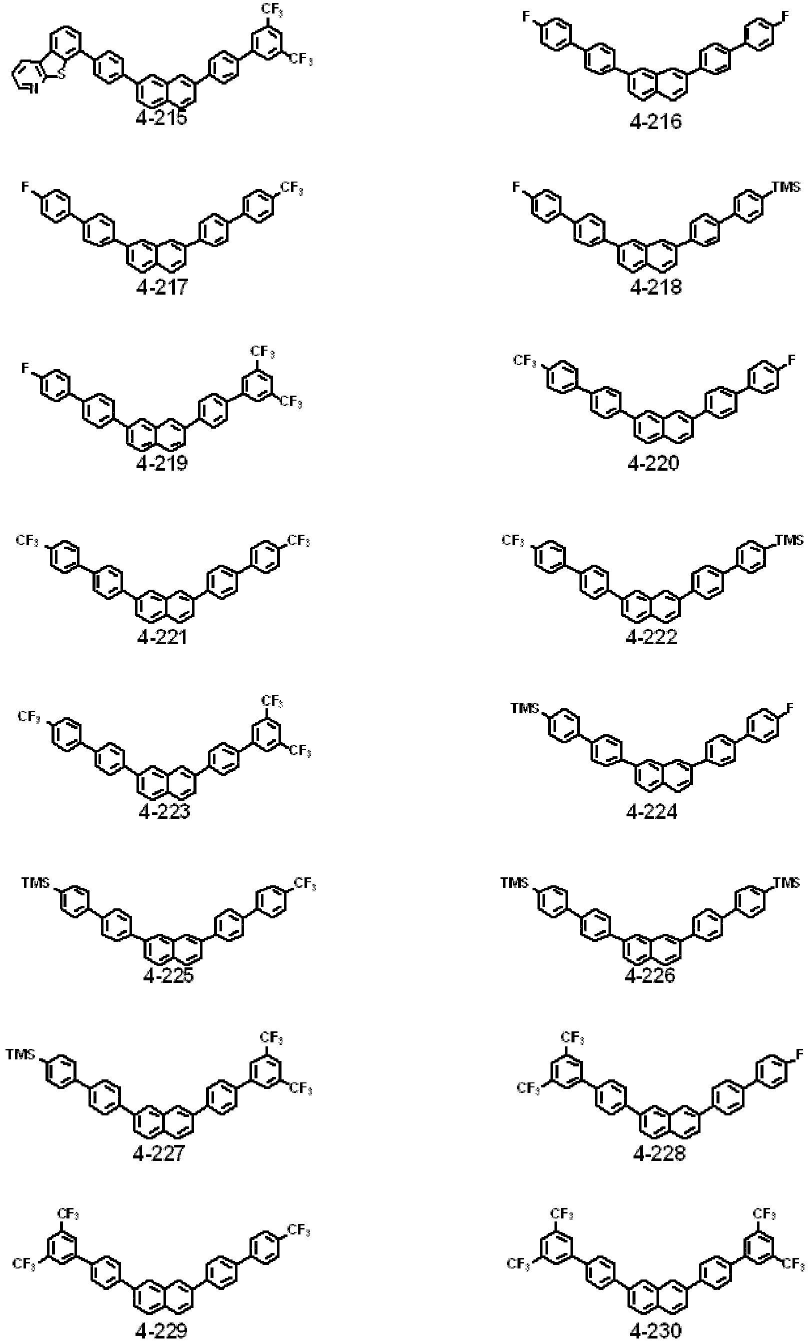

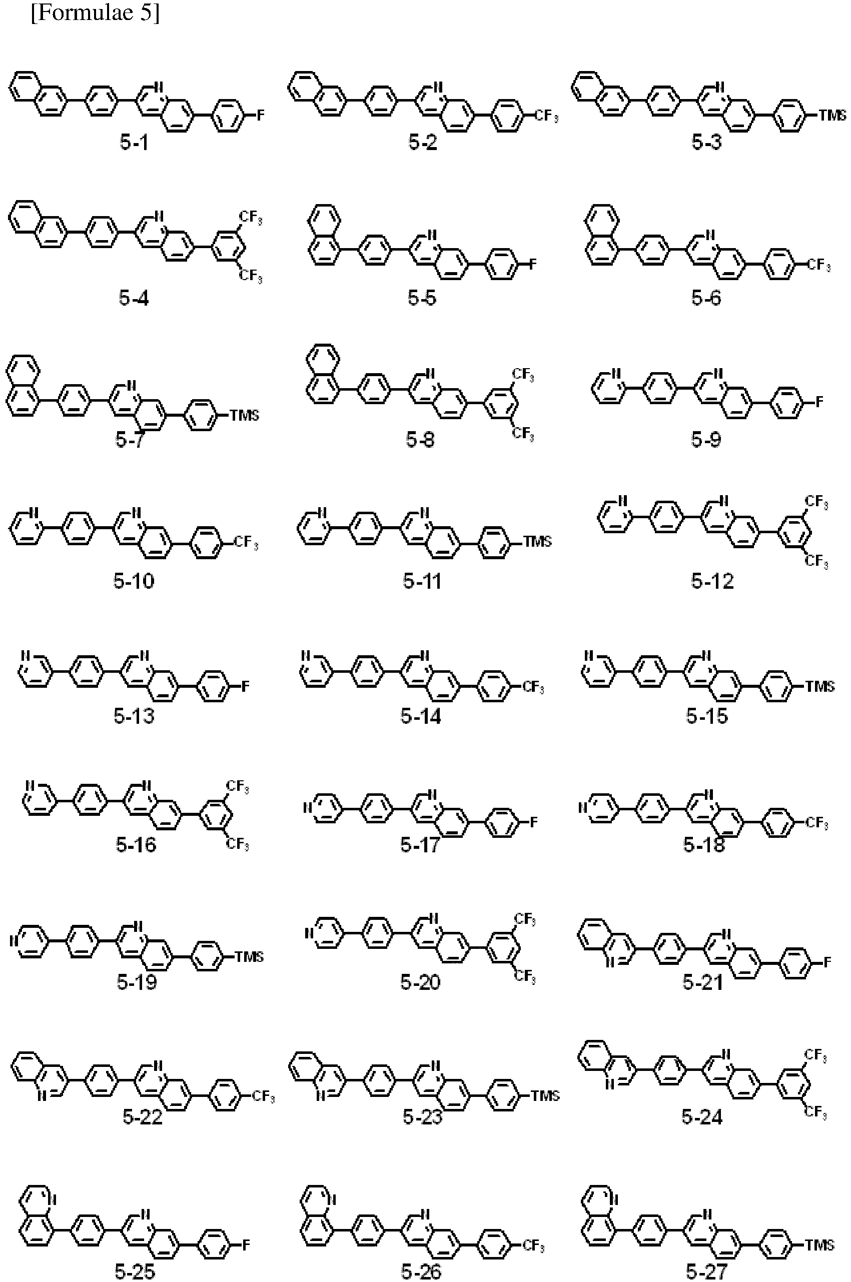

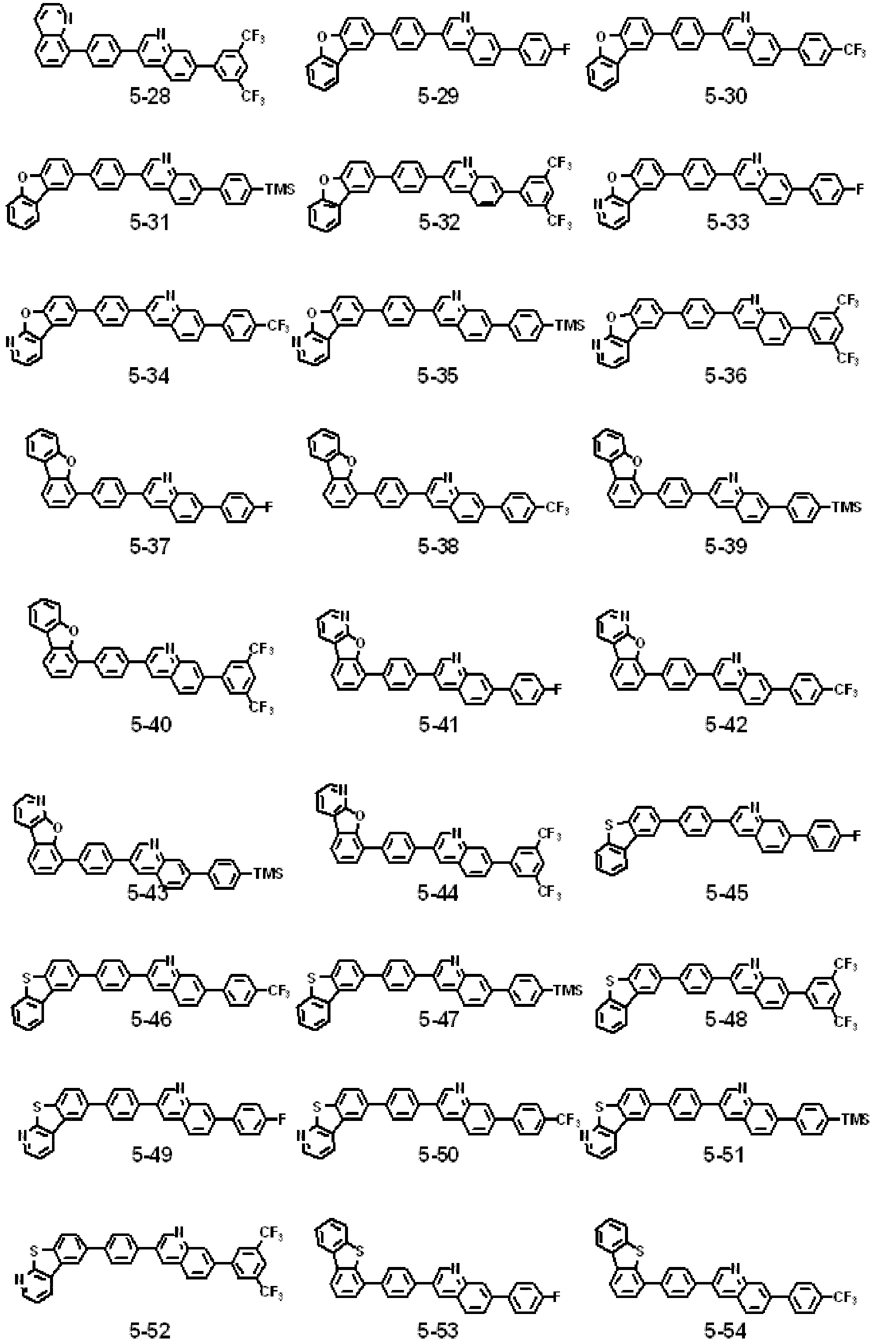

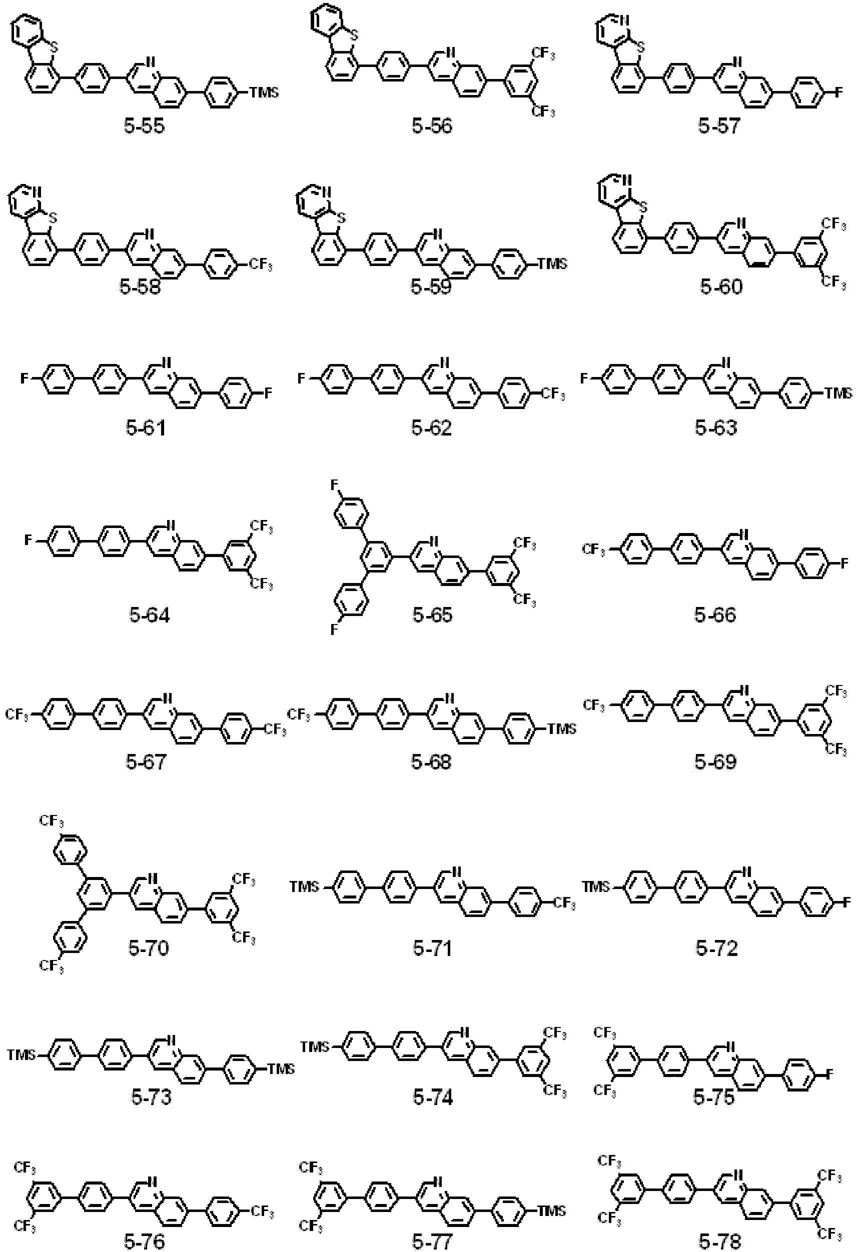

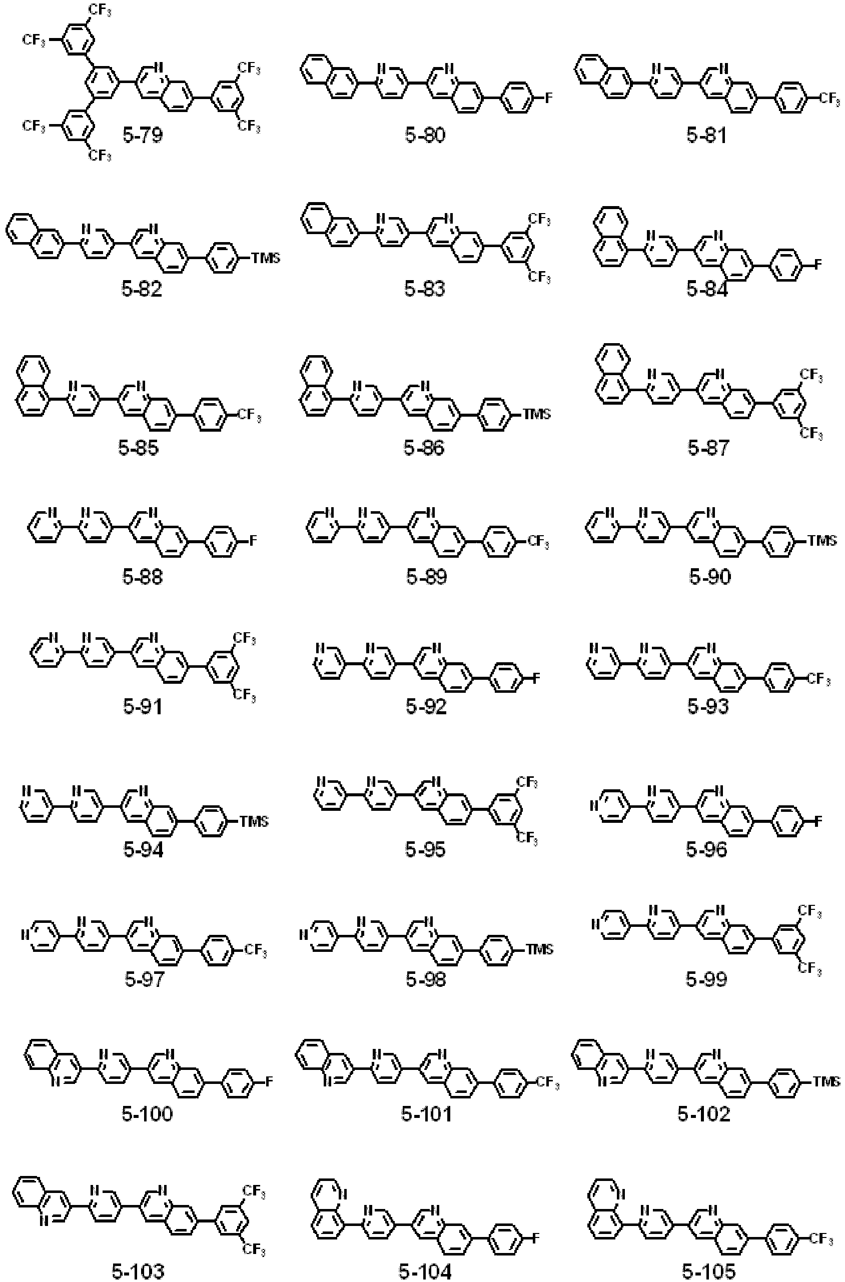

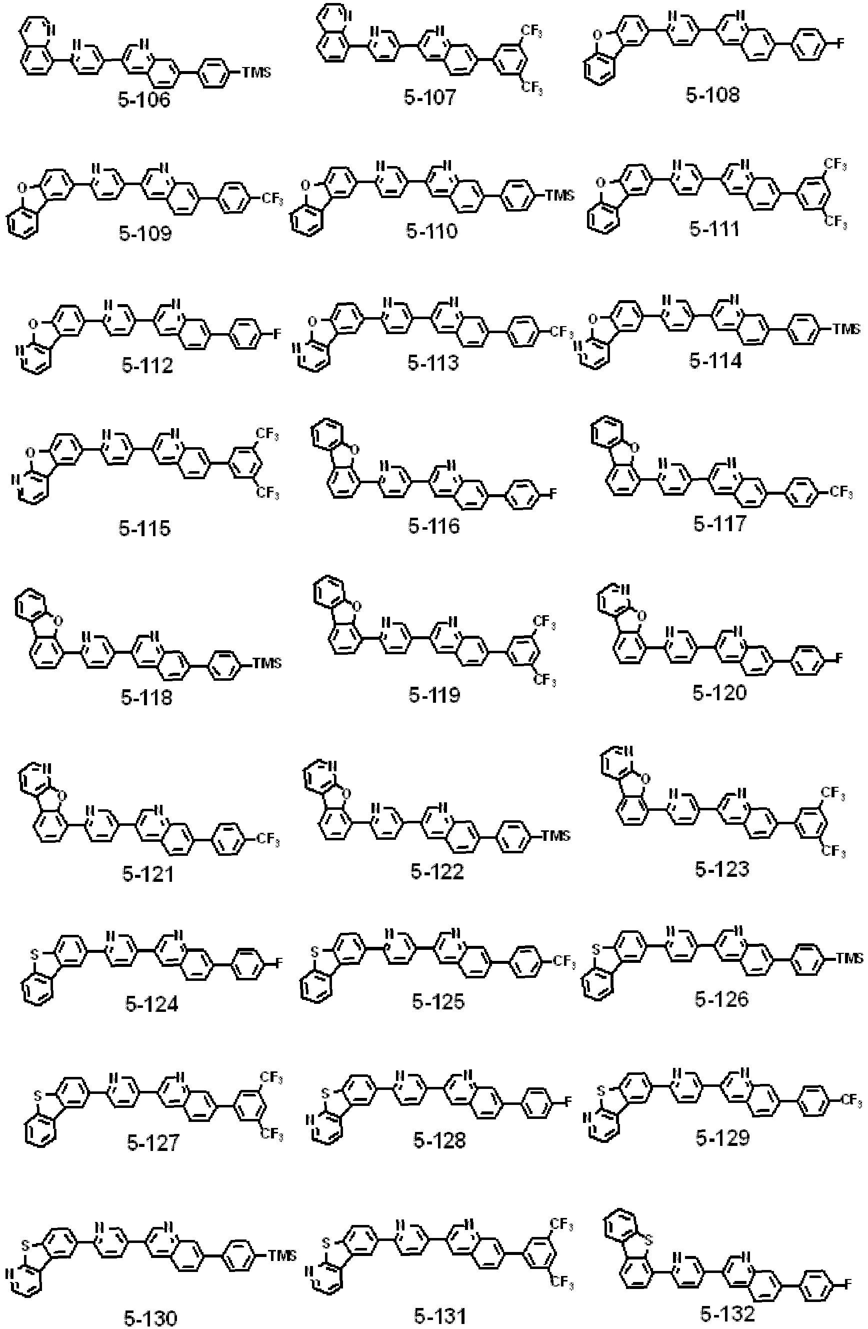

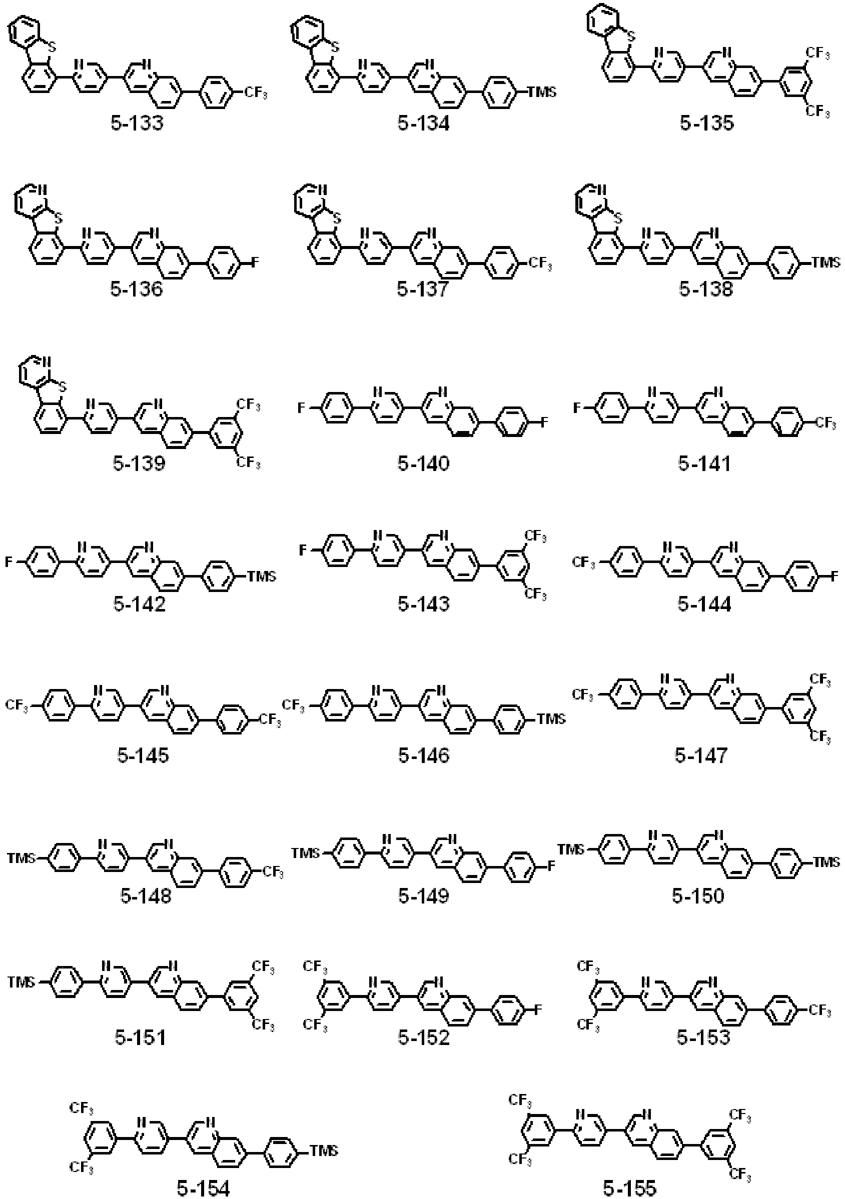

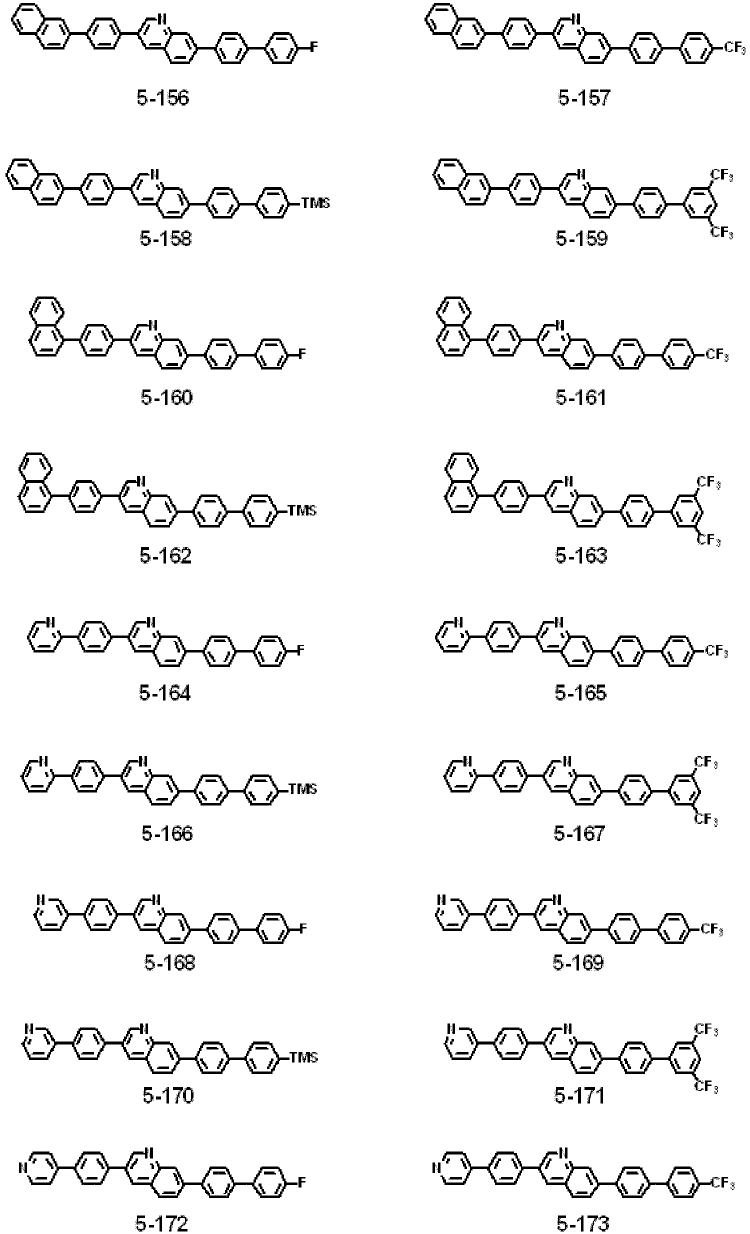

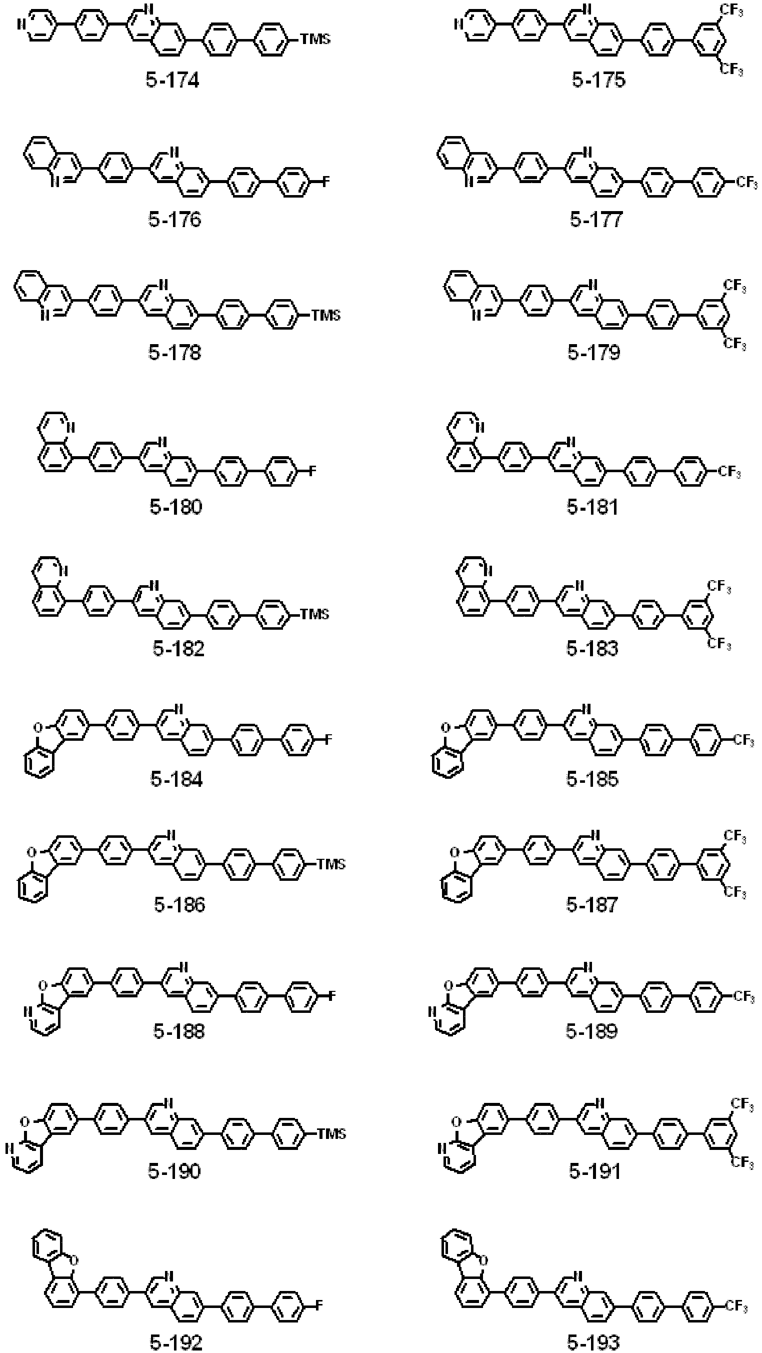

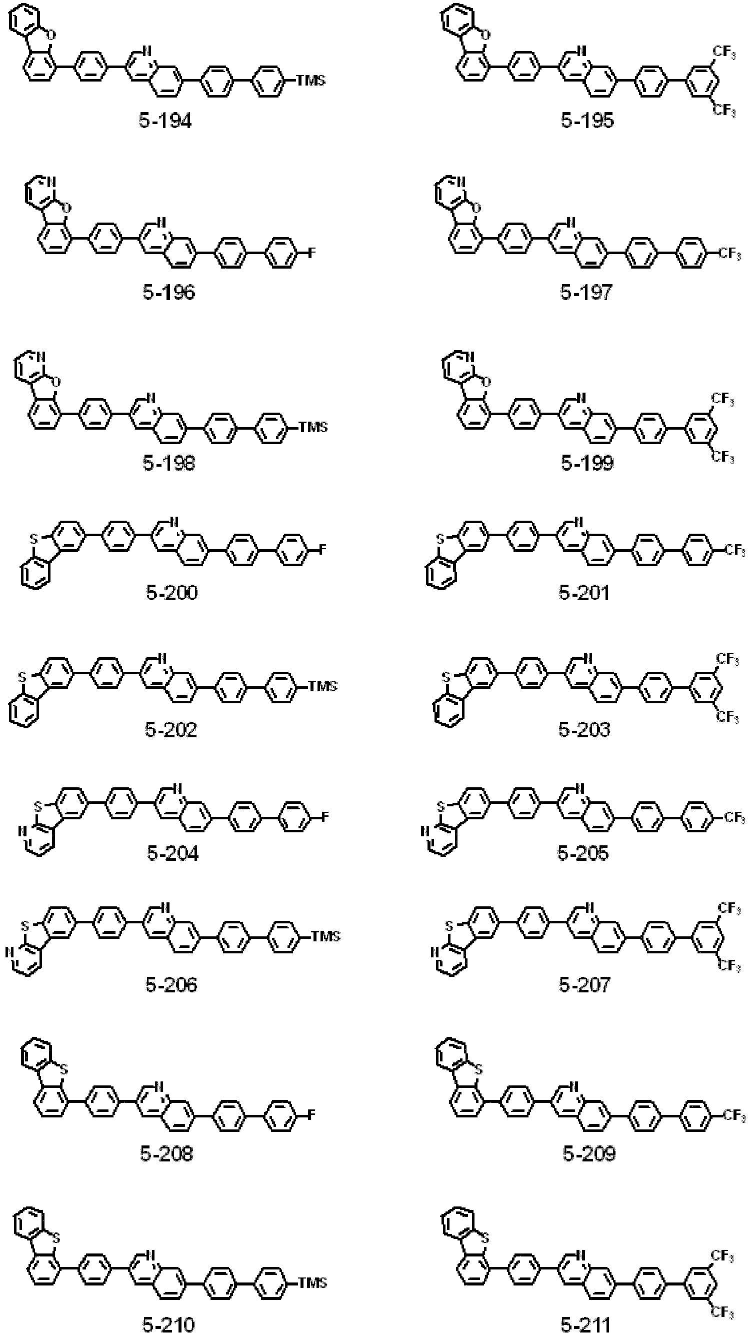

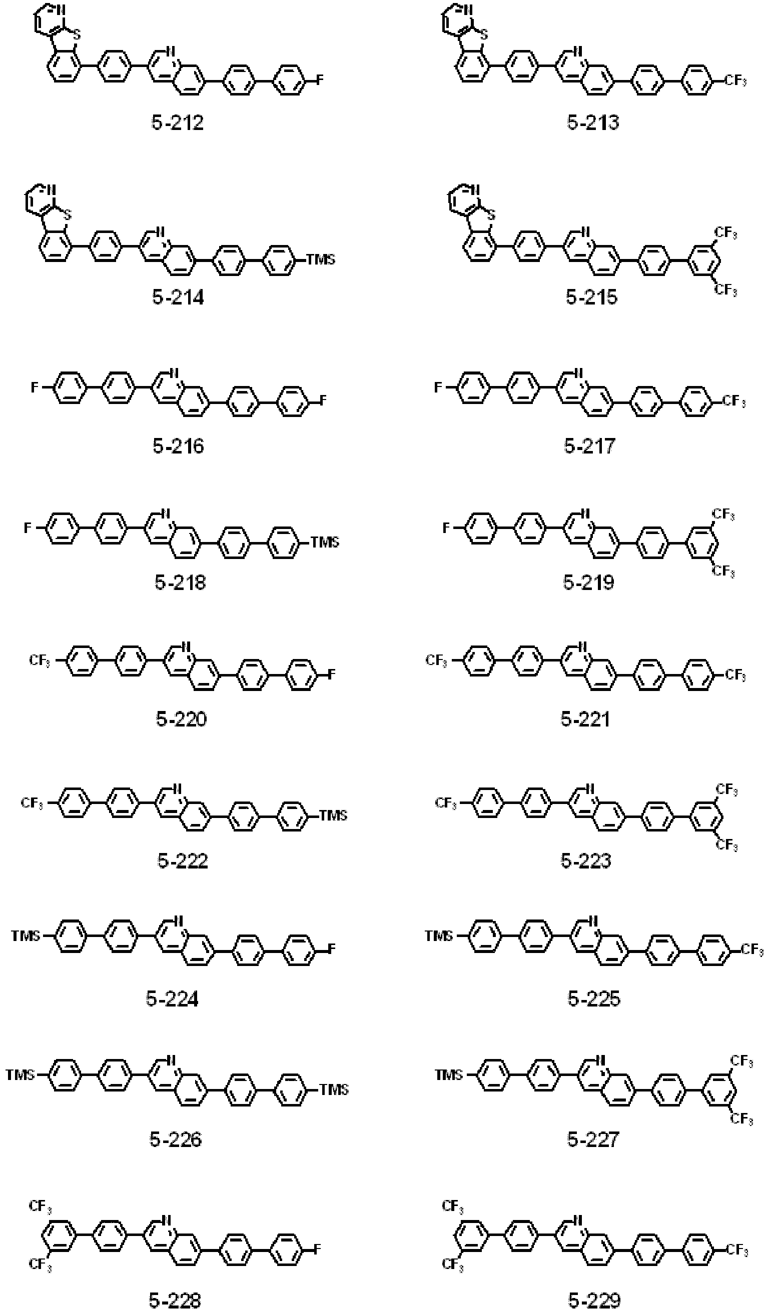

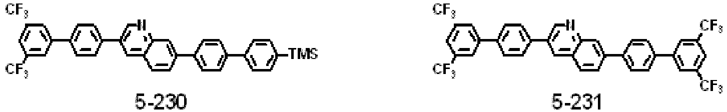

3. The organic compound according to claim 1, wherein the refractive index of the organic compound at 620 nm is less than 1.69

4. An organic electroluminescent device comprising a first electrode, an organic layer consisting of a plurality of layers arranged on the first electrode, a second electrode arranged on the organic layer, and a capping layer arranged on the second electrode wherein the organic layer or the capping layer comprises the organic compound according to any one of claims 1 and 3.

5. The organic electroluminescent device according to claim 4, wherein the capping layer comprises a plurality of layers having different refractive indices.
